# EUROPEAN PATENT APPLICATION

(11) **EP 4 631 499 A1**
(43) Date of publication of application: **15.10.2025**
(21) Application number: 23899964.3
(22) Date of filing: 05.12.2023
(51) Int. Cl.: A61K 9/24, A61K 9/28, A61K 9/30, A61K 9/22, A61K 31/198, A61K 31/195, A61P 25/16

(54) **DELAYED TIMED RELEASE PHARMACEUTICAL COMPOSITION, PREPARATION METHOD THEREFOR, AND USE THEREOF**

(30) Priority: 05.12.2022 CN 202211552563
(71) Applicant: Shanghai WD Pharmaceutical Co., Ltd, Shanghai 201203 (CN)
(72) Inventor: DONG, Liangchang, Shanghai 201203 (CN); SHI, Jingmin, Shanghai 201203 (CN); JIAO, Yan, Shanghai 201203 (CN); HAO, Jianing, Shanghai 201203 (CN); MA, Wenbo, Shanghai 201203 (CN); HUANG, Mingjun, Shanghai 201203 (CN); WEI, Xiaoxiong, Shanghai 201203 (CN); ZHANG, Danyong, Shanghai 201203 (CN); WANG, Jingyi, Shanghai 201203 (CN)
(74) Representative: Greaves Brewster LLP
(86) International application number: PCT/CN2023/136456
(87) International publication number: WO 2024/120389

(57) **Abstract**

A delayed timed release pharmaceutical composition, a preparation method therefor, and use thereof. The pharmaceutical composition comprises a tablet core. The tablet core comprises a drug-containing layer and a boosting layer stacked on the drug-containing layer. The drug-containing layer comprises a drug active ingredient. The drug active ingredient is levodopa or a derivative thereof, or a mixture of levodopa or a derivative thereof and a DOPA decarboxylase inhibitor, and accounts for 5-72.5 wt% of the content of the drug-containing layer. The pharmaceutical composition is a capsule-shaped tablet, can realize the effects of 1-3 h delayed release of the drug active ingredient and reaching a peak concentration at 6-10 h, can be used for reducing morning stiffness in patients with Parkinson's disease, and has good application prospects.

## Description

The present application claims the right of the priority of Chinese patent application 2022115525632 filed on December 5, 2022. The contents of the Chinese patent application are incorporated herein by reference in its entirety.

### TECHNICAL FIELD

The present disclosure relates to the field of biopharmaceuticals, in particular to a delayed timed release pharmaceutical composition, a preparation method therefor and use thereof.

### BACKGROUND

Parkinson's disease is a progressive neurodegenerative disease caused by the loss of cells that produce dopamine in the brain. Dopamine is a substance present naturally in the brain and spinal cord, which helps nerve cells in the brain to control motor function correctly. As dopamine levels in the brain decrease, symptoms of Parkinson's disease (PD) appear, such as muscle stiffness, slow movement and difficulty maintaining balance. Dopamine cannot cross the blood-brain barrier (BBB), which is the reason why its oral dosage form is not effective. Levodopa is a precursor of dopamine, which can cross the blood-brain barrier and be converted into dopamine in brain tissue. Levodopa therapy is the "gold standard" for the treatment of Parkinson's disease, and almost all patients with PD must receive levodopa treatment at some stage of the disease. However, most levodopa is decarboxylated to dopamine before reaching the brain. Therefore, levodopa is usually administered in combination with decarboxylase inhibitors such as carbidopa or benserazide to prevent the formation of peripheral dopamine, increase the content of dopamine in the brain, and alleviate adverse effects due to peripheral decarboxylation of levodopa.

Parkinson's disease, morning akinesia symptoms in Parkinson's disease and the shortcomings of existing treatment options are described in the following literatures:
(1) J Jankovic. Parkinson's disease: clinical features and diagnosis. Journal of Neurology, Neurosurgery & Psychiatry, 2008; 79: 368-376.
(2) NB Mercuri. Limitations of current Parkinson's disease therapy. Ann Neurol 2003; 53: S3-S15.
(3) K Ray Chaudhuri DSc. Non-motor symptoms of Parkinson's disease: dopaminergic pathophysiology and treatment. The Lancet Neurology 2009; 8: 464-474.
(4) NB Mercuri. 2005 The 'magic' of L-dopa: why is it the gold standard Parkinson's disease therapy? Trends in pharmacological sciences, 2005; 26:341-344.
(5) Nicola T, Simone S, Pasquale N, et al. Morning akinesia in Parkinson's disease-challenges and solutions. Journal of Parkinsonism and Restless Legs Syndrome. 2016; 6: 57-63.
(6) Rizos A, Martinez-Martin P, Odin P, et al. Characterizing motor and non-motor aspects of early-morning off periods in Parkinson's disease: an international multicenter study. Parkinsonism Relat Disord. 2014; 20(11):1231-1235.
(7) Chapuis S, Ouchchane L, Metz O, Gerbaud L, Durif F. Impact of the motor complications of Parkinson's disease on the quality of life. Mov Disord. 2005; 20(2):224-230.
(8) Factor SA, McAlarney T, Sanchez-Ramos JR, Weiner WJ. Sleep disorders and sleep effect in Parkinson's disease. Mov Disord. 1990; 5(4):280-285.
(9) Garcia-Borreguero D, Larrosa O, Bravo M. Parkinson's disease and sleep. Sleep Med Rev. 2003; 7(2):115-129.
(10) Pfeiffer RF. Gastrointestinal, urological, and sexual dysfunction in Parkinson's disease. Mov Disord. 2010; 25:94-97.
(11) Heetun ZS, Quigley EMM. Gastroparesis and Parkinson's disease: a systematic review. Parkinsonism Relat Disord. 2012; 18(5):433-440.
(12) Kurlan R, Rothfield KP, Woodward WR, et al. Erratic gastric emptying of levodopa may cause "random" fluctuations of parkinsonian mobility. Neurology. 1988; 38(3):419-421.
(13) Jost WH. Gastrointestinal motility problems in patients with Parkinson's disease. Effects of antiparkinsonian treatment and guidelines for management. Drugs Aging. 1997; 10:249-258.
(14) APOKYN Package Insert
(15) Stuart I, Mark L, William O, et al. Apomorphine Subcutaneous Injection for the Management of Morning Akinesia in Parkinson's Disease. Mov Disord 2016: doi: 10.1002/mdc3.12350.
(16) NEUPRO Package Insert
(17) Claudia T, Bryan K, et al. Rotigotine Effects on Early Morning Motor Function and Sleep in Parkinson's Disease: A Double-Blind, Randomized, Placebo-Controlled Study (RECOVER). Mov Disord, 2011; 26(1): 90-99.
(18) INBRIJA Package Insert
(19) Robert A. Hauser, et al. Orally inhaled levodopa (CVT-301) for early morning OFF periods in Parkinson's disease. Parkinsonism and Related Disorders, https://doi.org/10.1016/j.parkreldis.2019.03.026.
(20) Zibetti M, Rizzone M, Merola A, et al. Sleep improvement with levodopa-carbidopa intestinal gel infusion in Parkinson disease. Acta Neurol Scand 2013: 127: e28-e32 DOI: 10.1111/ane.12075.)

As shown in literatures 5-11, as Parkinson's disease progresses, patients will experience severe morning akinesia symptoms in the early morning because of insufficient dopamine storage at night. Morning akinesia symptoms are the most common and earliest motor complication in PD patients, which occur in about 60% of PD patients receiving dopamine therapy, especially in patients with mid-to-late stage Parkinson's disease, that is, patients with Hoehn-Yahr grade of 2.5-3. The occurrence of morning akinesia symptoms seriously affects the daily activities of PD patients, such as getting up, dressing, using the bathroom, washing and taking medicine. Morning akinesia is also associated with postprandial abdominal distension, abdominal discomfort, early satiety, nausea, vomiting, weight loss and malnutrition, as well as gastrointestinal dysfunction, manifested as dry mouth, salivation, dysphagia, esophageal motility disorder, gastroesophageal reflux, gastroparesis, constipation, defecation disorder, *etc.* These symptoms significantly affect the quality of life of PD patients.

As reported in literature 12, gastroparesis will lead to delayed delivery of levodopa to the duodenum, resulting in clinically delayed onset of action (ON) after levodopa administration. Therefore, we can infer that the absence of blood drug concentration of levodopa at night leads to the decline and deficiency of dopaminergic function, resulting in morning akinesia symptoms and other related symptoms, such as gastroparesis, which in turn delays the absorption and onset of action of levodopa taking in the morning, resulting in a vicious circle. As shown in literature 13, other Parkinson's treatment drugs such as dopaminergic agonists (pramipexole, rotigotine, ropinirole), anticholinergic drugs (amantadine), monoamine oxidase inhibitors (selegiline, rasagiline, safinamide), and COMT inhibitors (tolcapone, entacapone) can all lead to gastrointestinal dysfunction.

The existing treatment plan for morning akinesia symptoms is to give patients a rapid and effective blood drug concentration of levodopa in the early morning, which can relieve morning akinesia symptoms. However, due to pharmacodynamic and pharmacokinetic reasons such as gastric emptying time, absorption of levodopa only in the upper gastrointestinal tract, and competitive crossing of the blood-brain barrier with amino acids, the existing conventional levodopa preparations cannot solve the treatment problem of PD morning akinesia symptoms due to short half-life, irregular gastrointestinal absorption, competitive crossing of the blood-brain barrier, *etc.*

For example, literatures 14-15 show that subcutaneous injection of apomorphine preparation (APOKYN^{®}) was launched in the United States in 2004 for the emergency and intermittent treatment of OFF episodes in Parkinson's patients. It has a certain effect on morning akinesia symptoms, but because morning akinesia impairs the patient's motor function, it is difficult for the patient to self-administer the drug. The onset time after administration is 30-60 minutes, which seriously affects the patient's self-care. In addition, a common side effect of subcutaneous injections is the development of nodules, which often become infected, requiring antibiotic treatment or surgical debridement, and apomorphine can cause vomiting, requiring the addition of antiemetics routinely before administration.

The following literature 16 shows that apomorphine sublingual film (KYNMOBI^{®}) was just launched in the United States in May 2020, and it is also used for the emergency and intermittent treatment of OFF episodes in Parkinson's patients. The incidence of adverse reactions of nausea, oropharyngeal soft tissue edema, pain and paralysis is high.

As shown in literatures 17-18, rotigotine transdermal patch (NEUPRO^{®}) was launched in the United States in 2007 and China in 2018. As a dopamine receptor agonist, rotigotine transdermal patch has a certain effect on relieving morning akinesia symptoms and improving sleep. However, adverse reactions such as nausea, vomiting, lethargy and skin allergy limit its use.

As shown in literatures 19-20, levodopa inhalation (INBRIJA^{®}) was launched in the United States in 2018 and was approved by the FDA for emergency treatment of OFF episodes. However, due to the need to use a special device for oral inhalation, self-administration is also difficult, and its bioavailability is low. According to the instructions, its Cmax is only 50% of oral immediate-release preparations, and the incidence of respiratory adverse reactions is as high as 35%. Due to its risk of inducing bronchospasm, it is contraindicated in patients with asthma, chronic obstructive pulmonary disease (COPD) or chronic lung diseases.

In theory, patients need a high concentration of levodopa before getting up, which can reduce the occurrence of morning akinesia symptoms. Under the existing conditions, if the patient takes conventional levodopa preparations (such as Madopar^{®} and Sinemet^{®}) the night before, it is not only impossible to maintain the effective blood drug concentration until getting up in the morning, but also the high concentration of levodopa produced by rapid release has a certain excitatory effect, which may affect the patient's falling asleep.

Giving levodopa before going to bed may affect falling asleep, but providing a certain concentration of levodopa after falling asleep can relieve muscle tension and stiffness, which may be helpful in improving sleep quality. Therefore, a delayed-release controlled-release dosage form is needed to ensure that patients reach the effective blood drug concentration of levodopa when they wake up in the morning, reduce the occurrence of morning akinesia symptoms, and improve the symptoms of night akinesia, thereby improving the sleep quality of patients. Although, as shown in literature 21, continuous levodopa duodenal perfusion (DUODOPA/DUOPA) has a certain therapeutic effect on morning akinesia symptoms. However, after 2-4 months of continuous infusion of levodopa hydrogel, the PDSS-2 (PD-Sleep-Scale-version-2) total score and the sub-scores of "sleep disorder", "nocturnal motor symptoms" and "nocturnal PD symptoms" were significantly reduced, and the ESS (Epworth-Sleeping-Scale) value of daytime sleepiness also improved. In addition, this treatment causes too much trauma to the human body, and requires surgery to directly pump into the upper small intestine through a tube for continuous perfusion. The treatment cost is expensive, and the scope of application for patients is relatively narrow. At present, the drug is only used for 16 hours during the day, and not at night.

Therefore, for patients, the most ideal treatment option is non-invasive therapy. Therefore, there is an urgent clinical need to develop a non-invasive delayed timed release controlled-release product, which can provide effective levodopa blood drug concentration in PD patients to relieve morning akinesia during the morning awakening phase, so that patients can be in an "ON" state instead of an "OFF" state when they wake up in the morning.

### CONTENT OF THE PRESENT INVENTION

The technical problem to be solved by the present disclosure is to overcome the defects that the existing controlled-release drug containing levodopa cannot realize effective delay and sustained release after delay, and then cannot maintain the effective blood drug concentration of levodopa during the morning awakening phase, alleviate morning akinesia symptoms and other related symptoms caused therefrom, and therefore provide a delayed timed release pharmaceutical composition, a preparation method therefor and use thereof. The pharmaceutical composition of the present disclosure can achieve the effect of releasing the active pharmaceutical ingredient with a time lag of 1-3 hours and reaching the peak concentration in 6-10 hours; preferably, it can also achieve the effect of releasing the active pharmaceutical ingredient with a time lag of 2 hours and reaching the peak concentration in 6-8 hours, thereby reducing the occurrence of morning akinesia and other related symptoms caused thereby.

The present disclosure solves the above technical problems through the following technical solutions.

The present disclosure provides a pharmaceutical composition comprising a tablet core, wherein the tablet core comprises a drug-containing layer and a push layer stacked on the drug-containing layer; the drug-containing layer comprises an active pharmaceutical ingredient;
in the drug-containing layer, the content of the active pharmaceutical ingredient is 5-72.5wt%, and the content is the weight percentage of the active pharmaceutical ingredient in the drug-containing layer;
in the drug-containing layer, the active pharmaceutical ingredient is levodopa or a derivative thereof, or a mixture of "levodopa or a derivative thereof" and a dopa decarboxylase inhibitor;
when the active pharmaceutical ingredient is a mixture of "levodopa or a derivative thereof" and a dopa decarboxylase inhibitor, the content of the dopa decarboxylase inhibitor is ≤ 14.5wt% but not 0, and the content is the weight percentage of this component in the drug-containing layer;
the dosage form of the pharmaceutical composition is a capsule-shaped tablet.

In the present disclosure, the content of the active pharmaceutical ingredient in the drug-containing layer is preferably 30-72.5wt%, such as 31.5wt%, 50wt%, 53wt%, 58wt%, 59.6wt%, 63wt% or 68.1wt%.

In the present disclosure, the content of the levodopa or a derivative thereof in the drug-containing layer may be conventional in such preparations in the art, and may also be 27-63wt%, such as 27.7wt%, 31.5wt%, 41.7wt%, 46.9wt%, 50wt%, 51.1wt%, 53.6wt%, 55.5wt% or 63wt%, and the content is the weight percentage of this component in the drug-containing layer.

In the present disclosure, the derivative of levodopa in the drug-containing layer may be a levodopa hydrate, a levodopa alkyl ester, a pharmaceutically acceptable salt of levodopa, a deuterated levodopa alkyl ester or a pharmaceutically acceptable salt of a deuterated levodopa alkyl ester.

In the present disclosure, the type of the dopa decarboxylase inhibitor in the drug-containing layer may be a carbidopa hydrate, a pharmaceutically acceptable salt of carbidopa, benserazide or a pharmaceutically acceptable salt of benserazide, and is preferably, for example, carbidopa monohydrate.

In the present disclosure, when the active pharmaceutical ingredient in the drug-containing layer is a mixture of "levodopa or a derivative thereof" and a dopa decarboxylase inhibitor, the content of the dopa decarboxylase inhibitor may be 3.7-14.5wt%, such as 3.75wt%, 6.91wt%, 7.5wt%, 11.3wt%, 12.7wt% or 14.5wt%, and the content is the weight percentage of this component in the drug-containing layer.

In the present disclosure, the drug-containing layer may further comprise a pharmaceutical excipient. The pharmaceutical excipients may be conventional pharmaceutical excipients used in such preparations.

In a preferred embodiment of the present disclosure, the pharmaceutical excipients may comprise one or more of pharmaceutical carriers, fillers, surfactants, lubricants and antioxidants, and may further comprise "pharmaceutical carriers and lubricants", "pharmaceutical carriers, fillers and lubricants", "pharmaceutical carriers, fillers, surfactants and lubricants" or "pharmaceutical carriers, fillers, surfactants, antioxidants and lubricants".

Where the type and content of the pharmaceutical carrier may be conventional in such preparations in the art, and the content is the weight percentage of this component in the drug-containing layer.

The pharmaceutical carrier may be one or more of povidone, copovidone, carbomer, hypromellose, hydroxypropyl cellulose, hydroxyethyl cellulose, polyoxyethylene and sodium alginate, and may further be a mixture of povidone and hydroxypropyl cellulose or hydroxypropyl cellulose.

When the drug-containing layer comprises a pharmaceutical excipient and the pharmaceutical excipient comprises a pharmaceutical carrier, then the content of the pharmaceutical carrier may be ≤ 40wt%, but not 0, and may further be 10-40wt%, such as 15wt%, 16wt%, 29.5wt%, 30.0wt%, 31wt% or 38.5wt%.

When the pharmaceutical carrier is a mixture of povidone and hydroxypropyl cellulose, the weight ratio of hydroxypropyl cellulose to povidone is preferably (1-8):1, such as 2.1:1, 2.2:1, 3.1:1, 3.5:1 or 7.1:1.

Where the type and content of the filler may be conventional in such preparations in the art, and the content is the weight percentage of this component in the drug-containing layer.

The filler may be one or more of lactose, starch, pregelatinized starch, dextrin, mannitol, sorbitol and microcrystalline cellulose, and may further be a mixture of sorbitol and lactose, a mixture of sorbitol and mannitol, sorbitol, or mannitol.

When the drug-containing layer comprises a pharmaceutical excipient and the pharmaceutical excipient comprises a filler, then the content of the filler may be ≤ 51wt%, but not 0, and may further be *5-*51wt%, such as 5wt%, 8.5wt%, 10wt%, 15wt%, 18wt%, 19wt%, 19.5wt%, 20wt%, 32wt%, 41.5wt% or *51wt%.*

Preferably, in the drug-containing layer, when the pharmaceutical excipient comprises a pharmaceutical carrier and a filler, the weight ratio of the filler to the pharmaceutical carrier may be (1-6):1, and may further be (1-2): 1.

More preferably, when the pharmaceutical carrier is a mixture of povidone and hydroxypropyl cellulose or hydroxypropyl cellulose, and the filler is sorbitol, a mixture of sorbitol and lactose or a mixture of sorbitol and mannitol, then the weight ratio of sorbitol in the filler to hydroxypropyl cellulose in the pharmaceutical carrier is preferably (1-2):1, such as 1:1, 1.95:1 or 2:1.

Where the kind and content of the surfactant may be conventional in such preparations in the art, and the content is the weight percentage of this component in the drug-containing layer.

The surfactant may be one or more of polysorbate, poloxamer, fatty acid glyceride, sodium dodecylbenzenesulfonate and sodium dodecyl sulfate, such as poloxamer 407.

When the drug-containing layer comprises a pharmaceutical excipient and the pharmaceutical excipient comprises a surfactant, then the content of the surfactant may be ≤ 19wt%, but not 0, and may further be 5-19wt%, such as 5wt%, 10wt% or 19wt%.

Where the type and content of the lubricant may be conventional in such preparations in the art, and the content is the weight percentage of this component in the drug-containing layer.

The lubricant is preferably one or more of stearic acid, silica, magnesium stearate, calcium stearate, polyethylene glycol and sodium stearyl fumarate, more preferably magnesium stearate and/or silica.

When the drug-containing layer comprises a pharmaceutical excipient and the pharmaceutical excipient comprises a lubricant, then the content of the lubricant may be ≤ 2. 5wt%, but not 0, and may further be 1-2.5wt%, such as 1wt% or 2.5wt%.

Where the type and content of the antioxidant may be conventional in such preparations in the art, and the content is the weight percentage of this component in the drug-containing layer.

The antioxidant may be one or more of butylated hydroxytoluene, butylated hydroxyanisole, *tert*butylhydroquinone, propyl gallate, vitamin C and vitamin E, such as butylated hydroxytoluene.

When the drug-containing layer comprises a pharmaceutical excipient and the pharmaceutical excipient comprises an antioxidant, then the content of the antioxidant may be ≤ 1.0wt% but not 0, and may further be 0.1-0.5%, such as 0.33wt%.

In a preferred embodiment of the present disclosure, the drug-containing layer comprises any one of the following components:
(1) the active pharmaceutical ingredient, the pharmaceutical carrier and the lubricant;
(2) the active pharmaceutical ingredient, the pharmaceutical carrier, the filler and the lubricant;
(3) the active pharmaceutical ingredient, the pharmaceutical carrier, the filler, the surfactant and the lubricant;
(4) the active pharmaceutical ingredient, the pharmaceutical carrier, the filler, the surfactant, the antioxidant and the lubricant;
the amounts of each component are the same as described in the present disclosure.

In a preferred embodiment of the present disclosure, the drug-containing layer consists of any one of the following components:
(1) 14.5wt% carbidopa monohydrate, 53.6wt% levodopa, 31.0wt% hydroxypropyl cellulose and 1.0wt% magnesium stearate;
(2) 12.7wt% carbidopa monohydrate, 46.9wt% levodopa, 31wt% hydroxypropyl cellulose, 8.5wt% mannitol and 1wt% magnesium stearate;
(3) 11.3wt% carbidopa monohydrate, 41.7wt% levodopa, 31wt% hydroxypropyl cellulose, 15wt% mannitol and 0.5wt% magnesium stearate;
(4) 50wt% levodopa, 31.0wt% hydroxypropyl cellulose, 18wt% mannitol and 1wt% magnesium stearate;
(5) 63wt% levodopa, 11wt% hydroxypropyl cellulose, 10.0wt% sorbitol, 5wt% povidone, 10wt% poloxamer and 1wt% magnesium stearate;
(6) 31.5wt% levodopa, 20wt% hydroxypropyl cellulose, 19wt% sorbitol, 9.5wt% povidone, 19wt% poloxamer and 1wt% magnesium stearate;
(7) 31.5wt% levodopa, 29wt% hydroxypropyl cellulose, 19wt% sorbitol, 9.5wt% povidone, 10wt% poloxamer and 1wt% magnesium stearate;
(8) 63wt% levodopa, 10wt% hydroxypropyl cellulose, 19.5wt% sorbitol, 5wt% poloxamer, 0.5wt% silica and 2wt% magnesium stearate;
(9) 31.5wt% levodopa, 31wt% lactose, 10wt% hydroxypropyl cellulose, 20wt% sorbitol, 5wt% poloxamer 407, 0.5wt% silica and 2wt% magnesium stearate;
(10) 7.5wt% carbidopa monohydrate, 55.5wt% levodopa, 11wt% hydroxypropyl cellulose, 20wt% sorbitol, 5wt% poloxamer 407 and 1wt% magnesium stearate;
(11) 63.0wt% levodopa, 14.8wt% hydroxypropyl cellulose, 14.8wt% sorbitol, 5wt% poloxamer 407, 0.5% silica and 2.0wt% magnesium stearate;
(12) 63.0wt% levodopa, 11.8wt% hydroxypropyl cellulose, 17.7wt% sorbitol, 5wt% poloxamer 407, 0.5% silica and 2.0wt% magnesium stearate;
(13) 7.5wt% carbidopa monohydrate, 55.5wt% levodopa, 10wt% hydroxypropyl cellulose, 10wt% poloxamer 407, 10wt% sorbitol, 4.5wt% povidone, 2.0wt% magnesium stearate and 0.5wt% silica;
**(14)** 7.5wt% carbidopa monohydrate, 55.5wt% levodopa, 15wt% hydroxypropyl cellulose, 15wt% sorbitol, 4.17wt% povidone, 0.33wt% butylated hydroxytoluene, 2.0wt% magnesium stearate and 0.5wt% silica;
(15) 6.91wt% carbidopa monohydrate, 51.1wt% levodopa, 30wt% hydroxypropyl cellulose, 5wt% sorbitol, 4.19wt% povidone, 0.31wt% butylated hydroxytoluene, 0.5wt% silica and 2wt% magnesium stearate;
(16) 7.5wt% carbidopa monohydrate, 55.5wt% levodopa, 10wt% hydroxypropyl cellulose, 10wt% sorbitol, 10wt% poloxamer 407, 4.17wt% povidone, 0.33wt% butylated hydroxytoluene, 2.0wt% magnesium stearate and 0.5wt% silica;
(17) 7.5wt% carbidopa monohydrate, 55.5wt% levodopa, 15wt% hydroxypropyl cellulose, 15wt% sorbitol, 4.17wt% povidone, 0.33wt% butylated hydroxytoluene, 2.0wt% magnesium stearate and 0.5wt% silica;
(18) 3.75wt% carbidopa monohydrate, 27.7wt% levodopa, 31.5wt% mannitol, 10wt% hydroxypropyl cellulose, 10wt% poloxamer 407, 10wt% sorbitol, 4.5wt% povidone, 0.5wt% silica and 2wt% magnesium stearate;
(19) 3.75wt% carbidopa monohydrate, 27.7wt% levodopa, 10wt% hydroxypropyl cellulose, 10wt% poloxamer 407, 41.5wt% sorbitol, 4.5wt% povidone, 1.5wt% silica and 2wt% magnesium stearate.

In the present disclosure, the push layer may further comprise one or more of swelling agents, osmotic pressure enhancers, lubricants and colorants, and may further comprise a swelling agent, an osmotic pressure enhancer, a colorant and a lubricant.

The components and contents of the swelling agent, the osmotic pressure enhancer, the colorant and the lubricant may be conventional for such agents in the art, and the content is the weight percentage of this component in the push layer. The following components and contents are particularly preferred according to the present disclosure:
Where the swelling agent may be one or more of sodium carboxymethyl starch, hypromellose, hydroxypropyl cellulose, sodium carboxymethyl cellulose (such as sodium carboxymethyl cellulose 9H4XF), hydroxyethyl cellulose, carbomer, sodium alginate, carrageenan and polyoxyethylene, such as sodium carboxymethyl cellulose and hydroxypropyl cellulose.

When the swelling agent is sodium carboxymethyl cellulose and hydroxypropyl cellulose, the mass ratio of sodium carboxymethyl cellulose to hydroxypropyl cellulose is preferably (2-4):1, such as 2.5:1 or 3.5:1.

Where the content of the swelling agent may be 25-89wt%, such as 89wt% or 69wt%.

Where the osmotic pressure enhancer may be one or more of sodium chloride, potassium chloride, magnesium chloride, sodium sulfate, magnesium sulfate, ascorbic acid, tartaric acid, mannitol, sorbitol, xylitol, glucose, lactose and sucrose, such as sorbitol.

Where the content of the osmotic pressure enhancer may be 10-70wt%, and may further be 10-30wt%, such as 30wt%, and the content is the weight percentage of this component in the push layer.

Where the lubricant may be one or more of stearic acid, magnesium stearate, calcium stearate, polyethylene glycol and sodium stearyl fumarate, such as magnesium stearate.

Where the content of the lubricant may be 0.1-3wt%, such as 0.5wt%, and the content is the weight percentage of this component in the push layer.

Where the colorant may be one or more of iron oxide red, iron oxide yellow, iron oxide violet and iron oxide black, such as iron oxide red.

Where the content of the colorant may be 0.1-2wt%, such as 0.5wt%, and the content is the weight percentage of this component in the push layer.

In a preferred embodiment of the present disclosure, the push layer consists of the following components:
(1) 49wt% sodium carboxymethyl cellulose, 30wt% sorbitol, 20wt% hydroxypropyl cellulose, 0.5wt% iron oxide red and 0.5wt% magnesium stearate;
(2) 69wt% sodium carboxymethyl cellulose, 10wt% sorbitol, 20wt% hydroxypropyl cellulose, 0.5wt% red iron oxide and 0.5wt% magnesium stearate.

In the present disclosure, the weight ratio of the drug-containing layer to the push layer may be (0.5-4):1, and may further be (2-3):1, for example, 2:1, 2.5:1 or 2.8:1.

In a preferred embodiment of the present disclosure, the tablet core comprises any one of the following components:
(1) in the drug-containing layer: the active pharmaceutical ingredient, the pharmaceutical carrier and the lubricant; in the push layer: the swelling agent, the osmotic pressure enhancer, the colorant and the lubricant;
(2) in the drug-containing layer: the active pharmaceutical ingredient, the pharmaceutical carrier, the filler and the lubricant; in the push layer: the swelling agent, the osmotic pressure enhancer, the colorant and the lubricant;
(3) in the drug-containing layer: the active pharmaceutical ingredient, the pharmaceutical carrier, the filler, the surfactant and the lubricant; in the push layer: the swelling agent, the osmotic pressure enhancer, the colorant and the lubricant;
(4) in the drug-containing layer: the active pharmaceutical ingredient, the pharmaceutical carrier, the filler, the surfactant, the antioxidant and the lubricant; in the push layer: the swelling agent, the osmotic pressure enhancer, the colorant and the lubricant;
the contents of each component are the same as mentioned above.

In a preferred embodiment of the present disclosure, the tablet core consists of any one of the following components:
(1) in the drug-containing layer: 14.5wt% carbidopa monohydrate, 53.6wt% levodopa, 31.0wt% hydroxypropyl cellulose and 1.0wt% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(2) in the drug-containing layer: 12.7wt% carbidopa monohydrate, 46.9wt% levodopa, 31wt% hydroxypropyl cellulose, 8.5wt% mannitol and 1wt% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(3) in the drug-containing layer: 11.3wt% carbidopa monohydrate, 41.7wt% levodopa, 31wt% hydroxypropyl cellulose, 15wt% mannitol and 0.5wt% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(4) in the drug-containing layer: 50wt% levodopa, 31.0wt% hydroxypropyl cellulose, 18wt% mannitol and 1wt% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(5) in the drug-containing layer: 63wt% levodopa, 11wt% hydroxypropyl cellulose, 10.0wt% sorbitol, 5wt% povidone, 10wt% poloxamer and 1wt% magnesium stearate;
   49wt% sodium carboxymethyl cellulose, 30wt% sorbitol, 20wt% hydroxypropyl cellulose, 0.5wt% iron oxide red and 0.5wt% magnesium stearate;
(6) in the drug-containing layer: 31.5wt% levodopa, 20wt% hydroxypropyl cellulose, 19wt% sorbitol, 9.5wt% povidone, 19wt% poloxamer and 1wt% magnesium stearate;
   in the push layer: 69wt% sodium carboxymethyl cellulose, 10wt% sorbitol, 20wt% hydroxypropyl cellulose, 0.5wt% iron oxide red and 0.5wt% magnesium stearate;
(7) in the drug-containing layer: 31.5wt% levodopa, 29wt% hydroxypropyl cellulose, 19wt% sorbitol, 9.5wt% povidone, 10wt% poloxamer and 1wt% magnesium stearate;
   69wt% sodium carboxymethyl cellulose, 10wt% sorbitol, 20wt% hydroxypropyl cellulose, 0.5wt% iron oxide red and 0.5wt% magnesium stearate;
(8) in the drug-containing layer: 31.5wt% levodopa, 20wt% hydroxypropyl cellulose, 19wt% sorbitol, 9.5wt% povidone, 19wt% poloxamer and 1wt% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(9) in the drug-containing layer: 31.5wt% levodopa, 29wt% hydroxypropyl cellulose, 19wt% sorbitol, 9.5wt% povidone, 10wt% poloxamer and 1wt% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(10) in the drug-containing layer: 63wt% levodopa, 10wt% hydroxypropyl cellulose, 19.5wt% sorbitol, 5wt% poloxamer, 0.5wt% silica and 2.0wt% magnesium stearate;
   in the push layer: 69wt% sodium carboxymethyl cellulose, 10wt% sorbitol, 20wt% hydroxypropyl cellulose, 0.5wt% iron oxide red and 0.5wt% magnesium stearate;
(11) in the drug-containing layer: 31.5wt% levodopa, 31wt% lactose, 10wt% hydroxypropyl cellulose, 20wt% sorbitol, 5wt% poloxamer 407, 0.5wt% silica and 2wt% magnesium stearate;
   in the push layer: 69wt% sodium carboxymethyl cellulose 9H4XF, 10wt% sorbitol, 20wt% hydroxypropyl cellulose, 0.5wt% iron oxide red and 0.5wt% magnesium stearate;
(12) in the drug-containing layer: 7.5wt% carbidopa monohydrate, 55.5wt% levodopa, 11wt% hydroxypropyl cellulose, 20wt% sorbitol, 5wt% poloxamer 407 and 1wt% magnesium stearate;
   in the push layer: 69wt% sodium carboxymethyl cellulose 9H4XF, 10wt% sorbitol, 20wt% hydroxypropyl cellulose, 0.5wt% iron oxide red and 0.5wt% magnesium stearate;
(13) 63.0wt% levodopa, 14.8wt% hydroxypropyl cellulose, 14.8wt% sorbitol, 5wt% poloxamer 407, 0.5% silica and 2.0wt% magnesium stearate;
   in the push layer: 69wt% sodium carboxymethyl cellulose, 10wt% sorbitol, 20wt% hydroxypropyl cellulose, 0.5wt% iron oxide red and 0.5wt% magnesium stearate;
(14) 63.0wt% levodopa, 11.8wt% hydroxypropyl cellulose, 17.7wt% sorbitol, 5wt% poloxamer 407, 0.5% silica and 2.0wt% magnesium stearate;
   in the push layer: 69wt% sodium carboxymethyl cellulose, 10wt% sorbitol, 20wt% hydroxypropyl cellulose, 0.5wt% iron oxide red and 0.5wt% magnesium stearate;
(15) 7.50wt% carbidopa monohydrate, 55.51wt% levodopa, 10wt% hydroxypropyl cellulose, 10wt% poloxamer 407, 10wt% sorbitol, 4.50wt% povidone, 2.0wt% magnesium stearate and 0.5wt% silica;
   in the push layer: 49wt% sodium carboxymethyl cellulose, 30wt% sorbitol, 20wt% hydroxypropyl cellulose, 0.5wt% iron oxide red and 0.5wt% magnesium stearate;
(16) 7.5wt% carbidopa monohydrate, 55.5wt% levodopa, 15wt% hydroxypropyl cellulose, 15wt% sorbitol, 4.17wt% povidone, 0.33wt% butylated hydroxytoluene, 2.0wt% magnesium stearate and 0.5wt% silica;
   in the push layer: 49wt% sodium carboxymethyl cellulose, 30wt% sorbitol, 20wt% hydroxypropyl cellulose, 0.5wt% iron oxide red and 0.5wt% magnesium stearate;
(17) 6.91wt% carbidopa monohydrate, 51.1wt% levodopa, 30wt% hydroxypropyl cellulose, 5wt% sorbitol, 4.19wt% povidone, 0.31wt% butylated hydroxytoluene, 0.5wt% silica and 2wt% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(18) 7.5wt% carbidopa monohydrate, 55.5wt% levodopa, 10wt% hydroxypropyl cellulose, 10wt% poloxamer, 10wt% sorbitol, 4.17wt% povidone, 0.33wt% butylated hydroxytoluene, 2wt% magnesium stearate and 0.5wt% silica;
   in the push layer: 49wt% sodium carboxymethyl cellulose, 30wt% sorbitol, 20wt% hydroxypropyl cellulose, 0.5wt% iron oxide red and 0.5wt% magnesium stearate;
(19) 7.5wt% carbidopa monohydrate, 55wt% levodopa, 15wt% hydroxypropyl cellulose, 15wt% sorbitol, 4.17wt% povidone, 0.33wt% butylated hydroxytoluene, 0.5wt% silica and 2wt% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(20) 3.75wt% carbidopa monohydrate, 27.7wt% levodopa, 31.5wt% mannitol, 10wt% hydroxypropyl cellulose, 10wt% poloxamer 407, 10wt% sorbitol, 4.5wt% povidone, 1.5wt% silica and 2% magnesium stearate;
   in the push layer: 49wt% sodium carboxymethyl cellulose, 30wt% sorbitol, 20wt% hydroxypropyl cellulose, 0.5wt% iron oxide red and 0.5wt% magnesium stearate;
(21) 3.75wt% carbidopa monohydrate, 27.7wt% levodopa, 10wt% hydroxypropyl cellulose, 10wt% poloxamer 407, 41.5wt% sorbitol, 4.5wt% povidone, 1.5wt% silica and 2wt% magnesium stearate;
in the push layer: 49wt% sodium carboxymethyl cellulose, 30wt% sorbitol, 20wt% hydroxypropyl cellulose, 0.5wt% iron oxide red and 0.5wt% magnesium stearate.

In the present disclosure, the tablet core may be a two-layer tablet (consisting of a drug-containing layer and a push layer stacked on the drug-containing layer) or a three-layer tablet (consisting of a blank layer, a drug-containing layer and a push layer in turn), such as a two-layer tablet.

In the present disclosure, the tablet core may further comprise a controlled-release membrane coating coated on the tablet core.

Where the controlled-release membrane coating may further have one or more drug-release holes (the drug-release holes are punched by laser punching or mechanical punching, such as one drug-release hole, two drug-release holes or three drug-release holes). Preferably, the diameter of the drug-release hole is 0.3 mm-1.2 mm, such as 1.0 mm.

Where the controlled-release membrane coating is a semi-permeable coating membrane, and the membrane-forming material of the semi-permeable coating membrane may be one or more of cellulose acetate, ethyl cellulose and acrylic resin, such as cellulose acetate. The content of the membrane-forming material may be 50-90wt%, and may further be 60-90wt% (such as 60wt%, 65wt%, 70wt% or 90wt%), and the content is the weight percentage of this component in the controlled-release membrane coating.

Preferably, the semi-permeable coating membrane may further comprise a porogen and a plasticizer.

Where the porogen may be one or more of polyethylene glycol, glycerol, povidone, copovidone and hydroxypropyl cellulose, such as copovidone and hydroxypropyl cellulose (hydroxypropyl cellulose HXF).

Where the plasticizer may be one or more of polyethylene glycol, methyl phthalate, ethyl phthalate, dibutyl sebacate, triethyl citrate, tributyl citrate, acetyl tributyl citrate, glycerine acetate and castor oil, such as polyethylene glycol or triethyl citrate.

Where the semi-permeable coating membrane may comprise a mixture of cellulose acetate and copovidone, a mixture of "cellulose acetate, hydroxypropyl cellulose HXF and triethyl citrate" or a mixture of "cellulose acetate and triethyl citrate".

Where the weight ratio of the controlled-release membrane coating to the tablet core may be conventional in the art, and may be (2.0%-15.0%):1 (*e.g.,* 6.6%:1, 6.9%:1, 7.0%:1, 7.4%:1, 7.6%:1, 7.7%:1, 7.8%:1, 8.0%:1 or 8.4%:1); it may further be (6.6%-8.4%):1, and may further be (7.4%-7.8%):1.

In the present disclosure, an isolation coating layer may further be comprised between the tablet core and the controlled-release membrane coating (the isolation coating layer is coated on the tablet core, and the controlled-release membrane coating is coated on the isolation coating layer)

Where the membrane-forming material of the isolation coating layer may be hydroxypropyl cellulose EF.

Where the weight ratio of the isolation coating layer to the tablet core may be conventional in the art, and may be (3.0%-5.0%):1, such as 4.0%:1.

In the present disclosure, the pharmaceutical composition may further comprise an immediate-release layer comprising an active pharmaceutical ingredient coated on the controlled-release membrane coating. The pharmaceutical composition may further comprise an immediate-release layer. The immediate-release layer preferably comprises an active pharmaceutical ingredient, a binder and an antioxidant. The immediate-release layer further preferably comprises an active pharmaceutical ingredient, a plasticizer, an antioxidant and a binder. The immediate-release layer is further preferably composed of an active pharmaceutical ingredient, an antioxidant and a binder, or is composed of an active pharmaceutical ingredient, a plasticizer, an antioxidant and a binder.

When the immediate-release layer comprises a plasticizer, the content of the plasticizer is conventional in the art, preferably 1-2wt%.

When the immediate-release layer comprises a plasticizer, the plasticizer may be one or more of polyethylene glycol, methyl phthalate, ethyl phthalate, dibutyl sebacate, triethyl citrate, tributyl citrate, acetyl tributyl citrate, glycerine acetate and castor oil, such as triethyl citrate.

When the immediate-release layer comprises an antioxidant, the content of the antioxidant is conventional in the art, preferably 3-4wt%, such as 3.38wt% or 3.81wt%.

When the immediate-release layer comprises an antioxidant, the antioxidant may be one or more of butylated hydroxytoluene, butylated hydroxyanisole, dibutylphenol, vitamin C, vitamin E and sodium sulfite, such as butylated hydroxytoluene.

When the immediate-release layer comprises a binder, the content of the binder is conventional in the art, preferably 10-20wt%.

When the immediate-release layer comprises a binder, the binder may be one or more of povidone, copovidone, hydroxypropyl cellulose and hypromellose, such as a mixture of hydroxypropyl cellulose and copovidone (the weight ratio of the two is, for example, 1:1) or hydroxypropyl cellulose.

When the immediate-release layer comprises an active pharmaceutical ingredient, the content of the active pharmaceutical ingredient is conventional in the art, preferably 70-90wt%, such as 74.7wt%, 75.7wt%, 86.2wt% or 85.3wt%.

When the immediate-release layer comprises an active pharmaceutical ingredient,the active pharmaceutical ingredient comprises a dopa decarboxylase inhibitor. Preferably, the dopa decarboxylase inhibitor is a carbidopa hydrate, a pharmaceutically acceptable salt of carbidopa, benserazide or a pharmaceutically acceptable salt of benserazide, preferably a carbidopa hydrate, such as carbidopa monohydrate.

In a preferred embodiment of the present disclosure, the immediate-release layer consists of any one of the following components:
(a) 86.2wt% carbidopa monohydrate, 10.0wt% hydroxypropyl cellulose and 3.84wt% butylated hydroxytoluene;
(b) 85.3wt% carbidopa monohydrate, 10.0wt% hydroxypropyl cellulose, 1.02wt% triethyl citrate and 3.81wt% butylated hydroxytoluene;
(c) 74.7wt% carbidopa monohydrate, 10.0wt% hydroxypropyl cellulose, 10.0wt% copovidone, 2.00wt% triethyl citrate and 3.38wt% butylated hydroxytoluene;
(d) 75.7wt% carbidopa monohydrate, 10.0wt% hydroxypropyl cellulose, 10.0wt% copovidone, 1.00wt% triethyl citrate and 3.38wt% butylated hydroxytoluene.

In the present disclosure, the pharmaceutical composition may further comprise an enteric layer coated on the controlled-release membrane coating or the immediate-release layer. The material of the enteric layer may be an acrylic resin or a cellulose derivative. The acrylic resin is preferably one or more of a copolymer obtained by copolymerizing methacrylic acid and ethyl acrylate at a weight ratio of 1:1, a copolymer obtained by copolymerizing methacrylic acid and methyl methacrylate at a weight ratio of 1:1, a copolymer obtained by copolymerizing methacrylic acid and ethyl acrylate at a weight ratio of 1:2, and a copolymer obtained by copolymerizing methacrylic acid, methyl acrylate and methyl methacrylate at a weight ratio of 1:1:1. The cellulose derivative is preferably one or more of cellulose acetate phthalate, hypromellose titanate and hypromellose acetate succinate.

In the present disclosure, the shape of the capsule-shaped tablet may be a capsule shape formed by compressing in the long-axis direction or a capsule shape formed by compressing in a direction perpendicular to the long axis, for example, a capsule shape formed by compressing in the long-axis direction.

In the present disclosure, the cross-sectional shape of the capsule-shaped tablet may be a circular shape or a non-circular shape, such as a circular shape.

Where when the cross-sectional shape of the capsule-shaped tablet is a circular shape, the circular shape may have a diameter of 5-10 mm (*e.g.,* 6 mm, 7 mm or 8 mm) and a height of 4-30 mm (*e.g.,* 14.90 mm, 15.24 mm, 16.42 mm or 16.76 mm); the cross-sectional diameter may further be 5-7.5 mm and the height is 10-20 mm.

Where when the cross-sectional shape of the capsule-shaped tablet is a non-circular shape, the non-circular shape may be a non-circular shape with a symmetry axis. The length of the long axis of the non-circular shape with the symmetry axis may be 10-25 mm, and the length of the short axis may be 5-25 mm; the length of the long axis of the non-circular shape with the symmetry axis may further be 16-19 mm, and the length of the short axis may further be 7-7.5 mm.

In a preferred embodiment of the present disclosure, the pharmaceutical composition is any one of the following groups:
(1) in the drug-containing layer: the active pharmaceutical ingredient, the pharmaceutical carrier and the lubricant; in the push layer: the swelling agent, the osmotic pressure enhancer, the colorant and the lubricant; the cross-sectional shape of the capsule-shaped tablet is a non-circular shape (or the shape of the capsule-shaped tablet is a capsule shape formed by compressing in a direction perpendicular to the long axis);
(2) in the drug-containing layer: the active pharmaceutical ingredient, the pharmaceutical carrier, the filler and the lubricant; in the push layer: the swelling agent, the osmotic pressure enhancer, the colorant and the lubricant; the cross-sectional shape of the capsule-shaped tablet is a non-circular shape (or the shape of the capsule-shaped tablet is a capsule shape formed by compressing in a direction perpendicular to the long axis);
(3) in the drug-containing layer: the active pharmaceutical ingredient, the pharmaceutical carrier, the filler, the surfactant and the lubricant; in the push layer: the swelling agent, the osmotic pressure enhancer, the colorant and the lubricant; the cross-sectional shape of the capsule-shaped tablet is a non-circular shape (or the shape of the capsule-shaped tablet is a capsule shape formed by compressing in a direction perpendicular to the long-axis);
(4) in the drug-containing layer: the active pharmaceutical ingredient, the pharmaceutical carrier, the filler, the antioxidant and the lubricant; in the push layer: the swelling agent, the osmotic pressure enhancer, the colorant and the lubricant; the cross-sectional shape of the capsule-shaped tablet is a non-circular shape (or the shape of the capsule-shaped tablet is a capsule shape formed by compressing in a direction perpendicular to the long axis);
(5) in the drug-containing layer: the active pharmaceutical ingredient, the pharmaceutical carrier, the filler and the lubricant; in the push layer: the swelling agent, the osmotic pressure enhancer, the colorant and the lubricant; the cross-sectional shape of the capsule-shaped tablet is a circular shape (or the shape of the capsule-shaped tablet is a capsule shape formed by compressing in the long-axis direction);
(6) in the drug-containing layer: the active pharmaceutical ingredient, the pharmaceutical carrier, the filler, the surfactant and the lubricant; in the push layer: the swelling agent, the osmotic pressure enhancer, the colorant and the lubricant; the cross-sectional shape of the capsule-shaped tablet is a circular shape (or the shape of the capsule-shaped tablet is a capsule shape formed by compressing in the long-axis direction);
(7) in the drug-containing layer: the active pharmaceutical ingredient, the pharmaceutical carrier, the filler, the surfactant, the antioxidant and the lubricant; in the push layer: the swelling agent, the osmotic pressure enhancer, the colorant and the lubricant; the cross-sectional shape of the capsule-shaped tablet is a circular shape (or the shape of the capsule-shaped tablet is a capsule shape formed by compressing in the long-axis direction);
the contents of each component are the same as mentioned above.

In a preferred embodiment of the present disclosure, the pharmaceutical composition is the following group (I) or (II):
(I) in the drug-containing layer: the active pharmaceutical ingredient, the pharmaceutical carrier, the filler, the antioxidant and the lubricant; in the push layer: the swelling agent, the osmotic pressure enhancer, the colorant and the lubricant;
   in the immediate-release layer: the active pharmaceutical ingredient, the binder and the oxidant;
   the cross-sectional shape of the capsule-shaped tablet is a circular shape (or the shape of the capsule-shaped tablet is a capsule shape formed by compressing in the long-axis direction);
(II) in the drug-containing layer: the active pharmaceutical ingredient, the pharmaceutical carrier, the filler, the antioxidant and the lubricant; in the push layer: the swelling agent, the osmotic pressure enhancer, the colorant and the lubricant;
   in the immediate-release layer: the active pharmaceutical ingredient, the binder, the oxidant and the plasticizer;
   the cross-sectional shape of the capsule-shaped tablet is a circular shape (or the shape of the capsule-shaped tablet is a capsule shape formed by compressing in the long-axis direction).

In a preferred embodiment of the present disclosure, in the drug-containing layer, the active pharmaceutical ingredient is levodopa; the content of the levodopa is 27.7-63wt%;
the dopa decarboxylase inhibitor is carbidopa or a hydrate thereof; the content of the dopa decarboxylase inhibitor is 7.5-14.5wt%;
the drug-containing layer comprises a pharmaceutical excipient, and the pharmaceutical excipient comprises " a pharmaceutical carrier, a filler and a lubricant", "a pharmaceutical carrier, a filler, a surfactant and a lubricant" or "a pharmaceutical carrier, a filler, a surfactant, an antioxidant and a lubricant";
the pharmaceutical carrier is "a mixture of povidone and hydroxypropyl cellulose" or hydroxypropyl cellulose; the content of the pharmaceutical carrier is 10-40wt%; the filler is a mixture of sorbitol and lactose, a mixture of sorbitol and mannitol, or sorbitol; the content of the filler is 8.5-20wt%; the weight ratio of sorbitol in the filler to hydroxypropyl cellulose in the pharmaceutical carrier is (1-2):1;
the surfactant is poloxamer; the content of the surfactant is 5-19wt%;
the lubricant is magnesium stearate and/or silica; the content of the lubricant is 1-2.5wt%;
the antioxidant is butylated hydroxytoluene; the content of the antioxidant is 0.1-1.0%;
the shape of the capsule-shaped tablet is a capsule shape formed by compressing in the long-axis direction (or the cross-sectional shape of the capsule-shaped tablet is a circular shape).

Preferably, the weight ratio of the controlled-release membrane coating to the tablet core is (7.0%-8.0%): 1, and may further be (7.4%-7.8%): 1.

In the present disclosure, the capsule-shaped tablet is preferably a controlled-release tablet, such as an osmotic pump tablet. The levodopa in the controlled-release tablet is preferably 125 mg/tablet, 150 mg/tablet, 250 mg/tablet, 375 mg/tablet or 500 mg/tablet. In the present disclosure, the pharmaceutical composition is preferably administered before going to bed and lying down after administering.

In a preferred embodiment of the present disclosure, the drug-containing layer of the pharmaceutical composition consists of the active pharmaceutical ingredient and the pharmaceutical excipient.

The present disclosure further provides a preparation method for the pharmaceutical composition, which comprises the following steps:
stacking and composite compressing the raw material particles of the drug-containing layer and the raw material particles of the push layer to form the tablet core;
when the tablet core is further coated with a controlled-release membrane coating, coating is performed on the tablet core, *i.e.,* the controlled-release membrane coating is coated on the tablet core, and the controlled-release membrane coating on one side of the drug-containing layer is punched to form a drug-release hole.

When the pharmaceutical composition may further comprise an immediate-release layer, the immediate-release layer is coated on the capsule-shaped tablet coating the controlled-release membrane coating.

When the pharmaceutical composition further comprises an isolation coating layer, the isolation coating layer is first coated on the tablet core and then the coating is coated on the isolation coating layer.

Where the composite compressing may be performed in the long-axis direction or in a direction perpendicular to the long axis.

Where the raw material particles of the drug-containing layer may be obtained by a conventional granulation method in the art, such as dry granulation, wet granulation, hot melt granulation or fluidized bed granulation.

Where the raw material particles of the push layer may be obtained by a conventional granulation method in the art, such as dry granulation, wet granulation or fluidized bed granulation to obtain the raw material particles of the drug-containing layer.

The present disclosure further provides use of the pharmaceutical composition in the manufacture of a medicament for preventing or treating morning akinesia.

In use, the morning akinesia is preferably morning akinesia caused by Parkinson's disease.

Preferably, the pharmaceutical composition starts to release the active pharmaceutical ingredient with a time lag of 1-3 hours, and the active pharmaceutical ingredient reaches the peak concentration in 6-10 hours, and the time for which the blood drug concentration is maintained at 50% or higher of the peak concentration is 5 hours or more; more preferably, the pharmaceutical composition starts to release the active pharmaceutical ingredient with a time lag of 2 hours, and the active pharmaceutical ingredient reaches the peak concentration in 6-10 hours, and the time for which the blood drug concentration is maintained at 50% or higher of the peak concentration is 7 hours or more.

The release characteristics of the pharmaceutical composition are preferably: a time lag of 1-3 hours before the start of the release (during this time, the cumulative release rate of the active pharmaceutical ingredient is ≤ 10%); after the start of the release, the active pharmaceutical ingredient has a cumulative release rate of 85% or more (including 85%) for 6-10 hours; more preferably, the active pharmaceutical ingredient has a time lag of 2 hours before the start of the release (during this time, the cumulative release rate of the active pharmaceutical ingredient is ≤ 10%); the active pharmaceutical ingredient has a cumulative release rate of 85% or more (including 85%) for 8 hours after the start of the release.

The present disclosure further provides a delayed timed release pharmaceutical composition, wherein the dosage form of the pharmaceutical composition is a controlled-release tablet;
the composition comprises an active pharmaceutical ingredient, wherein the active pharmaceutical ingredient is levodopa or a derivative thereof, or a mixture of "levodopa or a derivative thereof' and a dopa decarboxylase inhibitor; the release (*in vitro*) characteristic of the composition is that the active pharmaceutical ingredient has a time lag of 1-3 hours before the start of the release; after the start of the release, the active pharmaceutical ingredient has a cumulative release rate of 85% or more (including 85%) for 6-10 hours; the time lag means that the cumulative release rate of the active pharmaceutical ingredient is ≤ 10% during this time.

Preferably, the blood drug concentration of the active pharmaceutical ingredient is maintained at 50% or higher of the peak concentration for 5 hours or more; more preferably, the blood drug concentration of the active pharmaceutical ingredient is maintained at 50% or higher of the peak concentration for 7 hours or more.

The release characteristics of the pharmaceutical composition are preferably: a time lag of 2 hours of the active pharmaceutical ingredient before the start of the release; the active pharmaceutical ingredient has a cumulative release rate of 85% or more (including 85%) for 8 hours after the start of the release.

Where the pharmaceutical composition is preferably administered before going to bed and lying down after administering. The pharmaceutical composition is preferably the above pharmaceutical composition. The pharmaceutical composition is preferably used for preventing or treating morning akinesia. The morning akinesia is preferably morning akinesia caused by Parkinson's disease.

Where the components of the pharmaceutical composition are the same as mentioned above.

The present disclosure further provides a method for preventing or treating morning akinesia, comprising administering to a subject an effective amount of the pharmaceutical composition.

In the method, the morning akinesia is preferably morning akinesia caused by Parkinson's disease. The pharmaceutical composition is preferably administered before going to bed and lying down after administering.

In the present disclosure, "long-axis direction" refers to a direction parallel to the longest symmetry axis among the symmetry axes of the capsule-shaped tablet.

In the present disclosure, the "cross section" refers to a plane parallel to the interface formed by the drug-containing layer and the push layer.

Without departing from common knowledge in the art, the above preferred conditions can be arbitrarily combined to obtain preferred examples of the present disclosure.

The reagents and starting materials used in the present disclosure are commercially available.

The positive and progressive effects of the present disclosure are that the pharmaceutical composition of the present disclosure has the following excellent controlled-release effects: *in vitro* delay of 1-3 hours, a cumulative release rate of the active pharmaceutical ingredient being 10% or less; sustained release for 6-10 hours, a cumulative release rate being 85% or more for 6-10 hours, further achieving a delay of 2 hours *in vitro*; a cumulative release rate of the active pharmaceutical ingredient being 10% or less, sustained release for 8-10 hours, and a cumulative release rate being 85% or more for 8-10 hours;
a time lag of 1-3 hours *in vivo,* reaching a peak concentration in 6-10 hours, maintaining the blood drug concentration at 50% or higher of the peak concentration for 5 hours or more, and especially starting to release after a lag of 2 hours;
the pharmaceutical composition of the present disclosure can achieve that the patient self-takes a drug before going to bed the night before, maintains the blood drug concentration above the lowest effective concentration before waking up in the morning, makes the patient in the "ON" state instead of the "OFF" state when waking up in the morning, and reduces the occurrence of morning akinesia and other related symptoms caused thereby.

### BRIEF DESCRIPTION OF THE DRAWINGS

FIG. 1 is a graph of the release profile of levodopa in the controlled-release tablet of Example 1.
FIG. 2 is a graph of the release profile of carbidopa in the controlled-release tablet of Example 1.
FIG. 3 is a graph of the release profile of levodopa in the controlled-release tablet of Example 2.
FIG. 4 is a graph of the release profile of levodopa in the controlled-release tablet of Example 3.
FIG. 5 is a graph of the release profile of levodopa in the controlled-release tablet of Example 4.
FIG. 6 is a graph of the release profile of carbidopa in the controlled-release tablet of Example 4.
FIG. 7 is a graph of the release profile of levodopa in the controlled-release tablet of Example 5.
FIG. 8 is a graph of the release profile of levodopa in the controlled-release tablet of Example 6.
FIG. 9 is a graph of the release profile of levodopa in the controlled-release tablet of Example 7.
FIG. 10 is a graph of the release profile of levodopa in the controlled-release tablet of Example 8.
FIG. 11 is a graph of the release profile of levodopa in the controlled-release tablet of Example 9.
FIG. 12 is a graph of the release profile of levodopa in the controlled-release tablet of Example 10.
FIG. 13 is a graph of the release profile of levodopa in the controlled-release tablet of Example 11.
FIG. 14 is a graph of the release profile of levodopa in the controlled-release tablet of Example 14.
FIG. 15 is a graph of the release profile of levodopa in the controlled-release tablet of Example 15.
FIG. 16 is a graph of the release profile of levodopa in the controlled-release tablet of Example 16.
FIG. 17 is a graph of the release profile of carbidopa in the controlled-release tablet of Example 16.
FIG. 18 is a graph of the release profile of levodopa in the controlled-release tablet of Example 17.
FIG. 19 is a graph of the release profile of levodopa in the controlled-release tablet of Example 18.
FIG. 20 is a graph of the release profile of levodopa in the controlled-release tablet of Example 19.
FIG. 21 is a graph of the release profile of carbidopa in the controlled-release tablet of Example 19.
FIG. 22 is a graph of the release profile of levodopa in the controlled-release tablet of Example 21.
FIG. 23 is a graph of the release profile of carbidopa in the controlled-release tablet of Example 21.
FIG. 24 is a graph of the release profile of levodopa in the controlled-release tablet of Example 22.
FIG. 25 is a graph of the release profile of carbidopa in the controlled-release tablet of Example 22.
FIG. 26 is a graph of the release profile of levodopa in the controlled-release tablet of Example 23.
FIG. 27 is a graph of the release profile of carbidopa in the controlled-release tablet of Example 23.
FIG. 28 is a graph of the release profile of levodopa in the controlled-release tablet of Example 24.
FIG. 29 is a graph of the release profile of carbidopa in the controlled-release tablet of Example 24.
FIG. 30 is a graph of the release profile of levodopa in the controlled-release tablet of Example 25.
FIG. 31 is a graph of the release profile of carbidopa in the controlled-release tablet of Example 25.
FIG. 32 is a graph of the release profile of levodopa in the controlled-release tablet of Example 26.
FIG. 33 is a graph of the release profile of carbidopa in the controlled-release tablet of Example 26.
FIG. 34 is a graph of the release profile of levodopa in the controlled-release tablet of Example 27.
FIG. 35 is a graph of the release profile of carbidopa in the controlled-release tablet of Example 27.

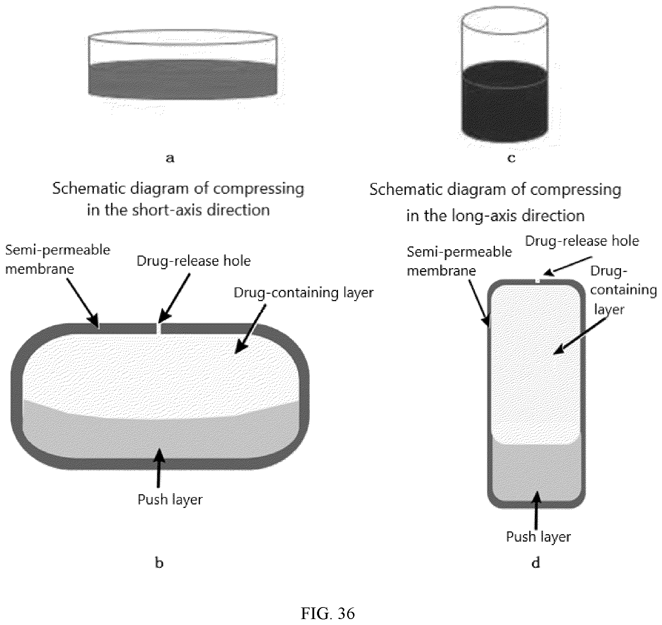

FIG. 36 is a shape diagram of the controlled-release tablet of the present disclosure, wherein a is a three-dimensional structural diagram of the controlled-release tablet obtained by prescription III in Example 1; b is the right side view of the controlled-release tablet obtained by prescription III in Example 1; wherein c is a three-dimensional structural diagram of the controlled-release tablet in Example 11; d is the right side view of the controlled-release tablet in Example 11.

### DETAILED DESCRIPTION OF THE PREFERRED EMBODIMENT

Hereinafter, the present disclosure will be further described by way of examples, but the present disclosure is not limited to the scope of the described examples. The experimental methods for which specific conditions are not specified in the following examples are selected according to the conventional methods and conditions, or according to the commercial instructions. In the following examples, "proportion wt%" refers to the weight percentage of the weight of this component to the total weight of the prescription of the layer in which the component is located. For example, in prescription I of Example 1, the proportion of carbidopa is 14.5wt%, which refers to the weight percentage of carbidopa to the total weight of the drug-containing layer prescription. Unless otherwise specified, mannitol is mannitol 200SD, hydroxypropyl cellulose is hydroxypropyl cellulose EXF, poloxamer is poloxamer 407 ≤ 80 mesh, sorbitol is sorbitol ≤ 80 mesh, povidone is povidone K29/32, and cellulose acetate is cellulose acetate 320S.

### Example 1: Capsule-shaped tablet (with a non-circular cross section, and compressed in a direction perpendicular to the long axis) with a specification of 37.5 mg/150 mg (carbidopa/levodopa), screened for mannitol ratio

The prescription is as follows:

| (1) | Drug-containing layer prescription (per tablet) | Prescription I | | Prescription II | | Prescription III | |
|---|---|---|---|---|---|---|---|
| | | mg/tablet | Proportion wt% | mg/tablet | Proportion wt% | mg/tablet | Proportion wt% |
| | Carbidopa monohydrate | 40.5 | 14.5 | 40.5 | 12.7 | 40.5 | 11.3 |
| | Levodopa | 150.0 | 53.6 | 150.0 | 46.9 | 150.0 | 41.7 |
| | Hydroxypropyl cellulose | 86.7 | 31.0 | 99.2 | 31.0 | 111.6 | 31.0 |
| | Mannitol | 0.0 | 0.0 | 27.1 | 8.5 | 54.3 | 15.0 |
| | Magnesium stearate | 2.8 | 1.0 | 3.2 | 1.0 | 3.6 | 0.5 |

| (2) | Push layer prescription (per tablet) | mg/tablet | Proportion wt% | mg/tablet | Proportion wt% | mg/tablet | Proportion wt% |
|---|---|---|---|---|---|---|---|
| | Sodium carboxymethyl cellulose | 68.6 | 49.0 | 78.4 | 49.0 | 88.2 | 49.0 |
| | Sorbitol | 42.0 | 30.0 | 48.0 | 30.0 | 54.0 | 30.0 |
| | Hydroxypropyl cellulose | 28.0 | 20.0 | 32.0 | 20.0 | 36.0 | 20.0 |
| | Iron oxide red | 0.70 | 0.50 | 0.80 | 0.50 | 0.9 | 0.50 |
| | Magnesium stearate | 0.70 | 0.50 | 0.80 | 0.50 | 0.9 | 0.50 |

| (3) | Controlled-release membrane prescription | | | | | | |
|---|---|---|---|---|---|---|---|
| | Cellulose acetate | 21.0 g | | | | | |
| | Copovidone | 9.00 g | | | | | |
| | Acetone | 675 mL | | | | | |
| | Ethanol | 75 mL | | | | | |

### Preparation method:

1. Preparation of the drug-containing layer: Carbidopa and levodopa were premixed with other excipients of the drug-containing layer (except magnesium stearate), added to a dry granulator and compressed into strips, granulated through a 16-mesh stainless steel screen, and finally added with magnesium stearate and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 16 * 7 mm (deep concave); the drug-containing layer materials were first filled and pre-compressed, followed by the filling of the push layer materials, and the final compression was performed to obtain a two-layer tablet core compressed in a direction perpendicular to the long axis.
4. Semi-permeable membrane coating: The two-layer tablet core was coated with the above controlled-release coating solution using a coating pan to a weight gain of 5.5wt% (percentages are weight percentages relative to the tablet core). The holes were punched using mechanical punching or laser punching, and the diameter of the drug-release hole was 1.0 mm. The specification of the controlled-release tablet in this example was 37.5 mg/150 mg (carbidopa/levodopa), a capsule-shaped biconvex tablet.
5. Dissolution test method: According to the dissolution test methods (USP <711> Apparatus 2 and Chinese Pharmacopoeia <0931>), the tablet was placed in a sinker, using 900 mL of 0.1 N hydrochloric acid solution as the dissolution medium, with the rotation speed of 75 revolutions per minute, and the operation was performed according to the dissolution test methods. At 0.5 hours, 1 hour, 2 hours, 3 hours, 4 hours, 6 hours, 8 hours, 10 hours and 12 hours, 2 mL of the online-filtered dissolution medium (rinsed online with 10 mL of the medium prior to sampling) was immediately taken as test solutions (1), (2), (3), (4), (5), (6), (7), (8) and (9), and 2 mL of 0.1 N hydrochloric acid solution at 37°C was immediately added to the dissolution vessel.

Chromatographic conditions: According to the high-performance liquid chromatography (USP <621> and Chinese Pharmacopoeia <0512>), octadecylsilane-bonded silica gel was used as the filler; methanol-phosphate buffered saline (pH 2.1) (5:95) was used as the mobile phase, the flow rate was 1.0 mL/min, the detection wavelength was 280 nm, the column temperature was 35°C, and the sample chamber temperature was 6°C.

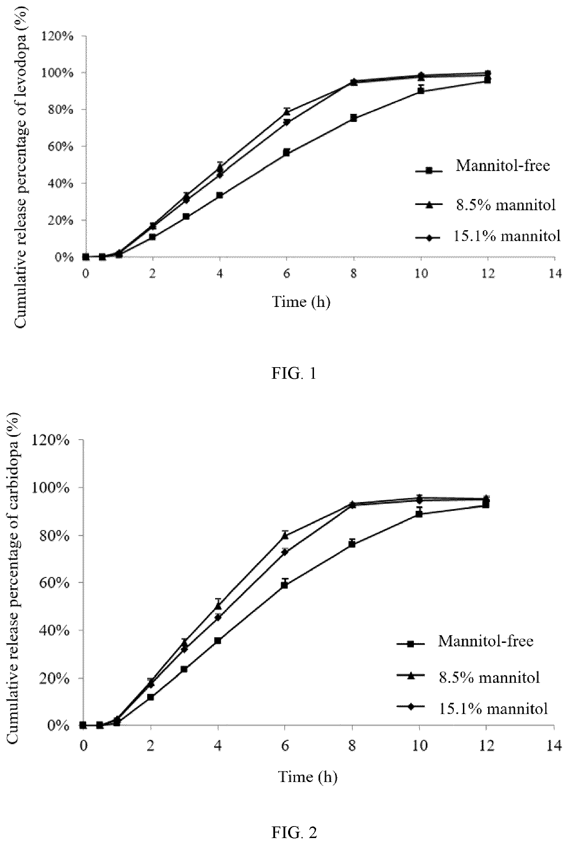

### Specific levodopa test results are shown in Table 1 and FIG. 1, and carbidopa test results are shown in Table 2 and FIG. 2.

**Table 1**

| Sampling point/h | Mannitol-free | SD | 8.5% mannitol | SD | 15.1% mannitol | SD |
|---|---|---|---|---|---|---|
| 0 | 0.0% | 0.0% | 0.0% | 0.0% | 0.0% | 0.0% |
| 0.5 | 0.1% | 0.0% | 0.1% | 0.0% | 0.1% | 0.1% |
| 1 | 1.1% | 0.5% | 2.4% | 0.4% | 2.3% | 0.6% |
| 2 | 10.7% | 0.2% | 17.1% | 1.2% | 16.5% | 1.8% |
| 3 | 21.7% | 0.8% | 33.5% | 1.4% | 30.9% | 1.8% |
| 4 | 33.3% | 0.6% | 48.9% | 2.5% | 44.6% | 2.0% |
| 6 | 56.0% | 2.5% | 78.7% | 2.0% | 72.9% | 1.7% |
| 8 | 74.9% | 2.5% | 94.8% | 0.8% | 95.4% | 0.6% |
| 10 | 89.9% | 3.4% | 97.7% | 0.8% | 98.7% | 0.8% |
| 12 | 95.6% | 1.7% | 98.4% | 0.7% | 99.9% | 1.1% |

**Table 2**

| Sampling point/h | Mannitol-free | SD | 8.5% mannitol | SD | 15.1% mannitol | SD |
|---|---|---|---|---|---|---|
| 0 | 0.0% | 0.0% | 0.0% | 0.0% | 0.0% | 0.0% |
| 0.5 | 0.0% | 0.0% | 0.0% | 0.0% | 0.0% | 0.0% |
| 1 | 0.8% | 0.8% | 2.6% | 0.5% | 2.5% | 0.8% |
| 2 | 11.6% | 0.3% | 18.3% | 1.3% | 17.2% | 1.9% |
| 3 | 23.3% | 0.8% | 34.9% | 1.6% | 31.7% | 1.7% |
| 4 | 35.5% | 0.7% | 50.4% | 2.7% | 45.1% | 1.9% |
| 6 | 58.8% | 2.8% | 79.7% | 2.2% | 72.8% | 1.6% |
| 8 | 75.9% | 2.4% | 93.0% | 0.5% | 92.5% | 0.9% |
| 10 | 88.8% | 3.0% | 95.7% | 1.0% | 94.5% | 1.2% |
| 12 | 92.6% | 1.9% | 95.3% | 0.7% | 94.8% | 1.5% |

### Example 2: Capsule-shaped tablet (with a non-circular cross section, and compressed in a direction perpendicular to the long axis) with a specification of 37.5 mg/150 mg (carbidopa/levodopa), with an isolation coating

The prescription is as follows:

| (1) | Drug-containing layer prescription (per tablet) | Same as prescription II in Example 1 |
|---|---|---|
| (2) | Push layer prescription (per tablet) | Same as prescription II in Example 1 |
| (3) | Isolation coating prescription | |
| | Hydroxypropyl cellulose EF | 25 g |
| | Ethanol | 475 g |

| (4) | Controlled-release membrane prescription | |
|---|---|---|
| | Cellulose acetate | 28.0 g |
| | Copovidone | 12.0 g |
| | Acetone | 900 mL |
| | Ethanol | 100 mL |

### Preparation method:

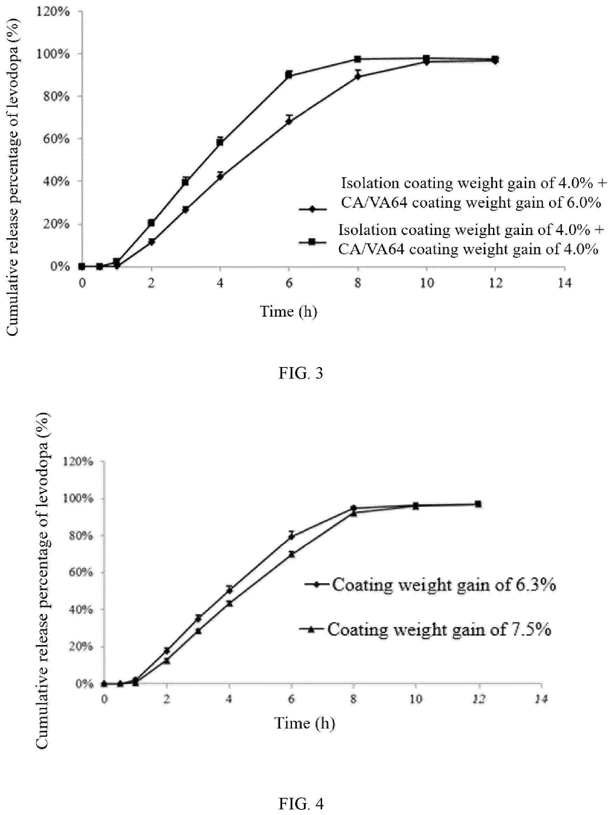

1. Preparation of the drug-containing layer: Same as in prescription II of Example 1.
2. Preparation of the push layer: Same as in prescription II of Example 1.
3. Tablet compressing: Two-layer tablets were compressed with a die size of 16 * 7 mm (deep concave); 320.0 mg of drug-containing layer material was first filled and pre-compressed, followed by the filling of 160.0 mg of push layer material, and the final compression was performed to obtain a two-layer tablet core with a weight of 480.0 mg/tablet compressed in a direction perpendicular to the long axis.
4. Isolation coating: The two-layer tablet core was coated with the above isolation coating solution using a coating pan to a weight gain of 4.0wt% (percentages are weight percentages relative to the tablet core).
5. Semi-permeable membrane coating: The two-layer tablet core coated by the isolation coating was coated with the above controlled-release coating solution using a coating pan to weight gains of 4.0wt% and 6.0wt% (percentages are weight percentages relative to the two-layer tablet core coated by the isolation coating). The holes were punched using mechanical punching or laser punching, and the diameter of the drug-release hole was 1.0 mm. The specification of the controlled-release tablet in this example was 37.5 mg/150 mg (carbidopa/levodopa), a capsule-shaped biconvex tablet.
6. Dissolution test method: Same as in Example 1. The specific test results of levodopa are shown in Table 3 and FIG. 3.

**Table 3**

| Sampling point/h | Isolation coating weight gain of 4.0% + CA/VA64 coating weight gain of 6.0% | SD | Isolation coating weight gain of 4.0% + CA/VA64 coating weight gain of 4.0% | SD |
|---|---|---|---|---|
| 0 | 0.0% | 0.0% | 0.0% | 0.0% |
| 0.5 | 0.0% | 0.0% | 0.0% | 0.0% |
| 1 | 0.2% | 0.3% | 2.1% | 0.6% |
| 2 | 11.3% | 1.6% | 20.1% | 2.1% |
| 3 | 26.6% | 1.6% | 39.5% | 2.4% |
| 4 | 42.1% | 2.4% | 58.0% | 2.9% |
| 6 | 68.1% | 3.0% | 89.5% | 2.4% |
| 8 | 89.2% | 3.3% | 97.3% | 0.1% |
| 10 | 96.2% | 1.0% | 97.7% | 0.2% |
| 12 | 96.6% | 1.2% | 97.5% | 0.6% |

### Example 3: Capsule-shaped tablet (with a non-circular cross section, and compressed in a direction perpendicular to the long axis) with a specification of 37.5 mg/150 mg (carbidopa/levodopa), with CA320S + hydroxypropyl cellulose HXF

The prescription is as follows:

| (1) | Drug-containing layer prescription (per tablet) | Same as prescription II in Example 1 |
|---|---|---|
| (2) | Push layer prescription (per tablet) | Same as prescription II in Example 1 |
| (3) | Controlled-release membrane prescription | |
| | Cellulose acetate 320S | 28.0 g |
| | Hydroxypropyl cellulose HXF | 8.0 g |
| | Triethyl citrate | 4.0 g |
| | Acetone | 900 mL |
| | Purified water | 100 mL |

### Preparation method:

1. Preparation of the drug-containing layer: Same as in prescription II of Example 1.
2. Preparation of the push layer: Same as in prescription II of Example 1.
3. Tablet compressing: Two-layer tablets were compressed with a die size of 16 * 7 mm (deep concave); 320.0 mg of drug-containing layer material was first filled and pre-compressed, followed by the filling of 160.0 mg of push layer material, and the final compression was performed to obtain a two-layer tablet core with a weight of 480.0 mg/tablet compressed in a direction perpendicular to the long axis.
4. Semi-permeable membrane coating: The two-layer tablet core coated by the isolation coating was coated with the above controlled-release coating solution using a coating pan to weight gains of 6.3wt% and 7.5wt% (percentages are weight percentages relative to the two-layer tablet core coated by the isolation coating). The holes were punched using mechanical punching or laser punching, and the diameter of the drug-release hole was 1.0 mm. The specification of the controlled-release tablet in this example was 37.5 mg/150 mg (carbidopa/levodopa), a capsule-shaped biconvex tablet.
5. Dissolution test method: Same as in Example 1. The specific test results are shown in Table 4 and FIG. 4.

**Table 4**

| Sampling point/h | Coating weight gain of 6.3% | SD | Coating weight gain of 7.5% | SD | Sampling point/h | Coating weight gain of 6.3% | SD | Coating weight gain of 7.5% | SD |
|---|---|---|---|---|---|---|---|---|---|
| 0 | 0.0% | 0.0% | 0.0% | 0.0% | 4 | 50.2% | 2.6% | 43.4% | 1.1% |
| 0.5 | 0.1% | 0.0% | 0.2% | 0.1% | 6 | 79.5% | 3.0% | 69.7% | 1.4% |
| 1 | 2.2% | 0.6% | 0.7% | 0.1% | 8 | 95.0% | 0.7% | 92.2% | 1.7% |
| 2 | 17.5% | 1.9% | 12.6% | 0.9% | 10 | 96.4% | 1.0% | 95.9% | 1.9% |
| 3 | 34.9% | 2.2% | 28.5% | 0.7% | 12 | 97.0% | 0.9% | 97.1% | 1.6% |

### Example 4: Capsule-shaped tablet (with a non-circular cross section, and compressed in a direction perpendicular to the long axis) with a specification of 37.5 mg/150 mg (carbidopa/levodopa), with CA320S + triethyl citrate

The prescription is as follows:

| (1) | Drug-containing layer prescription (per tablet) | Same as prescription II in Example 1 |
|---|---|---|
| (2) | Push layer prescription (per tablet) | Same as prescription II in Example 1 |
| (3) | Controlled-release membrane prescription | |
| | Cellulose acetate 320S | 36.0 g |
| | Triethyl citrate | 4.0 g |
| | Acetone | 900 mL |
| | Purified water | 100 mL |

### Preparation method:

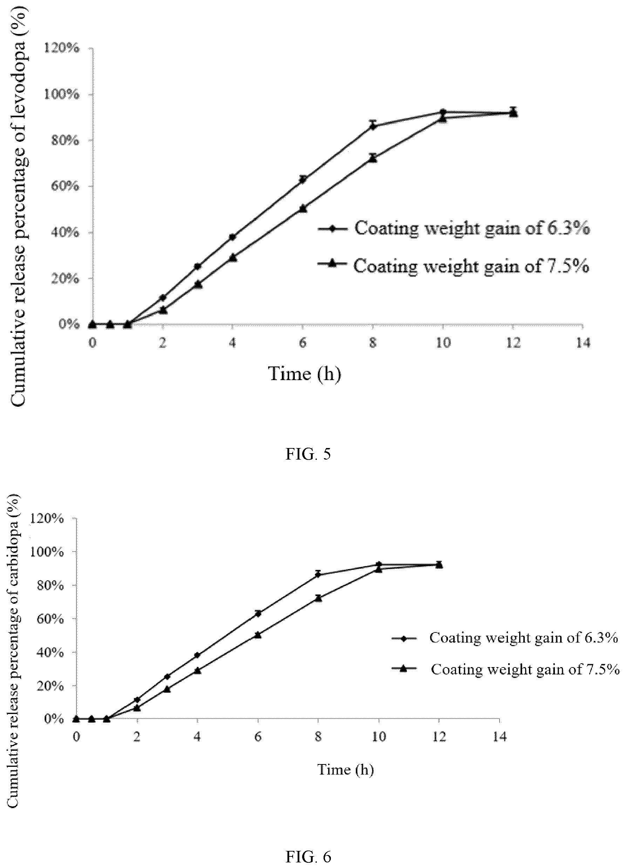

1. Preparation of the drug-containing layer: Same as in prescription II of Example 1.
2. Preparation of the push layer: Same as in prescription II of Example 1.
3. Tablet compressing: Two-layer tablets were compressed with a die size of 16 * 7 mm (deep concave); 320.0 mg of drug-containing layer material was first filled and pre-compressed, followed by the filling of 160.0 mg of push layer material, and the final compression was performed to obtain a two-layer tablet core with a weight of 480.0 mg/tablet compressed in a direction perpendicular to the long axis.
4. Semi-permeable membrane coating: The two-layer tablet core coated by the isolation coating was coated with the above controlled-release coating solution using a coating pan to weight gains of 6.3wt% and 7.5wt% (percentages are weight percentages relative to the two-layer tablet core coated by the isolation coating). The holes were punched using mechanical punching or laser punching, and the diameter of the drug-release hole was 1.0 mm. The specification of the controlled-release tablet in this example was 37.5 mg/150 mg (carbidopa/levodopa), a capsule-shaped biconvex tablet.
5. Dissolution test method: Same as in Example 1. Specific levodopa test results are shown in Table 5 and FIG. 5, and carbidopa test results are shown in Table 6 and FIG. 6.

**Table 5**

| Sampling point/h | Coating weight gain of 6.3% | SD | Coating weight gain of 7.5% | SD |
|---|---|---|---|---|
| 0 | 0.0% | 0.0% | 0.0% | 0.0% |
| 0.5 | 0.2% | 0.0% | 0.1% | 0.0% |
| 1 | 0.6% | 0.1% | 0.3% | 0.0% |
| 2 | 11.0% | 0.5% | 6.1% | 0.7% |
| 3 | 25.5% | 0.4% | 18.1% | 0.6% |
| 4 | 38.1% | 0.7% | 29.7% | 0.6% |
| 6 | 63.2% | 2.3% | 51.4% | 1.0% |
| 8 | 86.7% | 2.6% | 73.4% | 2.1% |
| 10 | 93.8% | 1.3% | 91.2% | 2.1% |
| 12 | 94.5% | 1.2% | 94.3% | 1.8% |

**Table 6**

| Sampling point/h | Coating weight gain of 6.3% | SD | Coating weight gain of 7.5% | SD |
|---|---|---|---|---|
| 0 | 0.0% | 0.0% | 0.0% | 0.0% |
| 0.5 | 0.0% | 0.0% | 0.0% | 0.0% |
| 1 | 0.0% | 0.0% | 0.0% | 0.0% |
| 2 | 11.5% | 0.6% | 6.5% | 0.8% |
| 3 | 25.2% | 0.8% | 17.6% | 0.4% |
| 4 | 37.9% | 0.8% | 29.0% | 0.1% |
| 6 | 62.6% | 2.0% | 50.4% | 0.7% |
| 8 | 86.0% | 2.4% | 72.0% | 2.1% |
| 10 | 92.2% | 1.0% | 89.5% | 2.2% |
| 12 | 92.1% | 0.7% | 91.9% | 2.1% |

### Example 5: Capsule-shaped tablet (with a non-circular cross section, and compressed in a direction perpendicular to the long axis) with a specification of 250 mg

The prescription is as follows:

| (1) | Drug-containing layer prescription (per tablet) | mg/tablet | Proportion wt% |
|---|---|---|---|
| | Levodopa | 250.0 | *50.0* |
| | Hydroxypropyl cellulose | 155.0 | 31.0 |
| | Mannitol | 90.0 | 18.0 |
| | Magnesium stearate | 5.0 | 1.0 |

| (2) (Same as Example 1) | Push layer prescription (per tablet) | mg/tablet | Proportion wt% |
|---|---|---|---|
| | Sodium carboxymethyl cellulose | 122.5 | 49.0 |
| | Sorbitol | 75.0 | 30.0 |
| | Hydroxypropyl cellulose | 50.0 | 20.0 |
| | Iron oxide red | 1.25 | 0.50 |
| | Magnesium stearate | 1.25 | 0.50 |

| (3) | Controlled-release membrane prescription | | |
|---|---|---|---|
| | Cellulose acetate | 24.0 g | |
| | Copovidone | 16.0 g | |
| | Acetone | 900 mL | |
| | Ethanol | 100 mL | |

### Preparation method:

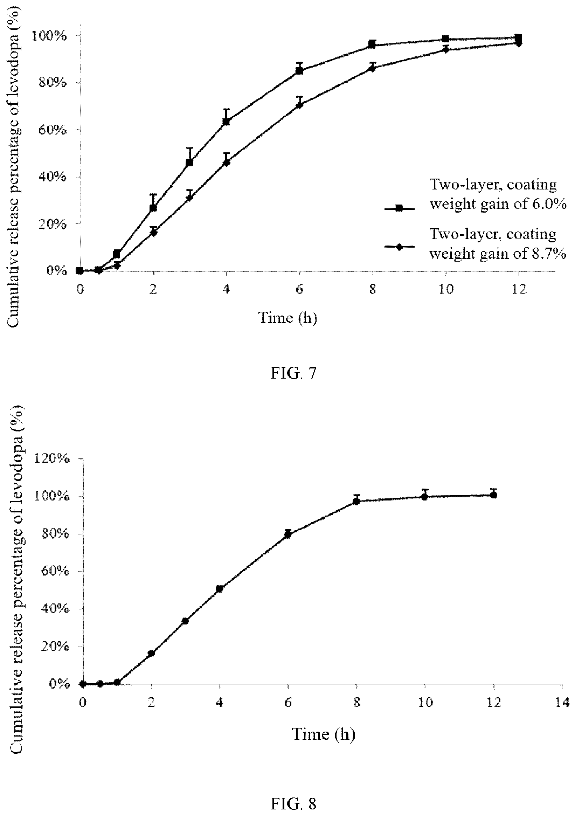

1. Preparation of the drug-containing layer: Levodopa was premixed with other excipients of the drug-containing layer (except magnesium stearate), added to a dry granulator and compressed into strips, granulated through a 16-mesh stainless steel screen, and finally added with magnesium stearate and mixed for later use.
2. Preparation of the push layer: Excipients of the push layer (except magnesium stearate) were premixed, added to a dry granulator and compressed into strips, granulated through a 16-mesh stainless steel screen, and finally added with magnesium stearate and mixed for later use.
3. Tablet compressing: Two-layer tablets were compressed with a die size of 19 * 7.5 mm (shallow concave); 500 mg of drug-containing layer material was first filled and pre-compressed, followed by the filling of 250 mg of push layer material, and the final compression was performed to obtain a two-layer tablet core with a weight of 750 mg/tablet compressed in a direction perpendicular to the long axis.
4. Semi-permeable membrane coating: The two-layer tablet core was coated with the above controlled-release coating solution using a coating pan to weight gains of 6.0wt% and 8.7wt% (percentages are weight percentages relative to the tablet core). The holes were punched using mechanical punching or laser punching, and the diameter of the drug-release hole was 1.0 mm. The specification of the controlled-release tablet in this example was 250 mg of levodopa, a capsule-shaped biconvex tablet.
5. Dissolution test method: Same as in Example 1. The specific test results are shown in Table 7 and FIG. 7.

**Table 7**

| Sampling point/h | Two-layer, coating weight gain of 6.0% | SD | Two-layer, coating weight gain of 8.7% | SD |
|---|---|---|---|---|
| 0 | 0.0% | 0.0% | 0.0% | 0.0% |
| 0.5 | 0.4% | 0.2% | 0.1% | 0.0% |
| 1 | 6.7% | 2.0% | 2.3% | 1.5% |
| 2 | 26.7% | 5.6% | 16.4% | 2.6% |
| 3 | 46.2% | 5.9% | 31.1% | 3.4% |
| 4 | 63.4% | 5.5% | 46.1% | 3.9% |
| 6 | 85.0% | 3.5% | 70.5% | 3.7% |
| 8 | 95.9% | 2.1% | 86.2% | 2.5% |
| 10 | 98.6% | 0.5% | 94.0% | 1.6% |
| 12 | 99.1% | 1.0% | 96.8% | 0.8% |

### Example 6: Capsule-shaped tablet (with a non-circular cross section, and compressed in a direction perpendicular to the long axis) with a specification of 250 mg

The prescription is as follows:

| (1) | Drug-containing layer prescription (per tablet) | mg/tablet | Proportion wt% |
|---|---|---|---|
| | Levodopa | 250.0 | 63.0 |
| | Hydroxypropyl cellulose | 43.6 | 11.0 |
| | Sorbitol | 39.7 | 10.0 |
| | Povidone | 19.8 | 5.00 |
| | Poloxamer | 39.7 | 10.0 |
| | Magnesium stearate | 3.97 | 1.0 |

| (2) (Same as Example 1) | Push layer prescription (per tablet) | mg/tablet | Proportion wt% |
|---|---|---|---|
| | Sodium carboxymethyl cellulose | 97.2 | 49.0 |
| | Sorbitol | 59.5 | 30.0 |
| | Hydroxypropyl cellulose | 39.7 | 20.0 |
| | Iron oxide red | 0.99 | 0.50 |
| | Magnesium stearate | 0.99 | 0.50 |

| (3) | Controlled-release membrane prescription | | |
|---|---|---|---|
| | Cellulose acetate | 26.0 g | |
| | Copovidone | 14.0 g | |
| | Acetone | 900 mL | |
| | Ethanol | 100 mL | |

### Preparation method:

1. Preparation of the drug-containing layer: Same as in Example 5.
2. Preparation of the push layer: Same as in Example 5.
3. Tablet compressing: Two-layer tablets were compressed with a die size of 16 * 7 mm (deep concave); 396.8 mg of drug-containing layer material was first filled and pre-compressed, followed by the filling of 198.4 mg of push layer material, and the final compression was performed to obtain a two-layer tablet core with a weight of 595.2 mg/tablet compressed in a direction perpendicular to the long axis.
4. Semi-permeable membrane coating: The two-layer tablet core was coated with the above controlled-release coating solution using a coating pan to a weight gain of 8.0wt% (percentages are weight percentages relative to the tablet core). The holes were punched using mechanical punching or laser punching, and the diameter of the drug-release hole was 1.0 mm. The specification of the controlled-release tablet in this example was 250 mg of levodopa, a capsule-shaped biconvex tablet.
5. Dissolution test method: Same as in Example 1. The specific test results are shown in Table 8 and FIG. 8.

**Table 8**

| Sampling point/h | Cumulative release (%) | SD | Sampling point/h | Cumulative release (%) | SD |
|---|---|---|---|---|---|
| 0 | 0.0% | 0.0% | 4 | 50.4% | 1.9% |
| 0.5 | 0.1% | 0.0% | 6 | 79.6% | 2.5% |
| 1 | 0.8% | 0.2% | 8 | 97.1% | 3.6% |
| 2 | 16.0% | 0.9% | 10 | 99.5% | 3.7% |
| 3 | 33.4% | 1.5% | 12 | 100.4% | 3.7% |

### Example 7: Capsule-shaped tablet (with a non-circular cross section, and compressed in a direction perpendicular to the long axis) with a specification of 125 mg

The prescription is as follows:

| (1) | Drug-containing layer prescription (per tablet) | mg/tablet | Proportion wt% |
|---|---|---|---|
| | Levodopa | 125.0 | 31.5 |
| | Hydroxypropyl cellulose | 79.4 | 20.0 |
| | Sorbitol | 75.4 | 19.0 |
| | Poloxamer 407 | 75.4 | 19.0 |
| | Povidone | 37.6 | 9.50 |
| | Magnesium stearate | 3.97 | 1.00 |

| (2) | Push layer prescription (per tablet) | mg/tablet | Proportion wt% |
|---|---|---|---|
| | Sodium carboxymethyl cellulose | 136.9 | 69.0 |
| | Sorbitol | 19.8 | 10.0 |
| | Hydroxypropyl cellulose | 39.6 | 20.0 |
| | Iron oxide red | 0.99 | 0.50 |
| | Magnesium stearate | 0.99 | 0.50 |

| (3) | Controlled-release membrane prescription (4.0% solid content in prescription) | | |
|---|---|---|---|
| | Cellulose acetate | 26.0 g | |
| | Copovidone | 14.0 g | |
| | Acetone | 900 mL | |
| | Ethanol | 100 mL | |

### Preparation method:

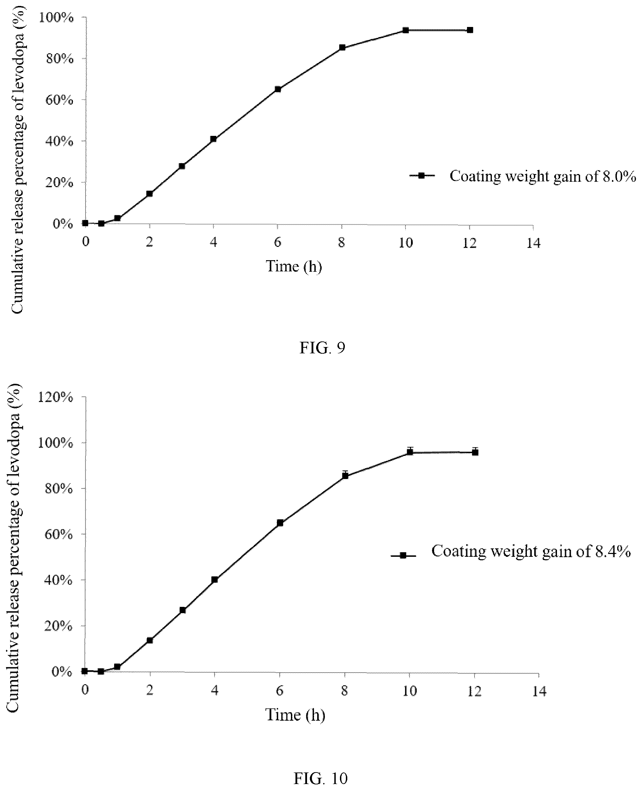

1. Preparation of the drug-containing layer: Same as in Example 5.
2. Preparation of the push layer: Same as in Example 5.
3. Tablet compressing: Two-layer tablets were compressed with a die size of 16 * 7 mm (deep concave); 396.8 mg of drug-containing layer material was first filled and pre-compressed, followed by the filling of 198.4 mg of push layer material, and the final compression was performed to obtain a two-layer tablet core with a weight of 595.2 mg/tablet compressed in a direction perpendicular to the long axis.
4. Semi-permeable membrane coating: The two-layer tablet core was coated with the above controlled-release coating solution using a coating pan to a weight gain of 8.0wt% (percentages are weight percentages relative to the tablet core). The holes were punched using mechanical punching or laser punching, and the diameter of the drug-release hole was 1.00 mm. The specification of the controlled-release tablet in this example was 125 mg of levodopa, a capsule-shaped biconvex tablet with a size of 16 * 7 mm.
5. Dissolution test method: Same as in Example 1. The specific test results are shown in Table 9 and FIG. 9.

**Table 9**

| Sampling point/h | Coating weight gain of 8.0% | SD | Sampling point/h | Coating weight gain of 8.0% | SD |
|---|---|---|---|---|---|
| 0 | 0.0% | 0.0% | 4 | 41.2% | 0.6% |
| 0.5 | 0.2% | 0.0% | 6 | 65.4% | 0.6% |
| 1 | 2.6% | 0.0% | 8 | 85.7% | 0.9% |
| 2 | 14.5% | 0.3% | 10 | 94.3% | 0.4% |
| 3 | 27.9% | 0.4% | 12 | 94.6% | 0.4% |

### Example 8: Capsule-shaped tablet (with a non-circular cross section, and compressed in a direction perpendicular to the long axis) with a specification of 125 mg

The prescription is as follows:

| (1) | Drug-containing layer prescription (per tablet) | mg/tablet | Proportion wt% |
|---|---|---|---|
| | Levodopa | 125.0 | 31.5 |
| | Hydroxypropyl cellulose | 115.1 | 29.0 |
| | Sorbitol | 75.4 | 19.0 |
| | Poloxamer 407 | 39.7 | 10.0 |
| | Povidone | 37.6 | 9.50 |
| | Magnesium stearate | 3.97 | 1.00 |

| (2) | Push layer prescription (per tablet) | mg/tablet | Proportion wt% |
|---|---|---|---|
| | Sodium carboxymethyl cellulose | 136.9 | 69.0 |
| | Sorbitol | 19.8 | 10.0 |
| | Hydroxypropyl cellulose | 39.6 | 20.0 |
| | Iron oxide red | 0.99 | 0.50 |
| | Magnesium stearate | 0.99 | 0.50 |

| (3) | Controlled-release membrane prescription (4.0% solid content in prescription) | | |
|---|---|---|---|
| | Cellulose acetate | 26.0 g | |
| | Copovidone | 14.0 g | |
| | Acetone | 900 mL | |
| | Ethanol | 100 mL | |

### Preparation method:

1. Preparation of the drug-containing layer: Same as in Example 5.
2. Preparation of the push layer: Same as in Example 5.
3. Tablet compressing: Two-layer tablets were compressed with a die size of 16 * 7 mm (deep concave); 396.8 mg of drug-containing layer material was first filled and pre-compressed, followed by the filling of 198.4 mg of push layer material, and the final compression was performed to obtain a two-layer tablet core with a weight of 595.2 mg/tablet compressed in a direction perpendicular to the long axis.
4. Semi-permeable membrane coating: The two-layer tablet core was coated with the above controlled-release coating solution using a coating pan to a weight gain of 8.4wt% (percentages are weight percentages relative to the tablet core). The holes were punched using mechanical punching or laser punching, and the diameter of the drug-release hole was 1.00 mm. The specification of the controlled-release tablet in this example was 125 mg of levodopa, a capsule-shaped biconvex tablet with a size of 16 * 7 mm.
5. Dissolution test method: Same as in Example 1. The specific test results are shown in Table 10 and FIG. 10.

**Table 10**

| Sampling point/h | Coating weight gain of 8.4% | SD | Sampling point/h | Coating weight gain of 8.4% | SD |
|---|---|---|---|---|---|
| 0 | 0.0% | 0.0% | 4 | 40.3% | 1.1% |
| 0.5 | 0.1% | 0.1% | 6 | 65.0% | 1.9% |
| 1 | 1.9% | 0.1% | 8 | 85.8% | 2.3% |
| 2 | 13.7% | 0.4% | 10 | 96.3% | 2.4% |
| 3 | 26.7% | 0.7% | 12 | 96.5% | 2.4% |

### Example 9: Capsule-shaped tablet (with a non-circular cross section, and compressed in a direction perpendicular to the long axis) with a specification of 125 mg

The prescription is as follows:

| (1) | Drug-containing layer prescription (per tablet) | mg/tablet | Proportion wt% |
|---|---|---|---|
| | Levodopa | 125.0 | 31.5 |
| | Hydroxypropyl cellulose | 79.4 | 20.0 |
| | Sorbitol | 75.4 | 19.0 |
| | Poloxamer 407 | 75.4 | 19.0 |
| | Povidone | 37.6 | 9.50 |
| | Magnesium stearate | 3.97 | 1.00 |

| (2) | Push layer prescription (per tablet) | mg/tablet | Proportion wt% |
|---|---|---|---|
| | Sodium carboxymethyl cellulose | 97.2 | 49.0 |
| | Sorbitol | 59.5 | 30.0 |
| | Hydroxypropyl cellulose | 39.7 | 20.0 |
| | Iron oxide red | 0.99 | 0.50 |
| | Magnesium stearate | 0.99 | 0.50 |

| (3) | Controlled-release membrane prescription (4.0% solid content in prescription) | | |
|---|---|---|---|
| | Cellulose acetate | 26.0 g | |

### Preparation method:

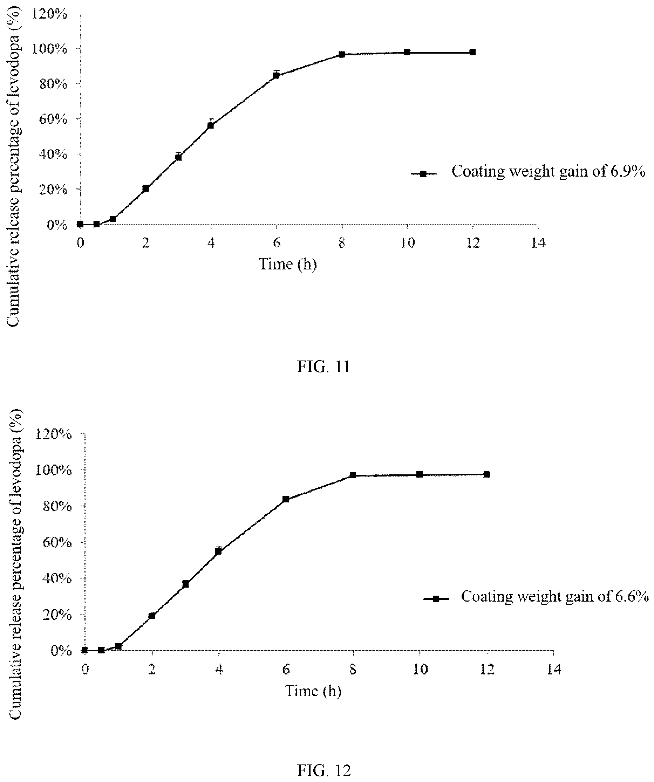

1. Preparation of the drug-containing layer: Same as in Example 5.
2. Preparation of the push layer: Same as in Example 5.
3. Tablet compressing: Two-layer tablets were compressed with a die size of 16 * 7 mm (deep concave); 396.8 mg of drug-containing layer material was first filled and pre-compressed, followed by the filling of 198.4 mg of push layer material, and the final compression was performed to obtain a two-layer tablet core with a weight of 595.2 mg/tablet.
4. Semi-permeable membrane coating: The two-layer tablet core was coated with the above controlled-release coating solution using a coating pan to a weight gain of 6.9wt% (percentages are weight percentages relative to the tablet core). The holes were punched using mechanical punching or laser punching, and the diameter of the drug-release hole was 1.00 mm. The specification of the controlled-release tablet in this example was 125 mg of levodopa, a capsule-shaped biconvex tablet with a size of 16 * 7 mm compressed in a direction perpendicular to the long axis.
5. Dissolution test method: Same as in Example 1. The specific test results are shown in Table 11 and FIG. 11.

**Table 11**

| Sampling point/h | Coating weight gain of 6.9% | SD | Sampling point/h | Coating weight gain of 6.9% | SD |
|---|---|---|---|---|---|
| 0 | 0.0% | 0.0% | 4 | 56.2% | 4.0% |
| 0.5 | 0.0% | 0.0% | 6 | 84.5% | 3.4% |
| 1 | 3.4% | 0.8% | 8 | 96.7% | 1.1% |
| 2 | 20.0% | 2.2% | 10 | 97.8% | 1.6% |
| 3 | 38.0% | 3.2% | 12 | 97.9% | 1.3% |

### Example 10: Capsule-shaped tablet (with a non-circular cross section, and compressed in a direction perpendicular to the long axis) with a specification of 125 mg

The prescription is as follows:

| (1) | Drug-containing layer prescription (per tablet) | mg/tablet | Proportion wt% |
|---|---|---|---|
| | Levodopa | 125.0 | 31.5 |
| | Hydroxypropyl cellulose | 115.1 | 29.0 |
| | Sorbitol | 75.4 | 19.0 |
| | Poloxamer 407 | 39.7 | 10.0 |
| | Povidone | 37.6 | 9.50 |
| | Magnesium stearate | 3.97 | 1.00 |

| (2) | Push layer prescription (per tablet) | mg/tablet | Proportion wt% |
|---|---|---|---|
| | Sodium carboxymethyl cellulose | 97.2 | 49.0 |
| | Sorbitol | 59.5 | 30.0 |
| | Hydroxypropyl cellulose | 39.7 | 20.0 |
| | Iron oxide red | 0.99 | 0.50 |
| | Magnesium stearate | 0.99 | 0.50 |

| (3) | Controlled-release membrane prescription (4.0% solid content in prescription) | | |
|---|---|---|---|
| | Cellulose acetate | 26.0 g | |
| | Copovidone | 14.0 g | |
| | Acetone | 900 mL | |
| | Ethanol | 100 mL | |

### Preparation method:

1. Preparation of the drug-containing layer: Same as in Example 5.
2. Preparation of the push layer: Same as in Example 5.
3. Tablet compressing: Two-layer tablets were compressed with a die size of 16 * 7 mm (deep concave); 396.8 mg of drug-containing layer material was first filled and pre-compressed, followed by the filling of 198.4 mg of push layer material, and the final compression was performed to obtain a two-layer tablet core with a weight of 595.2 mg/tablet compressed in a direction perpendicular to the long axis.
4. Semi-permeable membrane coating: The two-layer tablet core was coated with the above controlled-release coating solution using a coating pan to a weight gain of 6.6wt% (percentages are weight percentages relative to the tablet core). The holes were punched using mechanical punching or laser punching, and the diameter of the drug-release hole was 1.00 mm. The specification of the controlled-release tablet in this example was 125 mg of levodopa, a capsule-shaped biconvex tablet with a size of 16 * 7 mm.
5. Dissolution test method: Same as in Example 1. The specific test results are shown in Table 12 and FIG. 12.

**Table 12**

| Sampling point/h | Coating weight gain of 6.6% | SD | Sampling point/h | Coating weight gain of 6.6% | SD |
|---|---|---|---|---|---|
| 0 | 0.0% | 0.0% | 4 | 54.6% | 3.0% |
| 0.5 | 0.0% | 0.0% | 6 | 83.5% | 1.9% |
| 1 | 2.5% | 0.2% | 8 | 96.9% | 0.7% |
| 2 | 19.1% | 1.2% | 10 | 97.5% | 0.8% |
| 3 | 36.5% | 2.5% | 12 | 97.7% | 0.6% |

### Example 11: Capsule-shaped tablet (with a circular cross section, and compressed in the long-axis direction) with a specification of 250 mg

The prescription is as follows:

| (1) | Drug-containing layer prescription (per tablet) | mg/tablet | Proportion wt% |
|---|---|---|---|
| | Levodopa | 250.0 | 63.0 |
| | Hydroxypropyl cellulose | 39.7 | 10.0 |
| | Sorbitol | 77.4 | 19.5 |
| | Poloxamer 407 | 19.8 | 5.00 |
| | Silica | 1.98 | 0.5 |
| | Magnesium stearate | 7.92 | 2.00 |

| (2) | Push layer prescription (per tablet) | mg/tablet | Proportion wt% |
|---|---|---|---|
| | Sodium carboxymethyl cellulose | 111.3 | 69.0 |
| | Sorbitol | 16.1 | 10.0 |
| | Hydroxypropyl cellulose | 32.3 | 20.0 |
| | Iron oxide red | 0.81 | 0.50 |
| | Magnesium stearate | 0.81 | 0.50 |

| (3) | Controlled-release membrane prescription (4.0% solid content in prescription) | | |
|---|---|---|---|
| | Cellulose acetate | 24.0 g | |
| | Copovidone | 14.0 g | |
| | Polyethylene glycol 400 | 2.00 g | |
| | Acetone | 900 mL | |
| | Ethanol | 100 mL | |

### Preparation method:

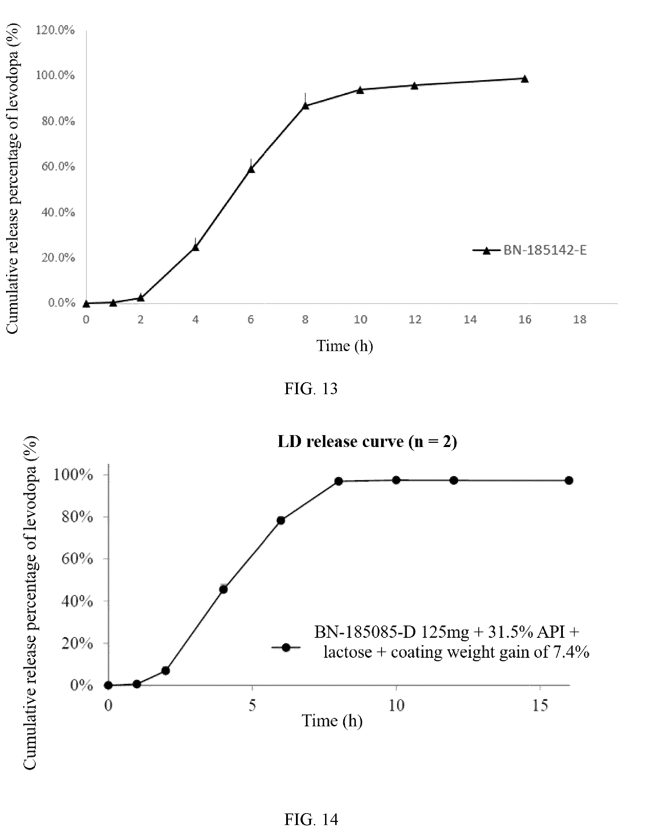

1. Preparation of the drug-containing layer: Levodopa was premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Same as in Example 5.
3. Tablet compressing: Two-layer tablets were compressed with a die size of Φ6 mm round punch (deep concave); 396.8 mg of drug-containing layer material was first filled and pre-compressed, followed by the filling of 161.3 mg of push layer material, and the final compression was performed to obtain a two-layer tablet core with a weight of 558.1 mg/tablet in the long-axis direction.
4. Semi-permeable membrane coating: The two-layer tablet core was coated with the above controlled-release coating solution using a coating pan to a weight gain of 7.8wt% (percentages are weight percentages relative to the tablet core). The holes were punched using mechanical punching or laser punching, and the diameter of the drug-release hole was 1.0 mm. The specification of the controlled-release tablet in this example was 250 mg of levodopa, a capsule-shaped tablet having a cross-sectional diameter of 6 mm and a height of 14.90 mm.
5. Dissolution test method: Same as in Example 1. The specific test results are shown in Table 13 and FIG. 13.

**Table 13**

| Sampling point/h | BN-185142-E (coating weight gain of 7.8%) | SD | Sampling point/h | BN-185142-E (coating weight gain of 7.8%) | SD |
|---|---|---|---|---|---|
| 0 | 0.0% | 0.0% | 8 | 86.7% | 5.7% |
| 1 | 0.4% | 0.2% | 10 | 93.8% | 1.8% |
| 2 | 2.4% | 2.2% | 12 | 95.6% | 1.2% |
| 4 | 24.8% | 4.1% | 16 | 98.7% | 1.6% |
| 6 | 58.9% | 4.6% | | | |

### Example 12: Capsule-shaped tablet (with a circular cross section, and compressed in the long-axis direction) with a specification of 375 mg

The prescription is as follows:

| (1) | Drug-containing layer prescription (per tablet) | mg/tablet | Proportion wt% |
|---|---|---|---|
| | Levodopa | 375.0 | 63.0 |
| | Hydroxypropyl cellulose | 59.6 | 10.0 |
| | Sorbitol | 116.1 | 19.5 |
| | Poloxamer 407 | 29.7 | 5.00 |
| | Silica | 2.97 | 0.5 |
| | Magnesium stearate | 11.9 | 2.00 |

| (2) | Push layer prescription (per tablet) | mg/tablet | Proportion wt% |
|---|---|---|---|
| | Sodium carboxymethyl cellulose | 167.0 | 69.0 |
| | Sorbitol | 24.2 | 10.0 |
| | Hydroxypropyl cellulose | 48.4 | 20.0 |
| | Iron oxide red | 1.21 | 0.50 |
| | Magnesium stearate | 1.21 | 0.50 |

| (3) | Controlled-release membrane prescription (4.0% solid content in prescription) | | |
|---|---|---|---|
| | Cellulose acetate | 24.0 g | |
| | Copovidone | 14.0 g | |
| | Polyethylene glycol 400 | 2.00 g | |
| | Acetone | 900 mL | |
| | Ethanol | 100 mL | |

### Preparation method:

1. Preparation of the drug-containing layer: Same as in Example 11.
2. Preparation of the push layer: Same as in Example 11.
3. Tablet compressing: Two-layer tablets were compressed with a die size of Φ7 mm round punch (deep concave); 595.3 mg of drug-containing layer material was first filled and pre-compressed, followed by the filling of 242.0 mg of push layer material, and the final compression was performed to obtain a two-layer tablet core with a weight of 837.3 mg/tablet compressed in the long-axis direction.
4. Semi-permeable membrane coating: The two-layer tablet core was coated with the above controlled-release coating solution using a coating pan to a weight gain of 7.0wt% (percentages are weight percentages relative to the tablet core). The holes were punched using mechanical punching or laser punching, and the diameter of the drug-release hole was 1.0 mm. The specification of the controlled-release tablet in this example was 375 mg of levodopa, a capsule-shaped tablet having a cross-sectional diameter of 7 mm and a height of 16.42 mm.

### Example 13: Capsule-shaped tablet (with a circular cross section, and compressed in the long-axis direction) with a specification of 500 mg

The prescription is as follows:

| (1) | Drug-containing layer prescription (per tablet) | mg/tablet | Proportion wt% |
|---|---|---|---|
| | Levodopa | 500.0 | 63.0 |
| | Hydroxypropyl cellulose | 79.4 | 10.0 |
| | Sorbitol | 154.8 | 19.5 |
| | Poloxamer 407 | 39.6 | 5.00 |
| | Silica | 3.96 | 0.5 |
| | Magnesium stearate | 15.84 | 2.00 |

| (2) | Push layer prescription (per tablet) | mg/tablet | Proportion wt% |
|---|---|---|---|
| | Sodium carboxymethyl cellulose | 222.6 | 69.0 |
| | Sorbitol | 32.3 | 10.0 |
| | Hydroxypropyl cellulose | 64.5 | 20.0 |
| | Iron oxide red | 1.61 | 0.50 |
| | Magnesium stearate | 1.61 | 0.50 |

| (3) | Controlled-release membrane prescription (4.0% solid content in prescription) | | |
|---|---|---|---|
| | Cellulose acetate | 24.0 g | |
| | Copovidone | 14.0 g | |
| | Polyethylene glycol 400 | 2.00 g | |
| | Acetone | 900 mL | |
| | Ethanol | 100 mL | |

### Preparation method:

1. Preparation of the drug-containing layer: Same as in Example 11.
2. Preparation of the push layer: Same as in Example 11.
3. Tablet compressing: Two-layer tablets were compressed with a die size of Φ8 mm round punch (deep concave); 793.6 mg of drug-containing layer material was first filled and pre-compressed, followed by the filling of 322.6 mg of push layer material, and the final compression was performed to obtain a two-layer tablet core with a weight of 1116.2 mg/tablet compressed in the long-axis direction.
4. Semi-permeable membrane coating: The two-layer tablet core was coated with the above controlled-release coating solution using a coating pan to a weight gain of 7.0wt% (percentages are weight percentages relative to the tablet core). The holes were punched using mechanical punching or laser punching, and the diameter of the drug-release hole was 1.0 mm. The specification of the controlled-release tablet in this example was 500 mg of levodopa, a capsule-shaped tablet having a cross-sectional diameter of 8 mm and a height of 16.76 mm.

### Example 14: Capsule-shaped tablet (with a circular cross section, and compressed in the long-axis direction) (LD) with a specification of 125 mg

The prescription is as follows:

| (1) | Drug-containing layer prescription (per tablet) | mg/tablet | Proportion wt% |
|---|---|---|---|
| | Levodopa | 125.0 | 31.5 |
| | Lactose | 123.0 | 31.0 |
| | Hydroxypropyl cellulose | 39.7 | 10.0 |
| | Sorbitol | 79.4 | 20.0 |
| | Poloxamer 407 | 19.8 | 5.00 |
| | Silica | 1.98 | 0.50 |
| | Magnesium stearate | 7.93 | 2.00 |

| (2) | Push layer prescription (per tablet) | mg/tablet | Proportion wt% |
|---|---|---|---|
| | Sodium carboxymethyl cellulose 9H4XF | 111.3 | 69.0 |
| | Sorbitol | 16.1 | 10.0 |
| | Hydroxypropyl cellulose | 32.3 | 20.0 |
| | Iron oxide red | 0.81 | 0.50 |
| | Magnesium stearate | 0.81 | 0.50 |

| (3) | Controlled-release membrane prescription (4.0% solid content in prescription) | | |
|---|---|---|---|
| | Cellulose acetate | 24.0 g | |
| | Copovidone | 14.0 g | |
| | Polyethylene glycol 400 | 2.00 g | |
| | Acetone | 900 mL | |
| | Ethanol | 100 mL | |

### Preparation method:

1. Preparation of the drug-containing layer: Same as in Example 11.
2. Preparation of the push layer: Same as in Example 11.
3. Tablet compressing: Two-layer tablets were compressed with a die size of Φ6 mm round punch (deep concave); 396.8 mg of drug-containing layer material was first filled and pre-compressed, followed by the filling of 161.3 mg of push layer material, and the final compression was performed to obtain a two-layer tablet core with a weight of 558.1 mg/tablet compressed in the long-axis direction.
4. Semi-permeable membrane coating: The two-layer tablet core was coated with the above controlled-release coating solution using a coating pan to a weight gain of 7.4wt% (percentages are weight percentages relative to the tablet core). The holes were punched using mechanical punching or laser punching, and the diameter of the drug-release hole was 1.00 mm. The specification of the controlled-release tablet in this example was 125 mg of levodopa, a capsule-shaped tablet having a cross-sectional diameter of 6 mm and a height of 15.24 mm.
5. Dissolution test method: Same as in Example 1. The specific test results are shown in Table 14 and FIG. 14.

**Table 14**

| Sampling point/h | BN-185085-D 125mg-31.0% lactose-coating weight gain of 7.4% | SD |
|---|---|---|
| 0 | 0.0% | 0.0% |
| 1 | 0.6% | 0.1% |
| 2 | 6.9% | 1.9% |
| 4 | 45.5% | 2.5% |
| 6 | 78.3% | 1.7% |
| 8 | 96.9% | 1.3% |
| 10 | 97.5% | 1.1% |
| 12 | 97.3% | 0.9% |
| 16 | 97.3% | 1.1% |

### Example 15: Capsule-shaped tablet (with a circular cross section, and compressed in the long-axis direction) (LD) with a specification of 125 mg

The prescription is as follows:

| (1) | Drug-containing layer prescription (per tablet) | mg/tablet | Proportion wt% |
|---|---|---|---|
| | Levodopa | 125.0 | 31.5 |
| | Mannitol | 123.0 | 31.0 |
| | Hydroxypropyl cellulose | 39.7 | 10.0 |
| | Sorbitol | 79.4 | 20.0 |
| | Poloxamer 407 | 19.8 | 5.00 |
| | Silica | 1.98 | 0.50 |
| | Magnesium stearate | 7.93 | 2.00 |

| (2) | Push layer prescription (per tablet) | mg/tablet | Proportion wt% |
|---|---|---|---|
| | Sodium carboxymethyl cellulose 9H4XF | 111.3 | 69.0 |
| | Sorbitol | 16.1 | 10.0 |
| | Hydroxypropyl cellulose | 32.3 | 20.0 |
| | Iron oxide red | 0.81 | 0.50 |
| | Magnesium stearate | 0.81 | 0.50 |

| (3) | Controlled-release membrane prescription (4.0% solid content in prescription) | | |
|---|---|---|---|
| | Cellulose acetate | 24.0 g | |
| | Copovidone | 14.0 g | |
| | Polyethylene glycol 400 | 2.00 g | |
| | Acetone | 900 mL | |
| | Ethanol | 100 mL | |

### Preparation method:

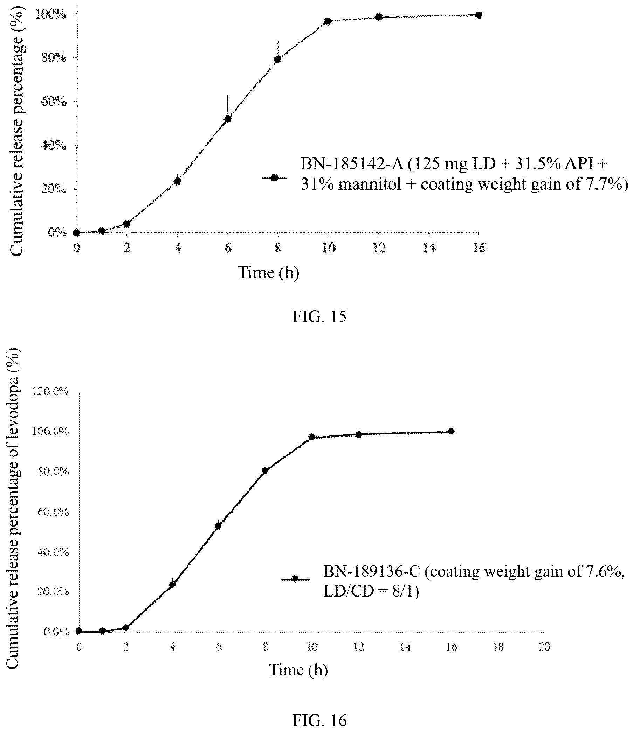

1. Preparation of the drug-containing layer: Same as in Example 11.
2. Preparation of the push layer: Same as in Example 11.
3. Tablet compressing: Two-layer tablets were compressed with a die size of Φ6 mm round punch (deep concave); 396.8 mg of drug-containing layer material was first filled and pre-compressed, followed by the filling of 161.3 mg of push layer material, and the final compression was performed to obtain a two-layer tablet core with a weight of 558.1 mg/tablet compressed in the long-axis direction.
4. Semi-permeable membrane coating: The two-layer tablet core was coated with the above controlled-release coating solution using a coating pan to a weight gain of 7.7wt% (percentages are weight percentages relative to the tablet core). The holes were punched using mechanical punching or laser punching, and the diameter of the drug-release hole was 1.00 mm. The specification of the controlled-release tablet in this example was 125 mg of levodopa, a capsule-shaped tablet having a cross-sectional diameter of 6 mm and a height of 15.24 mm.
5. Dissolution test method: Same as in Example 1. The specific test results are shown in Table 15 and FIG. 15.

**Table 15**

| Sampling point/h | BN-185142-A coating weight gain of 7.7 | SD | Sampling point/h | BN-185142-A coating weight gain of 7.7 | SD |
|---|---|---|---|---|---|
| 0 | 0.0% | 0.0% | 8 | 79.3% | 8.3% |
| 1 | 0.8% | 0.2% | 10 | 97.0% | 1.0% |
| 2 | 4.1% | 1.2% | 12 | 98.7% | 0.4% |
| 4 | 23.6% | 3.3% | 16 | 99.8% | 0.3% |
| 6 | 52.0% | 10.7% | | | |

### Example 16: Capsule-shaped tablets (with a circular cross section, and compressed in the long-axis direction) (CD/LD) with specifications of 31.3 mg/250 mg

The prescription is as follows:

| (1) | Drug-containing layer prescription (per tablet) | mg/tablet | Proportion wt% |
|---|---|---|---|
| | Levodopa | 250.0 | 55.5 |
| | Carbidopa monohydrate | 33.8 | 7.50 |
| | Hydroxypropyl cellulose | 49.6 | 11.0 |
| | Sorbitol | 90.1 | 20.0 |
| | Poloxamer 407 | 22.5 | 5.00 |
| | Magnesium stearate | 4.50 | 1.00 |

| (2) | Push layer prescription (per tablet) | mg/tablet | Proportion wt% |
|---|---|---|---|
| | Sodium carboxymethyl cellulose | 111.3 | 69.0 |
| | Sorbitol | 16.1 | 10.0 |
| | Hydroxypropyl cellulose | 32.3 | 20.0 |
| | Iron oxide red | 0.81 | 0.50 |
| | Magnesium stearate | 0.81 | 0.50 |

| (3) | Controlled-release membrane prescription (4.0% solid content in prescription) | | |
|---|---|---|---|
| | Cellulose acetate | 26.0 g | |
| | Copovidone | 14.0 g | |
| | Acetone | 900 mL | |
| | Ethanol | 100 mL | |

### Preparation method:

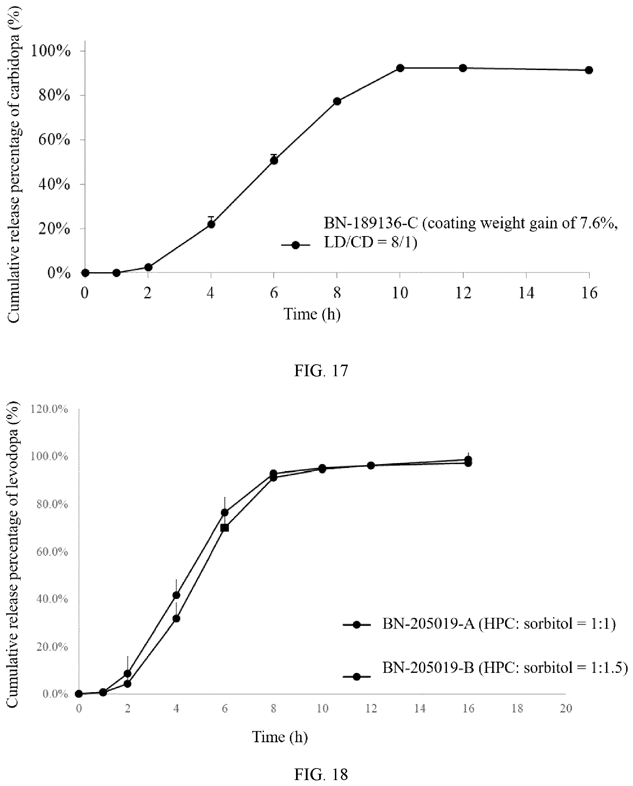

1. Preparation of the drug-containing layer: Same as in Example 1.
2. Preparation of the push layer: Same as in Example 1.
3. Tablet compressing: Two-layer tablets were compressed with a die size of Φ6 mm round punch (deep concave); 450.5 mg of drug-containing layer material was first filled and pre-compressed, followed by the filling of 183.1 mg of push layer material, and the final compression was performed to obtain a two-layer tablet core with a weight of 633.6 mg/tablet compressed in the long-axis direction.
4. Semi-permeable membrane coating: The two-layer tablet core was coated with the above controlled-release coating solution using a coating pan to a weight gain of 7.6wt% (percentages are weight percentages relative to the tablet core). The holes were punched using mechanical punching or laser punching, and the diameter of the drug-release hole was 1.00 mm. The specifications of the controlled-release tablet in this example were 250 mg of levodopa and 31.3 mg of carbidopa, a capsule-shaped tablet having a cross-sectional diameter of 6 mm and a height of 17.27 mm.
5. Dissolution test method: Same as in Example 1. Specific levodopa test results are shown in Table 16 and FIG. 16, and carbidopa test results are shown in Table 17 and FIG. 17.

**Table 16**

| Sampling point/h | BN-189136-C (coating weight gain of 7.6%, LD/CD = 8/1) | SD |
|---|---|---|
| 0 | 0.4% | 0.0% |
| 1 | 0.4% | 0.1% |
| 2 | 2.2% | 0.5% |
| 4 | 23.3% | 3.8% |
| 6 | 53.1% | 3.0% |
| 8 | 80.5% | 1.2% |
| 10 | 97.1% | 0.4% |
| 12 | 98.5% | 0.2% |
| 16 | 99.9% | 0.2% |

**Table 17**

| Sampling point/h | BN-189136-C (coating weight gain of 7.6%, LD/CD = 8/1) | SD |
|---|---|---|
| 0 | 0.0% | 0.0% |
| 1 | 0.0% | 0.0% |
| 2 | 2.4% | 0.5% |
| 4 | 21.9% | 3.5% |
| 6 | 50.7% | 2.6% |
| 8 | 77.5% | 0.4% |
| 10 | 92.5% | 0.6% |
| 12 | 92.4% | 0.9% |
| 16 | 91.6% | 0.8% |

### Example 17: Capsule-shaped tablet (with a circular cross section, and compressed in the long-axis direction) with a specification of 250 mg

On the basis of Example 11, the ratio of HPC: Sorbitol was 1:1 or 1:1.5, and the others were the same as in Example 11. The prescription is as follows:

| | | Prescription I (HPC: Sorbitol = 1:1) BN-205019-A | | Prescription II (HPC: Sorbitol = 1:1.5) BN-205019-B | |
|---|---|---|---|---|---|
| (1) | Drug-containing layer prescription (per tablet) | mg/tablet | Proportion wt% | mg/tablet | Proportion wt% |
| | Levodopa | 250.0 | 63.0 | 250.0 | 63.0 |
| | Hydroxypropyl cellulose | 58.5 | 14.8 | 46.8 | 11.8 |
| | Sorbitol | 58.5 | 14.8 | 70.2 | 17.7 |
| | Poloxamer 407 | 19.8 | 5.00 | 19.8 | 5.00 |
| | Silica | 1.98 | 0.5 | 1.98 | 0.5 |
| | Magnesium stearate | 7.93 | 2.00 | 7.93 | 2.00 |

| (2) | Push layer prescription (per tablet) | mg/tablet | Proportion wt% | mg/tablet | Proportion wt% |
|---|---|---|---|---|---|
| | Sodium carboxymethyl cellulose | 111.3 | 69.0 | 111.3 | 69.0 |
| | Sorbitol | 16.1 | 10.0 | 16.1 | 10.0 |
| | Hydroxypropyl cellulose | 32.3 | 20.0 | 32.3 | 20.0 |
| | Iron oxide red | 0.81 | 0.50 | 0.81 | 0.50 |
| | Magnesium stearate | 0.81 | 0.50 | 0.81 | 0.50 |

| (3) | Controlled-release membrane prescription (4.0% solid content in prescription) | | | | |
|---|---|---|---|---|---|
| | Cellulose acetate | 24.0 g | | | |
| | Copovidone | 14.0 g | | | |
| | Polyethylene glycol 400 | 2.00 g | | | |
| | Acetone | 900 mL | | | |
| | Ethanol | 100 mL | | | |

### Preparation method:

1. Preparation of the drug-containing layer: Same as in Example 11.
2. Preparation of the push layer: Same as in Example 11.
3. Tablet compressing: Two-layer tablets were compressed with a die size of Φ6 mm round punch (deep concave); 396.8 mg of drug-containing layer material was first filled and pre-compressed, followed by the filling of 161.3 mg of push layer material, and the final compression was performed to obtain a two-layer tablet core with a weight of 558.1 mg/tablet compressed in the long-axis direction.
4. Semi-permeable membrane coating: The two-layer tablet core was coated with the above controlled-release coating solution using a coating pan to a weight gain of 7.5wt% (percentages are weight percentages relative to the tablet core). The holes were punched using mechanical punching or laser punching, and the diameter of the drug-release hole was 1.0 mm. The specification of the controlled-release tablet in this example was 250 mg of levodopa, a capsule-shaped tablet having a cross-sectional diameter of 6 mm and a height of 14.90 mm.
5. Dissolution test method: Same as in Example 1. The specific test results are shown in Table 18 and FIG. 18.

**Table 18**

| Sampling point/h | Prescription I (HPC: Sorbitol = 1:1) BN-205019-A | | Prescription II (HPC: Sorbitol = 1:1.5) BN-205019-B | |
|---|---|---|---|---|
| | Dissolution | SD | Dissolution | SD |
| 0 | 0.0% | 0.0% | 0.0% | 0.0% |
| 1 | 0.8% | 0.3% | 0.6% | 0.0% |
| 2 | 8.5% | 7.5% | 4.2% | 3.7% |
| 4 | 41.6% | 6.5% | 31.7% | 6.8% |
| 6 | 76.4% | 6.6% | 69.9% | 6.5% |
| 8 | 92.8% | 0.7% | 91.1% | 2.9% |
| 10 | 95.1% | 1.3% | 94.6% | 0.8% |
| 12 | 96.1% | 1.6% | 96.1% | 0.5% |
| 16 | 97.1% | 2.6% | 98.6% | 3.0% |

### Example 18: Capsule-shaped tablets (with a non-circular cross section, and compressed in a direction perpendicular to the long axis) (CD/LD) with specifications of 31.3 mg/250 mg

The prescription is as follows:

| (1) | Drug-containing layer prescription (per tablet) | mg/tablet | Proportion wt% |
|---|---|---|---|
| | Levodopa | 250.0 | 55.5 |
| | Carbidopa monohydrate | 33.8 | 7.50 |
| | Hydroxypropyl cellulose | 45.1 | 10.0 |
| | Poloxamer 407 | 45.1 | 10.0 |
| | Sorbitol | 45.1 | 10.0 |
| | Povidone | 20.3 | 4.50 |
| | Magnesium stearate | 9.00 | 2.00 |
| | Silica | 2.25 | 0.50 |

| (2) | Push layer prescription (per tablet) | mg/tablet | Proportion wt% |
|---|---|---|---|
| | Sodium carboxymethyl cellulose | 89.8 | 49.0 |
| | Sorbitol | 36.6 | 30.0 |
| | Hydroxypropyl cellulose | 55.0 | 20.0 |
| | Iron oxide red | 0.92 | 0.50 |
| | Magnesium stearate | 0.92 | 0.50 |

| (3) | Controlled-release membrane prescription (4.0% solid content in prescription) | | |
|---|---|---|---|
| | Cellulose acetate | 24.0 g | |
| | Copovidone | 14.0 g | |
| | Polyethylene glycol 400 | 2.00 g | |
| | Acetone | 900 mL | |
| | Ethanol | 100 mL | |

### Preparation method:

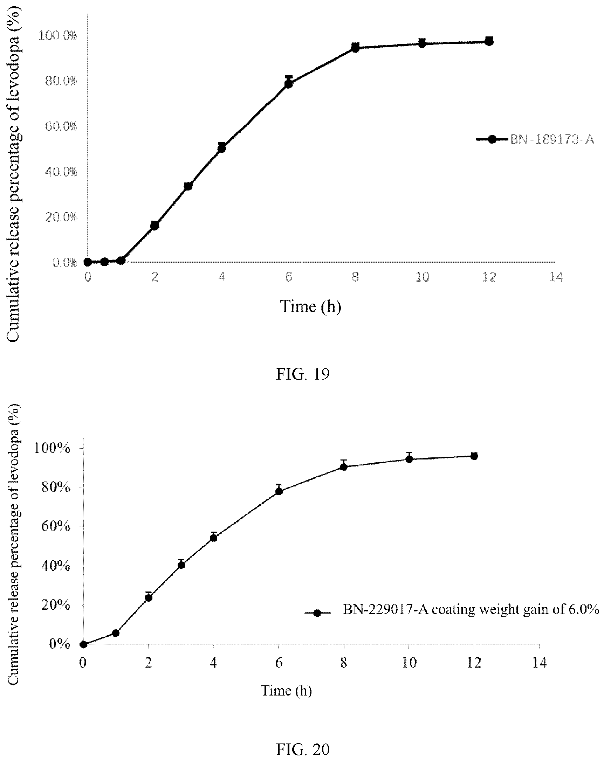

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 16 * 7 mm; 450.7 mg of drug-containing layer material was first filled and pre-compressed, followed by the filling of 183.3 mg of push layer material, and the final compression was performed to obtain a two-layer tablet core with a weight of 634.0 mg/tablet compressed in a direction perpendicular to the long axis.
4. Semi-permeable membrane coating: The two-layer tablet core was coated with the above controlled-release coating solution using a coating pan to a weight gain of 5.9wt% (percentages are weight percentages relative to the tablet core). The holes were punched using mechanical punching or laser punching, and the diameter of the drug-release hole was 1.00 mm. The specifications of the controlled-release tablets in this example were 31.3 mg of carbidopa and 250 mg of levodopa.
5. Dissolution test method: Same as in Example 1. The specific test results of levodopa are shown in Table 19 and FIG. 19.

**Table 19**

| Sampling point/h | BN-189173-A | SD | Sampling point/h | BN-189173-A | SD |
|---|---|---|---|---|---|
| 0 | 0.0% | 0.0% | 8 | 50.0% | 2.3% |
| 1 | 0.1% | 0.0% | 10 | 78.6% | 3.1% |
| 2 | 0.7% | 0.3% | 12 | 94.3% | 2.0% |
| 4 | 15.8% | 1.5% | 16 | 96.2% | 1.9% |
| 6 | 33.4% | 1.2% | | | |

### Example 19: Capsule-shaped tablets (with a non-circular cross section, and compressed in a direction perpendicular to the long axis) (CD/LD) with specifications of 31.3 mg/250 mg

The prescription is as follows:

| (1) | Drug-containing layer prescription (per tablet) | mg/tablet | Proportion wt% |
|---|---|---|---|
| | Levodopa | 250.0 | 55.5 |
| | Carbidopa monohydrate | 33.8 | 7.50 |
| | Hydroxypropyl cellulose | 67.6 | 15.0 |
| | Sorbitol | 67.6 | 15.0 |
| | Povidone | 18.8 | 4.17 |
| | Butylated hydroxytoluene | 1.50 | 0.33 |
| | Magnesium stearate | 9.01 | 2.00 |
| | Silica | 2.25 | 0.50 |

| (2) | Push layer prescription (per tablet) | mg/tablet | Proportion wt% |
|---|---|---|---|
| | Sodium carboxymethyl cellulose | 89.8 | 49.0 |
| | Sorbitol | 55.0 | 30.0 |
| | Hydroxypropyl cellulose | 36.6 | 20.0 |
| | Iron oxide red | 0.92 | 0.50 |
| | Magnesium stearate | 0.92 | 0.50 |

| (3) | Controlled-release membrane prescription (4.0% solid content in prescription) | | |
|---|---|---|---|
| | Cellulose acetate | 24.0 g | |
| | Copovidone | 16.0 g | |
| | Acetone | 900 mL | |
| | Ethanol | 100 mL | |

### Preparation method:

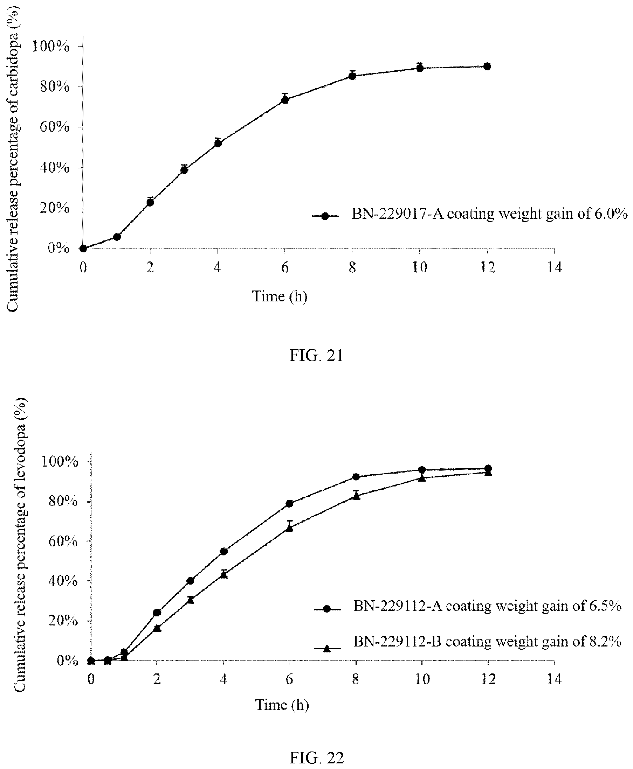

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 16 * 7 mm; 450.6 mg of drug-containing layer material was first filled and pre-compressed, followed by the filling of 183.2 mg of push layer material, and the final compression was performed to obtain a two-layer tablet core with a weight of 633.8 mg/tablet compressed in a direction perpendicular to the long axis.
4. Semi-permeable membrane coating: The two-layer tablet core was coated with the above controlled-release coating solution using a coating pan to a weight gain of 5.9wt% (percentages are weight percentages relative to the tablet core). The holes were punched using mechanical punching or laser punching, and the diameter of the drug-release hole was 1.00 mm. The specifications of the controlled-release tablets in this example were 31.3 mg of carbidopa and 250 mg of levodopa.
5. Dissolution test method: Same as in Example 1. Specific levodopa test results are shown in Table 20 and FIG. 20, and carbidopa test results are shown in Table 21 and FIG. 21.

**Table 20**

| Sampling point/h | BN-229017-A coating weight gain of 6.0% | SD | Sampling point/h | BN-229017-A coating weight gain of 6.0% | SD |
|---|---|---|---|---|---|
| 0 | 0.0% | 0.0% | 6 | 77.8% | 3.5% |
| 1 | 5.5% | 0.9% | 8 | 90.4% | 3.5% |
| 2 | 23.7% | 3.0% | 10 | 94.3% | 3.7% |
| 3 | 40.2% | 3.2% | 12 | 95.8% | 1.8% |
| 4 | 54.3% | 2.8% | | | |

**Table 21**

| Sampling point/h | BN-229017-A coating weight gain of 6.0% | SD | Sampling point/h | BN-229017-A coating weight gain of 6.0% | SD |
|---|---|---|---|---|---|
| 0 | 0.0% | 0.0% | 6 | 73.6% | 2.9% |
| 1 | 5.5% | 0.9% | 8 | 85.2% | 2.6% |
| 2 | 22.7% | 2.6% | 10 | 89.2% | 2.5% |
| 3 | 38.7% | 2.6% | 12 | 90.2% | 1.1% |
| 4 | 52.0% | 2.5% | | | |

### Example 20:

### 1. Capsule-shaped tablets (250 mg specification) were prepared using the formulation of capsule-shaped tablets of Example 11 having a circular cross-section and compressed in the long-axis direction

The prescription is as follows:

| (1) | Drug-containing layer prescription (per tablet) | mg/tablet | Proportion wt% |
|---|---|---|---|
| | Levodopa | 250.0 | 63.0 |
| | Hydroxypropyl cellulose | 39.7 | 10.0 |
| | Sorbitol | 77.4 | 19.5 |
| | Poloxamer 407 | 19.8 | 5.00 |
| | Magnesium stearate | 7.93 | 2.00 |
| | Silica | 1.98 | 0.50 |

| (2) | Push layer prescription (per tablet) | mg/tablet | Proportion wt% |
|---|---|---|---|
| | Sodium carboxymethyl cellulose | 111.3 | 69.0 |
| | Sorbitol | 16.1 | 10.0 |
| | Hydroxypropyl cellulose | 32.3 | 20.0 |
| | Iron oxide red | 0.81 | 0.50 |
| | Magnesium stearate | 0.81 | 0.50 |

| (3) | Controlled-release membrane prescription (4.0% solid content in prescription) | | |
|---|---|---|---|
| | Cellulose acetate | 24.0 g | |
| | Copovidone | 14.0 g | |
| | Polyethylene glycol 400 | 2.00 g | |
| | Acetone | 900 mL | |
| | Ethanol | 100 mL | |

### Preparation method:

1. Preparation of the drug-containing layer: Same as in Example 11.
2. Preparation of the push layer: Same as in Example 11.
3. Tablet compressing: Two-layer tablets were compressed with a die size of 16 * 7 mm; 396.8 mg of drug-containing layer material was first filled and pre-compressed, followed by the filling of 161.3 mg of push layer material, and the final compression was performed to obtain a two-layer tablet core with a weight of 558.1 mg/tablet compressed in a direction perpendicular to the long axis.
4. Semi-permeable membrane coating: The two-layer tablet core was coated with the above controlled-release coating solution using a coating pan to weight gains of 7.5wt% and 8.9wt% (percentages are weight percentages relative to the tablet core). The holes were punched using mechanical punching or laser punching, and the diameter of the drug-release hole was 1.00 mm. The specification of the controlled-release tablet in this example was 250 mg of levodopa.
5. Dissolution test method: Same as in Example 1.

### 2. Capsule-shaped tablets with a non-circular cross-section and compressed in a direction perpendicular to the long axis (125 mg specification) were prepared using the formulation of capsule-shaped tablets of Example 15 having a circular cross-section and compressed in the long-axis direction

The prescription is as follows:

| (1) | Drug-containing layer prescription (per tablet) | mg/tablet | Proportion wt% |
|---|---|---|---|
| | Levodopa | 125.0 | 31.5 |
| | Hydroxypropyl cellulose | 39.7 | 10.0 |
| | Mannitol | 123.0 | 31.0 |
| | Sorbitol | 79.4 | 20.0 |
| | Poloxamer 407 | 19.8 | 5.00 |
| | Magnesium stearate | 7.93 | 2.00 |
| | Silica | 1.98 | 0.50 |

| (2) | Push layer prescription (per tablet) | mg/tablet | Proportion wt% |
|---|---|---|---|
| | Sodium carboxymethyl cellulose | 111.3 | 69.0 |
| | Sorbitol | 16.1 | 10.0 |
| | Hydroxypropyl cellulose | 32.3 | 20.0 |
| | Iron oxide red | 0.81 | 0.50 |
| | Magnesium stearate | 0.81 | 0.50 |

| (3) | Controlled-release membrane prescription (4.0% solid content in prescription) | | |
|---|---|---|---|
| | Cellulose acetate | 24.0 g | |
| | Copovidone | 14.0 g | |
| | Polyethylene glycol 400 | 2.00 g | |
| | Acetone | 900 mL | |
| | Ethanol | 100 mL | |

### Preparation method:

1. Preparation of the drug-containing layer: Same as in Example 11.
2. Preparation of the push layer: Same as in Example 11.
3. Tablet compressing: Two-layer tablets were compressed with a die size of 16 * 7 mm; 396.8 mg of drug-containing layer material was first filled and pre-compressed, followed by the filling of 161.3 mg of push layer material, and the final compression was performed to obtain a two-layer tablet core with a weight of 558.1 mg/tablet compressed in a direction perpendicular to the long axis.
4. Semi-permeable membrane coating: The two-layer tablet core was coated with the above controlled-release coating solution using a coating pan to weight gains of 7.5wt% and 9.2wt% (percentages are weight percentages relative to the tablet core). The holes were punched using mechanical punching or laser punching, and the diameter of the drug-release hole was 1.00 mm. The specification of the controlled-release tablet in this example was 125 mg of levodopa.
5. Dissolution test method: Same as in Example 1.

### Example 21: Capsule-shaped tablets (with a non-circular cross section, and compressed in a direction perpendicular to the long axis) (CD/LD) with specifications of 31.3 mg/250 mg

The prescription is as follows:

| (1) | Drug-containing layer prescription (per tablet) | mg/tablet | Proportion wt% |
|---|---|---|---|
| | Levodopa | 250.0 | 51.1 |
| | Carbidopa monohydrate | 33.8 | 6.91 |
| | Hydroxypropyl cellulose | 146.8 | 30.0 |
| | Sorbitol | 24.5 | 5.00 |
| | Povidone | 20.5 | 4.19 |
| | Butylated hydroxytoluene | 1.50 | 0.31 |
| | Magnesium stearate | 9.79 | 2.00 |
| | Silica | 2.45 | 0.50 |

| (2) | Push layer prescription (per tablet) | mg/tablet | Proportion wt% |
|---|---|---|---|
| | Sodium carboxymethyl cellulose | 97.5 | 49.0 |
| | Sorbitol | 59.7 | 30.0 |
| | Hydroxypropyl cellulose | 39.8 | 20.0 |
| | Iron oxide red | 1.0 | 0.50 |
| | Magnesium stearate | 1.0 | 0.50 |

| (3) | Controlled-release membrane prescription (4.0% solid content in prescription) | | |
|---|---|---|---|
| | Cellulose acetate | 24.0 g | |
| | Copovidone | 16.0 g | |
| | Acetone | 900 mL | |
| | Ethanol | 100 mL | |

### Preparation method:

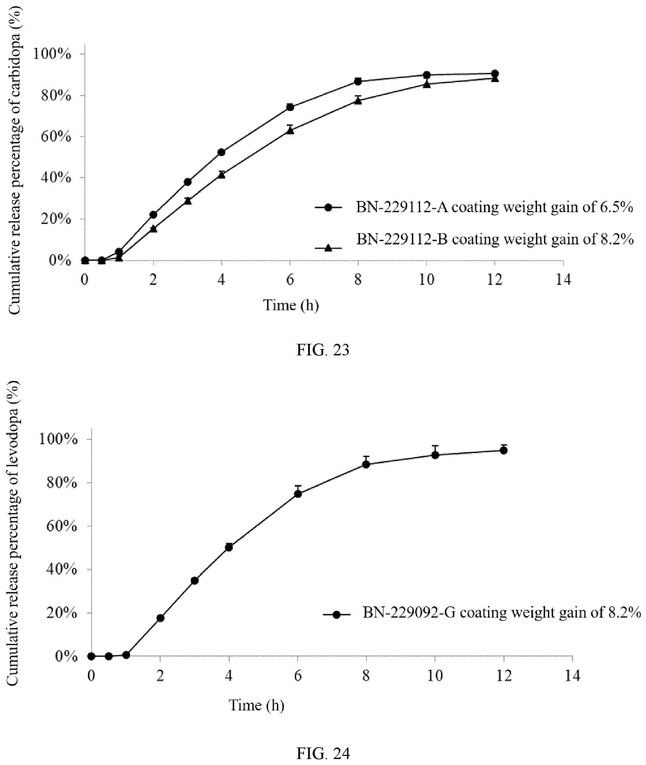

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 17.5 * 7.5 mm; 489.3 mg of drug-containing layer material was first filled and pre-compressed, followed by the filling of 198.9 mg of push layer material, and the final compression was performed to obtain a two-layer tablet core with a weight of 688.2 mg/tablet compressed in a direction perpendicular to the long axis.
4. Semi-permeable membrane coating: The two-layer tablet core was coated with the above controlled-release coating solution using a coating pan to weight gains of 6.5wt% and 8.2wt% (percentages are weight percentages relative to the tablet core). The holes were punched using mechanical punching or laser punching, and the diameter of the drug-release hole was 1.00 mm. The specifications of the controlled-release tablets in this example were 31.3 mg of carbidopa and 250 mg of levodopa.
5. Dissolution test method: Same as in Example 1. Specific levodopa test results are shown in Table 22 and FIG. 22, and carbidopa test results are shown in Table 23 and FIG. 23.

**Table 22**

| Sampling point/h | BN-229112-A coating weight gain of .5% | SD | BN-229112-B coating weight gain of 8.2% | SD |
|---|---|---|---|---|
| 0 | 0.0% | 0.0% | 0.0% | 0.0% |
| 0.5 | 0.2% | 0.0% | 0.0% | 0.0% |
| 1 | 4.3% | 1.1% | 1.7% | 0.4% |
| 2 | 24.0% | 0.4% | 16.2% | 0.7% |
| 3 | 40.1% | 0.5% | 30.5% | 1.5% |
| 4 | 55.0% | 1.2% | 43.4% | 2.1% |
| 6 | 78.9% | 1.6% | 66.9% | 3.5% |
| 8 | 92.5% | 1.4% | 82.8% | 2.6% |
| 10 | 95.9% | 0.4% | 91.8% | 3.2% |
| 12 | 96.6% | 0.1% | 94.8% | 1.5% |

**Table 23**

| Sampling point/h | BN-229112-A coating weight gain of 6.5% | SD | BN-229112-B coating weight gain of 8.2% | SD |
|---|---|---|---|---|
| 0 | 0.0% | 0.0% | 0.0% | 0.0% |
| 0.5 | 0.0% | 0.0% | 0.0% | 0.0% |
| 1 | 4.1% | 1.0% | 1.5% | 0.4% |
| 2 | 22.4% | 0.4% | 15.4% | 0.4% |
| 3 | 38.0% | 0.4% | 29.0% | 1.3% |
| 4 | 52.5% | 0.9% | 41.5% | 1.7% |
| 6 | 74.1% | 1.8% | 62.9% | 2.8% |
| 8 | 86.9% | 1.6% | 77.5% | 2.2% |
| 10 | 90.0% | 0.0% | 85.5% | 3.0% |
| 12 | 90.6% | 0.1% | 88.4% | 1.4% |

### Example 22: Capsule-shaped tablets (with a non-circular cross section, and compressed in a direction perpendicular to the long axis) (CD/LD) with specifications of 31.3 mg/250 mg

The prescription is as follows:

| (1) | Drug-containing layer prescription (per tablet) | mg/tablet | Proportion wt% |
|---|---|---|---|
| | Levodopa | 250.0 | 55.5 |
| | Carbidopa monohydrate | 33.8 | 7.50 |
| | Hydroxypropyl cellulose | 45.1 | 10.0 |
| | Poloxamer | 45.1 | 10.0 |
| | Sorbitol | 45.1 | 10.0 |
| | Povidone | 18.8 | 4.17 |
| | Butylated hydroxytoluene | 1.50 | 0.33 |
| | Magnesium stearate | 9.01 | 2.00 |
| | Silica | 2.25 | 0.50 |

| (2) | Push layer prescription (per tablet) | mg/tablet | Proportion wt% |
|---|---|---|---|
| | Sodium carboxymethyl cellulose | 89.8 | 49.0 |
| | Sorbitol | 54.9 | 30.0 |
| | Hydroxypropyl cellulose | 36.7 | 20.0 |
| | Iron oxide red | 0.92 | 0.50 |
| | Magnesium stearate | 0.92 | 0.50 |

| (3) | Controlled-release membrane prescription (4.0% solid content in prescription) | | |
|---|---|---|---|
| | Cellulose acetate | 24.0 g | |
| | Copovidone | 16.0 g | |
| | Acetone | 900 mL | |
| | Ethanol | 100 mL | |

### Preparation method:

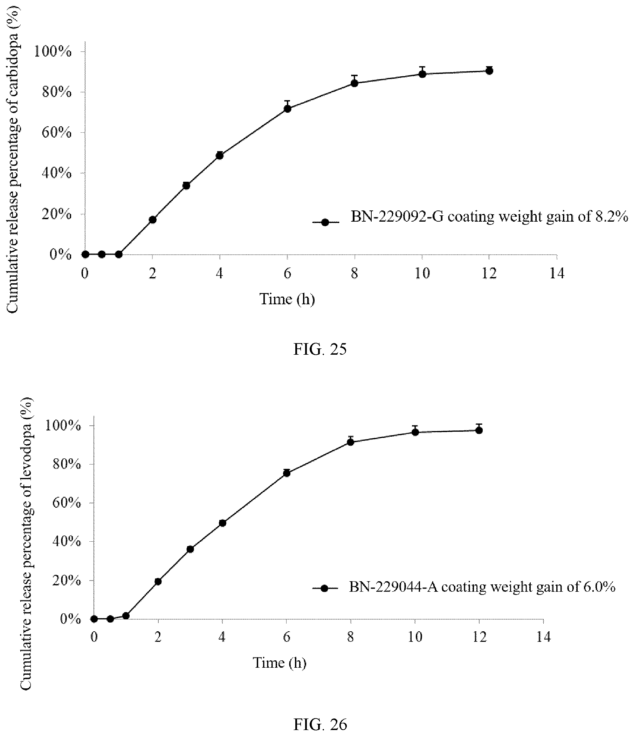

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 16 * 7 mm (deep concave); 450.6 mg of drug-containing layer material was first filled and pre-compressed, followed by the filling of 183.3 mg of push layer material, and the final compression was performed to obtain a two-layer tablet core with a weight of 633.9 mg/tablet compressed in a direction perpendicular to the long axis.
4. Semi-permeable membrane coating: The two-layer tablet core was coated with the above controlled-release coating solution using a coating pan to a weight gain of 8.2wt% (percentages are weight percentages relative to the tablet core). The holes were punched using mechanical punching or laser punching, and the diameter of the drug-release hole was 1.00 mm. The specifications of the controlled-release tablets in this example were 31.3 mg of carbidopa and 250 mg of levodopa.
5. Dissolution test method: Same as in Example 1. Specific levodopa test results are shown in Table 24 and FIG. 24, and carbidopa test results are shown in Table 25 and FIG. 25.

**Table 24**

| Sampling point/h | BN-229092-G coating weight gain of 8.2% | SD | Sampling point/h | BN-229092-G coating weight gain of 8.2% | SD |
|---|---|---|---|---|---|
| 0 | 0.0% | 0.0% | 4 | 50.1% | 1.8% |
| 0.5 | 0.0% | 0.0% | 6 | 75.0% | 3.7% |
| 1 | 0.6% | 0.2% | 8 | 88.5% | 3.8% |
| 2 | 17.7% | 0.9% | 10 | 92.9% | 4.2% |
| 3 | 35.0% | 1.4% | 12 | 94.9% | 2.7% |

**Table 25**

| Sampling point/h | BN-229092-G coating weight gain of 8.2% | SD | Sampling point/h | BN-229092-G coating weight gain of 8.2% | SD |
|---|---|---|---|---|---|
| 0 | 0.0% | 0.0% | 4 | 48.5% | 2.0% |
| 0.5 | 0.0% | 0.0% | 6 | 71.7% | 3.7% |
| 1 | 0.0% | 0.0% | 8 | 84.3% | 3.8% |
| 2 | 17.2% | 0.8% | 10 | 88.9% | 3.5% |
| 3 | 33.8% | 1.5% | 12 | 90.4% | 1.9% |

### Example 23: Capsule-shaped tablets (with a non-circular cross section, and compressed in a direction perpendicular to the long axis) (CD/LD) with specifications of 62.5 mg/250 mg

The prescription is as follows:

| (1) | Drug-containing layer prescription (per tablet) | mg/tablet | Proportion wt% |
|---|---|---|---|
| | Levodopa | 250.0 | 55.5 |
| | Carbidopa monohydrate | 33.8 | 7.50 |
| | Hydroxypropyl cellulose | 67.6 | 15.0 |

| (2) | Push layer prescription (per tablet) | mg/tablet | Proportion wt% |
|---|---|---|---|
| | Sodium carboxymethyl cellulose | 89.8 | 49.0 |
| | Sorbitol | 55.0 | 30.0 |
| | Hydroxypropyl cellulose | 36.6 | 20.0 |
| | Iron oxide red | 0.92 | 0.50 |
| | Magnesium stearate | 0.92 | 0.50 |

| (3) | Controlled-release membrane prescription (4.0% solid content in prescription) | | |
|---|---|---|---|
| | Cellulose acetate | 24.0 g | |
| | Copovidone | 16.0 g | |
| | Acetone | 900 mL | |
| | Ethanol | 100 mL | |

| (4) | Immediate-release prescription (10.0% solid content in prescription) | | |
|---|---|---|---|
| | Immediate-release layer prescription (per tablet) | mg/tablet | Proportion wt% |
| | Carbidopa monohydrate | 33.7 | 86.2 |
| | Hydroxypropyl cellulose | 3.91 | 10.0 |
| | Butylated hydroxytoluene | 1.50 | 3.84 |
| | Ethanol | 720.9 g | |

### Preparation method:

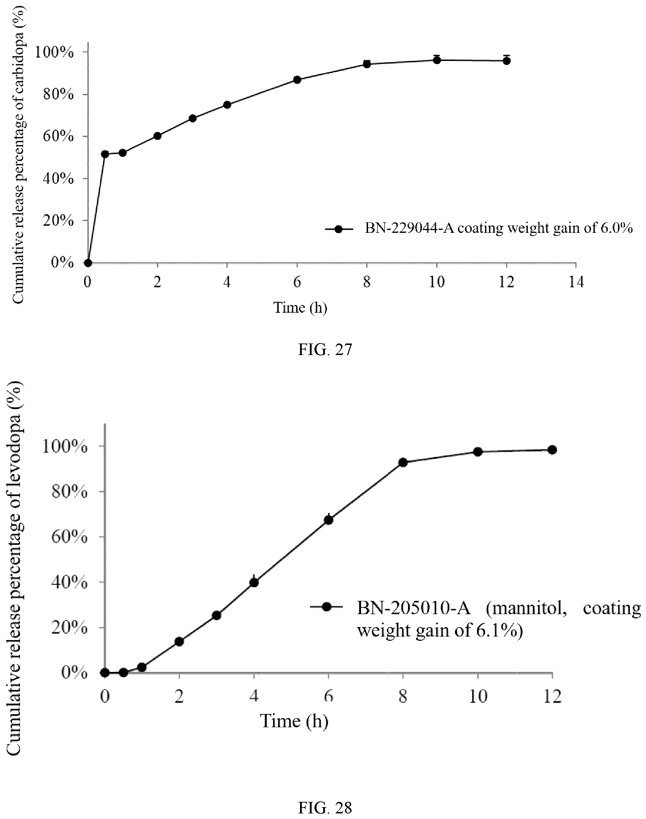

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 16 * 7 mm; 450.6 mg of drug-containing layer material was first filled and pre-compressed, followed by the filling of 183.2 mg of push layer material, and the final compression was performed to obtain a two-layer tablet core with a weight of 633.8 mg/tablet compressed in a direction perpendicular to the long axis.
4. Semi-permeable membrane coating: The two-layer tablet core was coated with the above controlled-release coating solution using a coating pan to a weight gain of 6.0wt% (percentages are weight percentages relative to the tablet core). The holes were punched using mechanical punching or laser punching, and the diameter of the drug-release hole was 1.00 mm. The specifications of the controlled-release tablets in this example were 31.3 mg of carbidopa and 250 mg of levodopa.
5. Immediate-release coating: The tablet core coated by the controlled-release coating solution was coated with the above immediate-release coating solution using a coating pan to a weight gain of 39.1 mg per tablet. The specifications of the controlled-release tablets in this example were 62.5 mg of carbidopa and 250 mg of levodopa.
6. Dissolution test method: Same as in Example 1. Specific levodopa test results are shown in Table 26 and FIG. 26, and carbidopa test results are shown in Table 27 and FIG. 27.

**Table 26**

| Sampling point/h | BN-229044-A coating weight gain of 6.0% | SD | Sampling point/h | BN-229044-A coating weight gain of 6.0% | SD |
|---|---|---|---|---|---|
| 0 | 0.0% | 0.0% | 4 | 49.6% | 1.5% |
| 0.5 | 0.1% | 0.1% | 6 | 75.4% | 1.9% |
| 1 | 1.6% | 0.4% | 8 | 91.3% | 3.0% |
| 2 | 19.2% | 1.4% | 10 | 96.5% | 3.4% |
| 3 | 36.0% | 1.4% | 12 | 97.3% | 3.4% |

**Table 27**

| Sampling point/h | BN-229044-A coating weight gain of 6.0% | SD | Sampling point/h | BN-229044-A coating weight gain of 6.0% | SD |
|---|---|---|---|---|---|
| 0 | 0.0% | 0.0% | 4 | 74.9% | 0.7% |
| 0.5 | 51.4% | 1.4% | 6 | 86.8% | 1.3% |
| 1 | 52.1% | 1.0% | 8 | 94.3% | 1.6% |
| 2 | 60.3% | 0.9% | 10 | 96.3% | 2.2% |
| 3 | 68.5% | 0.7% | 12 | 96.0% | 2.5% |

### Example 24: Capsule-shaped tablets (with a non-circular cross section, and compressed in a direction perpendicular to the long axis) (CD/LD) with specifications of 15.7 mg/125 mg

The prescription is as follows:

| (1) | Drug-containing layer prescription (per tablet) | mg/tablet | Proportion wt% |
|---|---|---|---|
| | Levodopa | 125.0 | 27.7 |
| | Carbidopa monohydrate | 16.9 | 3.75 |
| | Mannitol 200SD | 142.0 | 31.5 |
| | Hydroxypropyl cellulose EXF | 45.1 | 10.0 |
| | Poloxamer 407 ≤ 80 mesh | 45.1 | 10.0 |
| | Sorbitol ≤ 80 mesh | 45.1 | 10.0 |
| | Povidone K29/32 | 20.3 | 4.50 |
| | Magnesium stearate | 9.01 | 2.00 |
| | Silica | 6.76 | 1.50 |

| (2) | Push layer prescription (per tablet) | mg/tablet | Proportion wt% |
|---|---|---|---|
| | Sodium carboxymethyl cellulose | 89.8 | 49.0 |
| | Sorbitol ≤ 80 mesh | 54.9 | 30.0 |
| | Hydroxypropyl cellulose | 36.7 | 20.0 |
| | Iron oxide red | 0.92 | 0.50 |
| | Magnesium stearate | 0.92 | 0.50 |

| (3) | Controlled-release membrane prescription (4.0% solid content in prescription) | | |
|---|---|---|---|
| | Cellulose acetate | 26.0 g | |
| | Copovidone | 14.0 g | |
| | Acetone | 900 mL | |
| | Ethanol | 100 mL | |

### Preparation method:

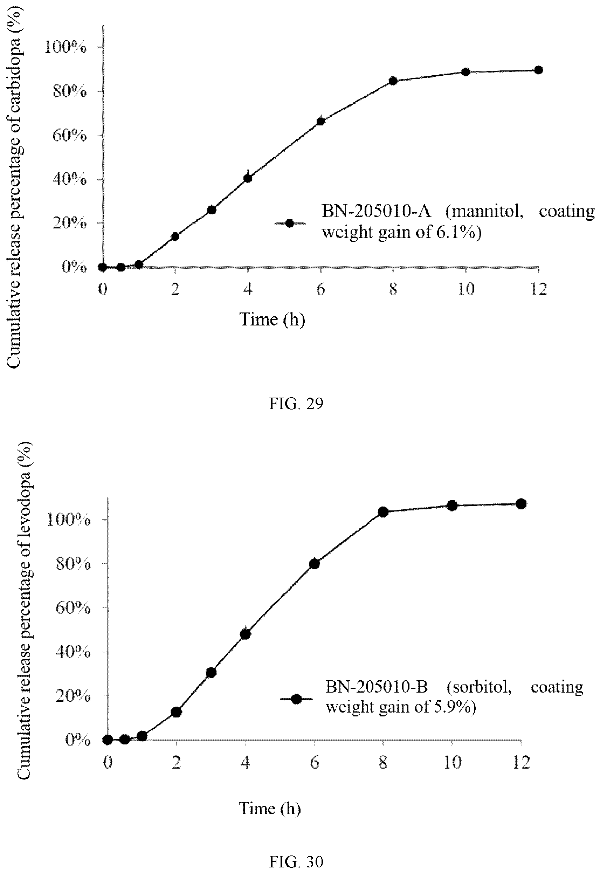

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 16 * 7 mm (deep concave); 450.6 mg of drug-containing layer material was first filled and pre-compressed, followed by the filling of 183.3 mg of push layer material, and the final compression was performed to obtain a two-layer tablet core with a weight of 633.9 mg/tablet.
4. Semi-permeable membrane coating: The two-layer tablet core was coated with the above controlled-release coating solution using a coating pan to a weight gain of 6.1wt% (percentages are weight percentages relative to the tablet core). The holes were punched using mechanical punching or laser punching, and the diameter of the drug-release hole was 1.00 mm. The specifications of the controlled-release tablets in this example were 15.7 mg of carbidopa and 125 mg of levodopa.
5. Dissolution test method: Same as in Example 1. Specific levodopa test results are shown in Table 28 and FIG. 28, and carbidopa test results are shown in Table 29 and FIG. 29.

**Table 28**

| Sampling point/h | BN-205010-A (mannitol, coating weight gain of 6.1%) | SD | Sampling point/h | BN-205010-A (mannitol, coating weight gain of 6.1%) | SD |
|---|---|---|---|---|---|
| 0 | 0.0% | 0.0% | 4 | 39.7% | 3.4% |
| 0.5 | 0.1% | 0.0% | 6 | 67.4% | 2.9% |
| 1 | 2.4% | 0.4% | 8 | 92.8% | 0.9% |
| 2 | 13.7% | 0.2% | 10 | 97.5% | 1.1% |
| 3 | 25.3% | 1.2% | 12 | 98.3% | 0.8% |

**Table 29**

| Sampling point/h | BN-205010-A (mannitol, coating weight gain of 6.1%) | SD | Sampling point/h | BN-205010-A (mannitol, coating weight gain of 6.1%) | SD |
|---|---|---|---|---|---|
| 0 | 0.0% | 0.0% | 4 | 40.4% | 4.0% |
| 0.5 | 0.0% | 0.0% | 6 | 66.3% | 3.3% |
| 1 | 1.2% | 1.7% | 8 | 84.7% | 0.9% |
| 2 | 13.8% | 0.9% | 10 | 88.7% | 1.7% |
| 3 | 26.1% | 2.5% | 12 | 89.6% | 1.3% |

### Example 25: Capsule-shaped tablets (with a non-circular cross section, and compressed in a direction perpendicular to the long axis) (CD/LD) with specifications of 15.6 mg/125 mg

The prescription is as follows:

| (1) | Drug-containing layer prescription (per tablet) | mg/tablet | Proportion wt% |
|---|---|---|---|
| | Levodopa | 125.0 | 27.7 |
| | Carbidopa monohydrate | 16.9 | 3.75 |
| | Hydroxypropyl cellulose EXF | 45.1 | 10.0 |
| | Poloxamer 407 ≤ 80 mesh | 45.1 | 10.0 |
| | Sorbitol ≤ 80 mesh | 187.1 | 41.5 |
| | Povidone K29/32 | 20.3 | 4.50 |
| | Magnesium stearate | 9.01 | 2.00 |
| | Silica | 6.76 | 1.50 |

| (2) | Push layer prescription (per tablet) | mg/tablet | Proportion wt% |
|---|---|---|---|
| | Sodium carboxymethyl cellulose | 89.8 | 49.0 |
| | Sorbitol ≤ 80 mesh | 54.9 | 30.0 |
| | Hydroxypropyl cellulose | 36.7 | 20.0 |
| | Iron oxide red | 0.92 | 0.50 |
| | Magnesium stearate | 0.92 | 0.50 |

| (3) | Controlled-release membrane prescription (4.0% solid content in prescription) | | |
|---|---|---|---|
| | Cellulose acetate | 26.0 g | |
| | Copovidone | 14.0 g | |
| | Acetone | 900 mL | |
| | Ethanol | 100 mL | |

### Preparation method:

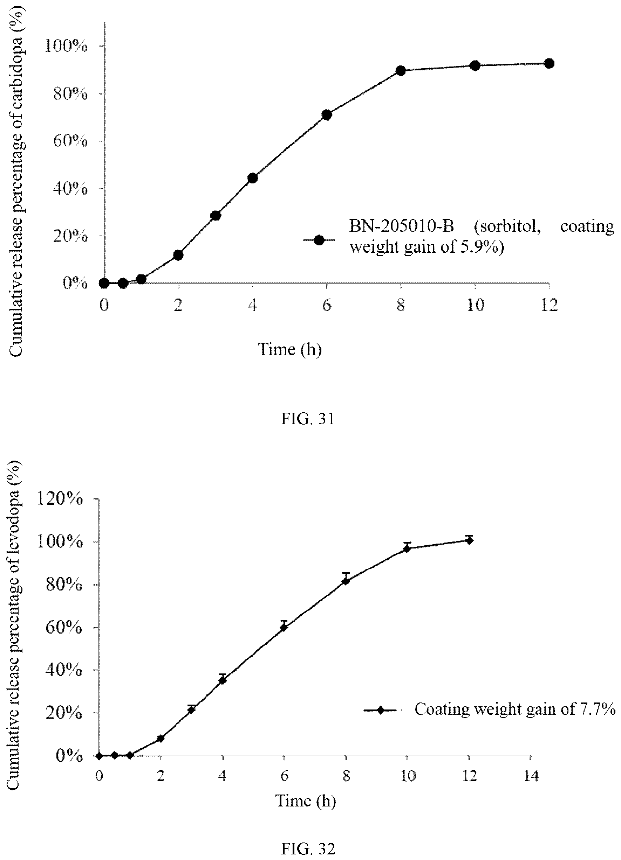

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 16 * 7 mm (deep concave); 450.6 mg of drug-containing layer material was first filled and pre-compressed, followed by the filling of 183.3 mg of push layer material, and the final compression was performed to obtain a two-layer tablet core with a weight of 633.9 mg/tablet compressed in a direction perpendicular to the long axis.
4. Semi-permeable membrane coating: The two-layer tablet core was coated with the above controlled-release coating solution using a coating pan to a weight gain of 5.9wt% (percentages are weight percentages relative to the tablet core). The holes were punched using mechanical punching or laser punching, and the diameter of the drug-release hole was 1.00 mm. The specifications of the controlled-release tablets in this example were 15.7 mg of carbidopa and 125 mg of levodopa.
5. Dissolution test method: Same as in Example 1. Specific levodopa test results are shown in Table 30 and FIG. 30, and carbidopa test results are shown in Table 31 and FIG. 31.

**Table 30**

| Sampling point/h | BN-205010-B (sorbitol, coating weight gain of 5.9%) | SD |
|---|---|---|
| 0 | 0.0% | 0.0% |
| 0.5 | 0.2% | 0.0% |
| 1 | 1.7% | 0.1% |
| 2 | 12.6% | 1.0% |
| 3 | 30.5% | 2.2% |
| 4 | 48.2% | 3.5% |
| 6 | 80.0% | 3.2% |
| 8 | 103.5% | 0.6% |
| 10 | 106.3% | 2.0% |
| 12 | 107.1% | 2.4% |

**Table 31**

| Sampling point/h | BN-205010-B (sorbitol, coating weight gain of 5.9%) | SD |
|---|---|---|
| 0 | 0.0% | 0.0% |
| 0.5 | 0.0% | 0.0% |
| 1 | 1.7% | 0.2% |
| 2 | 12.0% | 0.7% |
| 3 | 28.6% | 1.7% |
| 4 | 44.3% | 2.9% |
| 6 | 71.1% | 1.9% |
| 8 | 89.6% | 1.1% |
| 10 | 91.7% | 1.9% |
| 12 | 92.7% | 2.1% |

### Example 26: Capsule-shaped tablets (with a non-circular cross section, and compressed in a direction perpendicular to the long axis) (CD/LD) with specifications of 62.5 mg/250 mg

The prescription is as follows:

| (1) | Drug-containing layer prescription (per tablet) | mg/tablet | Proportion wt% |
|---|---|---|---|
| | Levodopa | 250.0 | 55.5 |
| | Carbidopa monohydrate | 33.8 | 7.50 |
| | Hydroxypropyl cellulose | 67.6 | 15.0 |
| | Sorbitol | 67.6 | 15.0 |
| | Povidone | 18.8 | 4.17 |
| | Butylated hydroxytoluene | 1.50 | 0.33 |
| | Magnesium stearate | 9.01 | 2.00 |
| | Silica | 2.25 | 0.50 |

| (2) | Push layer prescription (per tablet) | mg/tablet | Proportion wt% |
|---|---|---|---|
| | Sodium carboxymethyl cellulose | 89.8 | 49.0 |
| | Sorbitol | 55.0 | 30.0 |
| | Hydroxypropyl cellulose | 36.6 | 20.0 |
| | Iron oxide red | 0.92 | 0.50 |
| | Magnesium stearate | 0.92 | 0.50 |

| (3) | Controlled-release membrane prescription (4.0% solid content in prescription) | | |
|---|---|---|---|
| | Cellulose acetate | 26.0 g | |
| | Copovidone | 14.0 g | |
| | Acetone | 900 mL | |
| | Ethanol | 100 mL | |

| (4) | Immediate-release prescription (10.0% solid content in prescription) | | |
|---|---|---|---|
| | Immediate-release layer prescription (per tablet) | mg/tablet | Proportion wt% |
| | Carbidopa monohydrate | 33.7 | 86.2 |
| | Hydroxypropyl cellulose | 3.91 | 10.0 |
| | Butylated hydroxytoluene | 1.50 | 3.84 |
| | Ethanol | 351.9 g | |

### Preparation method:

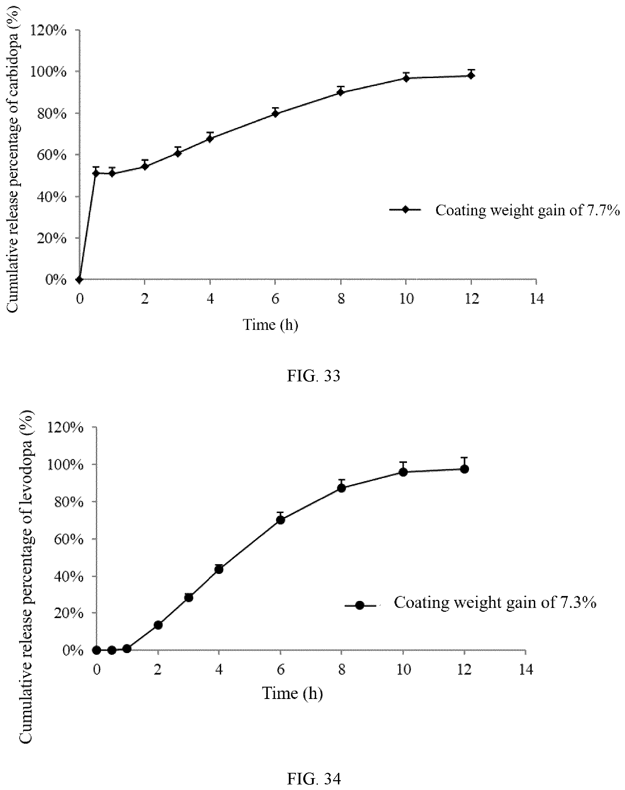

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 16 * 7 mm; 450.6 mg of drug-containing layer material was first filled and pre-compressed, followed by the filling of 183.2 mg of push layer material, and the final compression was performed to obtain a two-layer tablet core with a weight of 633.8 mg/tablet compressed in a direction perpendicular to the long axis.
4. Semi-permeable membrane coating: The two-layer tablet core was coated with the above controlled-release coating solution using a coating pan to a weight gain of 7.7wt% (percentages are weight percentages relative to the tablet core). The holes were punched using mechanical punching or laser punching, and the diameter of the drug-release hole was 1.00 mm. The specifications of the controlled-release tablets in this example were 31.3 mg of carbidopa and 250 mg of levodopa.
5. Immediate-release coating: The tablet core coated by the controlled-release coating solution was coated with the above immediate-release coating solution using a coating pan to a weight gain of 39.1 mg per tablet. The specifications of the controlled-release tablets in this example were 62.5 mg of carbidopa and 250 mg of levodopa.
6. Dissolution test method: Same as in Example 1. Specific levodopa test results are shown in Table 32 and FIG. 32, and carbidopa test results are shown in Table 33 and FIG. 33.

**Table 32**

| Sampling point/h | Coating weight gain of 7.7% | SD | Sampling point/h | Coating weight gain of 7.7% | SD |
|---|---|---|---|---|---|
| 0 | 0.0% | 0.0% | 4 | 35.2% | 2.7% |
| 0.5 | 0.2% | 0.0% | 6 | 59.8% | 3.2% |
| 1 | 0.4% | 0.1% | 8 | 81.6% | 3.7% |
| 2 | 8.0% | 1.0% | 10 | 96.9% | 2.6% |
| 3 | 21.3% | 2.0% | 12 | 100.5% | 2.4% |

**Table 33**

| Sampling point/h | Coating weight gain of 7.7% | SD | Sampling point/h | Coating weight gain of 7.7% | SD |
|---|---|---|---|---|---|
| 0 | 0.0% | 0.0% | 4 | 67.6% | 3.0% |
| 0.5 | 51.1% | 3.1% | 6 | 79.7% | 2.8% |
| 1 | 50.9% | 2.8% | 8 | 89.9% | 2.7% |
| 2 | 54.3% | 3.1% | 10 | 96.7% | 2.7% |
| 3 | 60.7% | 2.9% | 12 | 98.0% | 3.0% |

### Example 27: Capsule-shaped tablets (with a non-circular cross section, and compressed in a direction perpendicular to the long axis) (CD/LD) with specifications of 31.25 mg/125 mg

The prescription is as follows:

| (1) | Drug-containing layer prescription (per tablet) | mg/tablet | Proportion wt% |
|---|---|---|---|
| | Levodopa | 125.0 | 55.5 |
| | Carbidopa monohydrate | 16.9 | 7.50 |
| | Hydroxypropyl cellulose | 22.5 | 10.0 |
| | Sorbitol | 22.5 | 10.0 |
| | Poloxamer | 22.5 | 10.0 |
| | Povidone | 10.5 | 4.66 |
| | Butylated hydroxytoluene | 0.75 | 0.33 |
| | Magnesium stearate | 3.38 | 1.50 |
| | Silica | 1.13 | 0.50 |

| (2) | Push layer prescription (per tablet) | mg/tablet | Proportion wt% |
|---|---|---|---|
| | Sodium carboxymethyl | 44.8 | 49.0 |
| | cellulose | | |
| | Sorbitol | 27.5 | 30.0 |
| | Hydroxypropyl cellulose | 18.3 | 20.0 |
| | Iron oxide red | 0.46 | 0.50 |
| | Magnesium stearate | 0.46 | 0.50 |

| (3) | Controlled-release membrane prescription (4.0% solid content in prescription) | | |
|---|---|---|---|
| | Cellulose acetate | 28.0 g | |
| | Copovidone | 12.0 g | |
| | Acetone | 900 mL | |
| | Ethanol | 100 mL | |

| (4) | Immediate-release prescription (10.0% solid content in prescription) | | |
|---|---|---|---|
| | Immediate-release layer prescription (per tablet) | mg/tablet | Proportion wt% |
| | Carbidopa monohydrate | 16.8 | 85.3 |
| | Hydroxypropyl cellulose | 1.97 | 10.0 |
| | Triethyl citrate | 0.20 | 1.02 |
| | Butylated hydroxytoluene | 0.75 | 3.81 |
| | Ethanol | 354.6 g | |

### Preparation method:

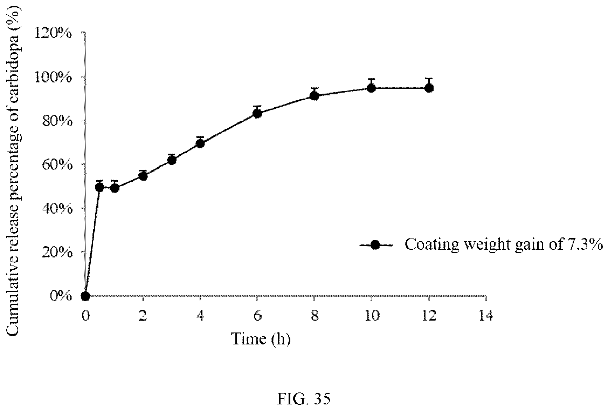

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 13 * 6 mm; 225.2 mg of drug-containing layer material was first filled and pre-compressed, followed by the filling of 91.5 mg of push layer material, and the final compression was performed to obtain a two-layer tablet core with a weight of 316.7 mg/tablet compressed in a direction perpendicular to the long axis.
4. Semi-permeable membrane coating: The two-layer tablet core was coated with the above controlled-release coating solution using a coating pan to a weight gain of 7.3wt% (percentages are weight percentages relative to the tablet core). The holes were punched using mechanical punching or laser punching, and the diameter of the drug-release hole was 1.00 mm. The specifications of the controlled-release tablets in this example were 15.6 mg of carbidopa and 125 mg of levodopa.
5. Immediate-release coating: The tablet core coated by the controlled-release coating solution was coated with the above immediate-release coating solution using a coating pan to a weight gain of 19.7 mg per tablet. The specifications of the controlled-release tablets in this example were 31.25 mg of carbidopa and 125 mg of levodopa.
6. Dissolution test method: Same as in Example 1. Specific levodopa test results are shown in Table 34 and FIG. 34, and carbidopa test results are shown in Table 35 and FIG. 35.

**Table 34**

| Sampling point/h | Coating weight gain of 7.3% | SD | Sampling point/h | Coating weight gain of 7.3% | SD |
|---|---|---|---|---|---|
| 0 | 0.0% | 0.0% | 4 | 43.4% | 2.6% |
| 0.5 | 0.2% | 0.1% | 6 | 70.3% | 3.7% |
| 1 | 0.9% | 0.1% | 8 | 87.5% | 4.3% |
| 2 | 13.6% | 0.9% | 10 | 95.9% | 5.5% |
| 3 | 28.5% | 1.8% | 12 | 97.4% | 6.1% |

**Table 35**

| Sampling point/h | Coating weight gain of 7.3% | SD | Sampling point/h | Coating weight gain of 7.3% | SD |
|---|---|---|---|---|---|
| 0 | 0.0% | 0.0% | 4 | 69.7% | 2.9% |
| 0.5 | 49.8% | 2.7% | 6 | 83.4% | 3.0% |
| 1 | 49.3% | 3.1% | 8 | 91.3% | 3.5% |
| 2 | 54.8% | 2.5% | 10 | 94.7% | 4.2% |
| 3 | 61.9% | 2.4% | 12 | 94.7% | 4.4% |

### Example 28: Capsule-shaped tablets (with a non-circular cross section, and compressed in a direction perpendicular to the long axis) (CD/LD) with specifications of 31.25 mg/125 mg

The prescription is as follows:

| (1) | Drug-containing layer prescription (per tablet) | mg/tablet | Proportion wt% |
|---|---|---|---|
| | Levodopa | 125.0 | 55.5 |
| | Carbidopa monohydrate | 16.9 | 7.50 |
| | Hydroxypropyl cellulose | 22.5 | 10.0 |
| | Sorbitol | 22.5 | 10.0 |
| | Poloxamer | 22.5 | 10.0 |
| | Povidone | 10.5 | 4.66 |
| | Butylated hydroxytoluene | 0.75 | 0.33 |
| | Magnesium stearate | 3.38 | 1.50 |
| | Silica | 1.13 | 0.50 |

| (2) | Push layer prescription (per tablet) | mg/tablet | Proportion wt% |
|---|---|---|---|
| | Sodium carboxymethyl cellulose | 44.8 | 49.0 |
| | Sorbitol | 27.5 | 30.0 |
| | Hydroxypropyl cellulose | 18.3 | 20.0 |
| | Iron oxide red | 0.46 | 0.50 |
| | Magnesium stearate | 0.46 | 0.50 |

| (3) | Controlled-release membrane prescription (4.0% solid content in prescription) | | |
|---|---|---|---|
| | Cellulose acetate | 28.0 g | |
| | Copovidone | 12.0 g | |
| | Acetone | 900 mL | |
| | Ethanol | 100 mL | |

| (4) | Immediate-release prescription (10.0% solid content in prescription) | | |
|---|---|---|---|
| | Immediate-release layer prescription (per tablet) | mg/tablet | Proportion wt% |
| | Carbidopa monohydrate | 16.8 | 74.7 |
| | Hydroxypropyl cellulose | 2.25 | 10.0 |
| | Copovidone | 2.25 | 10.0 |
| | Triethyl citrate | 0.45 | 2.00 |
| | Butylated hydroxytoluene | 0.75 | 3.38 |
| | Ethanol | 405.0 g | |

### Preparation method:

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 13 * 6 mm; 225.2 mg of drug-containing layer material was first filled and pre-compressed, followed by the filling of 91.5 mg of push layer material, and the final compression was performed to obtain a two-layer tablet core with a weight of 316.7 mg/tablet compressed in a direction perpendicular to the long axis.
4. Semi-permeable membrane coating: The two-layer tablet core was coated with the above controlled-release coating solution using a coating pan to a weight gain of 7.3wt% (percentages are weight percentages relative to the tablet core). The holes were punched using mechanical punching or laser punching, and the diameter of the drug-release hole was 1.00 mm. The specifications of the controlled-release tablets in this example were 15.6 mg of carbidopa and 125 mg of levodopa.
5. Immediate-release coating: The tablet core coated by the controlled-release coating solution was coated with the above immediate-release coating solution using a coating pan to a weight gain of 22.5 mg per tablet. The specifications of the controlled-release tablets in this example were 31.25 mg of carbidopa and 125 mg of levodopa.

### Example 29: Capsule-shaped tablets (with a non-circular cross section, and compressed in a direction perpendicular to the long axis) (CD/LD) with specifications of 31.25 mg/125 mg

The prescription is as follows:

| (1) | Drug-containing layer prescription (per tablet) | mg/tablet | Proportion wt% |
|---|---|---|---|
| | Levodopa | 125.0 | 55.5 |
| | Carbidopa monohydrate | 16.9 | 7.50 |
| | Hydroxypropyl cellulose | 22.5 | 10.0 |
| | Sorbitol | 22.5 | 10.0 |
| | Poloxamer | 22.5 | 10.0 |
| | Povidone | 10.5 | 4.66 |
| | Butylated hydroxytoluene | 0.75 | 0.33 |
| | Magnesium stearate | 3.38 | 1.50 |
| | Silica | 1.13 | 0.50 |

| (2) | Push layer prescription (per tablet) | mg/tablet | Proportion wt% |
|---|---|---|---|
| | Sodium carboxymethyl cellulose | 44.8 | 49.0 |
| | Sorbitol | 27.5 | 30.0 |
| | Hydroxypropyl cellulose | 18.3 | 20.0 |
| | Iron oxide red | 0.46 | 0.50 |
| | Magnesium stearate | 0.46 | 0.50 |

| (3) | Controlled-release membrane prescription (4.0% solid content in prescription) | | |
|---|---|---|---|
| | Cellulose acetate | 28.0 g | |
| | Copovidone | 12.0 g | |
| | Acetone | 900 mL | |
| | Ethanol | 100 mL | |

| (4) | Immediate-release prescription (10.0% solid content in prescription) | | |
|---|---|---|---|
| | Immediate-release layer prescription (per tablet) | mg/tablet | Proportion wt% |
| | Carbidopa monohydrate | 16.8 | 75.7 |
| | Hydroxypropyl cellulose | 2.22 | 10.0 |
| | Copovidone | 2.22 | 10.0 |
| | Triethyl citrate | 0.22 | 1.00 |
| | Butylated hydroxytoluene | 0.75 | 3.38 |
| | Ethanol | 399.6 g | |

### Preparation method:

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 13 * 6 mm; 225.2 mg of drug-containing layer material was first filled and pre-compressed, followed by the filling of 91.5 mg of push layer material, and the final compression was performed to obtain a two-layer tablet core with a weight of 316.7 mg/tablet compressed in a direction perpendicular to the long axis.
4. Semi-permeable membrane coating: The two-layer tablet core was coated with the above controlled-release coating solution using a coating pan to a weight gain of 7.3wt% (percentages are weight percentages relative to the tablet core). The holes were punched using mechanical punching or laser punching, and the diameter of the drug-release hole was 1.00 mm. The specifications of the controlled-release tablets in this example were 15.6 mg of carbidopa and 125 mg of levodopa.
5. Immediate-release coating: The tablet core coated by the controlled-release coating solution was coated with the above immediate-release coating solution using a coating pan to a weight gain of 22.2 mg per tablet. The specifications of the controlled-release tablets in this example were 31.25 mg of carbidopa and 125 mg of levodopa.

### Example 30: Capsule-shaped tablets (with a non-circular cross section, and compressed in a direction perpendicular to the long axis) (CD/LD) with specifications of 31.25 mg/125 mg

The prescription is as follows:

| (1) | Drug-containing layer prescription (per tablet) | mg/tablet | Proportion wt% |
|---|---|---|---|
| | Levodopa | 125.0 | 55.5 |
| | Carbidopa monohydrate | 16.9 | 7.50 |
| | Hydroxypropyl cellulose | 22.5 | 10.0 |
| | Sorbitol | 22.5 | 10.0 |
| | Poloxamer | 22.5 | 10.0 |
| | Povidone | 10.5 | 4.66 |
| | Butylated hydroxytoluene | 0.75 | 0.33 |
| | Magnesium stearate | 3.38 | 1.50 |
| | Silica | 1.13 | 0.50 |

| (2) | Push layer prescription (per tablet) | mg/tablet | Proportion wt% |
|---|---|---|---|
| | Sodium carboxymethyl cellulose | 44.8 | 49.0 |
| | Sorbitol | 27.5 | 30.0 |
| | Hydroxypropyl cellulose | 18.3 | 20.0 |
| | Iron oxide red | 0.46 | 0.50 |
| | Magnesium stearate | 0.46 | 0.50 |

| (3) | Controlled-release membrane prescription (4.0% solid content in prescription) | | |
|---|---|---|---|
| | Cellulose acetate | 28.0 g | |
| | Copovidone | 12.0 g | |
| | Acetone | 900 mL | |
| | Ethanol | 100 mL | |

| (4) | Immediate-release prescription (10.0% solid content in prescription) | | |
|---|---|---|---|
| | Immediate-release layer prescription (per tablet) | mg/tablet | Proportion wt% |
| | Carbidopa monohydrate | 16.8 | 76.7 |
| | Hydroxypropyl cellulose | 4.38 | 20.0 |
| | Butylated hydroxytoluene | 0.75 | 3.42 |
| | Ethanol | 395.1 g | |

### Preparation method:

1. Preparation of the drug-containing layer: Levodopa and carbidopa monohydrate were premixed with other excipients of the drug-containing layer (except magnesium stearate and silica), added to a dry granulator and compressed into strips, granulated through a 16-mesh stainless steel screen, and finally added with magnesium stearate and silica and mixed.
2. Preparation of the push layer: Excipients of the push layer (except magnesium stearate) were premixed, added to a dry granulator and compressed into strips, granulated through a 16-mesh stainless steel screen, and finally added with magnesium stearate and mixed.
3. Tablet compressing: Two-layer tablets were compressed with a die size of 13 * 6 mm; 225.2 mg of drug-containing layer material was first filled and pre-compressed, followed by the filling of 91.5 mg of push layer material, and the final compression was performed to obtain a two-layer tablet core with a weight of 316.7 mg/tablet compressed in a direction perpendicular to the long axis.
4. Semi-permeable membrane coating: The two-layer tablet core was coated with the above controlled-release coating solution using a coating pan to a weight gain of 7.3wt% (percentages are weight percentages relative to the tablet core). The holes were punched using mechanical punching or laser punching, and the diameter of the drug-release hole was 1.00 mm. The specifications of the controlled-release tablets in this example were 15.6 mg of carbidopa and 125 mg of levodopa.
5. Immediate-release coating: The tablet core coated by the controlled-release coating solution was coated with the above immediate-release coating solution using a coating pan to a weight gain of 21.9 mg per tablet. The specifications of the controlled-release tablets in this example were 31.25 mg of carbidopa and 125 mg of levodopa.

## Claims

1. A delayed timed release pharmaceutical composition, wherein the dosage form of the pharmaceutical composition is a controlled-release tablet;
the pharmaceutical composition comprises an active pharmaceutical ingredient, and the active pharmaceutical ingredient is levodopa or a derivative thereof, or a mixture of "levodopa or a derivative thereof" and a dopa decarboxylase inhibitor;
the release characteristics of the composition are: before starting to release, the active pharmaceutical ingredient has a time lag of 1-3 hours; after starting to release, the active pharmaceutical ingredient has a cumulative release rate of 85% or more in 6-10 hours.

2. A pharmaceutical composition, wherein the pharmaceutical composition comprises a tablet core, and the tablet core comprises a drug-containing layer and a push layer stacked on the drug-containing layer; the drug-containing layer comprises an active pharmaceutical ingredient;
in the drug-containing layer, the content of the active pharmaceutical ingredient is 5-72.5wt%, and the content is the weight percentage of the active pharmaceutical ingredient in the drug-containing layer;
in the drug-containing layer, the active pharmaceutical ingredient is levodopa or a derivative thereof, or a mixture of "levodopa or a derivative thereof" and a dopa decarboxylase inhibitor;
when the active pharmaceutical ingredient is a mixture of "levodopa or a derivative thereof" and a dopa decarboxylase inhibitor, the content of the dopa decarboxylase inhibitor is ≤ 14.5wt% but not 0, and the content is the weight percentage of this component in the drug-containing layer;
the dosage form of the pharmaceutical composition is a capsule-shaped tablet.

3. The pharmaceutical composition according to claim 2, wherein the pharmaceutical composition satisfies one or more of the following conditions:
(1) in the drug-containing layer, the content of the active pharmaceutical ingredient is 30-72.5wt%, such as 31.5wt%, 50wt%, 53wt%, 58wt%, 59.6wt%, 63wt% or 68.1wt%;
(2) in the drug-containing layer, the content of the levodopa or the derivative thereof is 27.7-63wt%, such as 27.7wt%, 31.5wt%, 41.7wt%, 46.9wt%, 50wt%, 51.1wt%, 53.6wt%, 55.5wt% or 63wt%;
(3) in the drug-containing layer, the derivative of levodopa is a levodopa hydrate, a levodopa alkyl ester, a pharmaceutically acceptable salt of levodopa, a deuterated levodopa alkyl ester or a pharmaceutically acceptable salt of a deuterated levodopa alkyl ester;
(4) in the drug-containing layer, the dopa decarboxylase inhibitor is a carbidopa hydrate, a pharmaceutically acceptable salt of carbidopa, benserazide or a pharmaceutically acceptable salt of benserazide, preferably a carbidopa hydrate, such as carbidopa monohydrate;
(5) in the drug-containing layer, the content of the dopa decarboxylase inhibitor is 3.7-14.5wt%, such as 3.75wt%, 6.91wt%, 7.5wt%, 11.3wt%, 12.7wt% or 14.5wt%;
(6) the drug-containing layer further comprises a pharmaceutical excipient, and the pharmaceutical excipient comprises one or more of pharmaceutical carriers, fillers, surfactants, lubricants and antioxidants, and may further comprise "pharmaceutical carriers and lubricants", "pharmaceutical carriers, fillers and lubricants", "pharmaceutical carriers, fillers, surfactants and lubricants" or "pharmaceutical carriers, fillers, surfactants, antioxidants and lubricants";
(7) the cross-sectional shape of the capsule-shaped tablet is a circular shape or a non-circular shape;
(8) the shape of the capsule-shaped tablet is a capsule shape formed by compressing in the long-axis direction or a capsule shape formed by compressing in a direction perpendicular to the long axis.

4. The pharmaceutical composition according to claim 3, wherein the pharmaceutical composition satisfies one or more of the following conditions:
(1) the pharmaceutical carrier is one or more of povidone, copovidone, carbomer, hypromellose, hydroxypropyl cellulose, hydroxyethyl cellulose, polyoxyethylene and sodium alginate, and may further be a mixture of povidone and hydroxypropyl cellulose or hydroxypropyl cellulose;
(2) when the drug-containing layer comprises a pharmaceutical excipient and the pharmaceutical excipient comprises a pharmaceutical carrier, then the content of the pharmaceutical carrier is ≤ 40wt%, but not 0, and may be 10-40wt%, such as 15wt%, 16wt%, 29.5wt%, 30.0wt%, 31wt% or 38.5wt%;
(3) the filler is one or more of lactose, starch, pregelatinized starch, dextrin, mannitol, sorbitol and microcrystalline cellulose, such as a mixture of sorbitol and lactose, a mixture of sorbitol and mannitol, sorbitol, or mannitol;
(4) when the drug-containing layer comprises a pharmaceutical excipient and the pharmaceutical excipient comprises a filler, then the content of the filler is ≤ 51wt% but not 0, and may be 5-51wt%, such as 5wt%, 8.5wt%, 10wt%, 15wt%, 18wt%, 19wt%, 19.5wt%, 20wt%, 32wt%, 41.5wt% or 51wt%;
(5) the surfactant is one or more of polysorbate, poloxamer, fatty acid glyceride, sodium dodecylbenzenesulfonate and sodium dodecyl sulfate, such as poloxamer;
(6) when the drug-containing layer comprises a pharmaceutical excipient and the pharmaceutical excipient comprises a surfactant, then the content of the surfactant is ≤ 19wt%, but not 0, and may further be 5-19wt%, such as 5wt%, 10wt% or 19wt%;
(7) the lubricant is one or more of stearic acid, silica, magnesium stearate, calcium stearate, polyethylene glycol and sodium stearyl fumarate, such as magnesium stearate and/or silica;
(8) when the drug-containing layer comprises a pharmaceutical excipient and the pharmaceutical excipient comprises a lubricant, then the content of the lubricant is ≤ 2.5wt%, but not 0, and may be 1-2.5wt%, such as 1wt% or 2.5wt%;
(9) the antioxidant is one or more of butylated hydroxytoluene, butylated hydroxyanisole, *tert-*butylhydroquinone, propyl gallate, vitamin C and vitamin E, such as butylated hydroxytoluene;
(10) when the drug-containing layer comprises a pharmaceutical excipient and the pharmaceutical excipient comprises an antioxidant, then the content of the antioxidant is ≤ 1.0wt%, but not 0, and may be 0.1-0.5%, such as 0.33wt%;
(11) when the cross-sectional shape of the capsule-shaped tablet is a circular shape, the circular shape has a diameter of 5-10 mm and a height of 4-30 mm; the cross-sectional diameter may be 5-7.5 mm, and the height may be 10-20 mm;
(12) when the cross-sectional shape of the capsule-shaped tablet is a non-circular shape, the non-circular shape is a non-circular shape having a symmetry axis, and the length of the long axis of the non-circular shape with the symmetry axis may be 10-25 mm, and the length of the short axis may be 5-25 mm; the length of the long axis of the non-circular shape with the symmetry axis may further be 16-19 mm, and the length of the short axis may further be 7-7.5 mm.

5. The pharmaceutical composition according to claim 4, wherein when the pharmaceutical excipient comprises a pharmaceutical carrier and a filler, the weight ratio of the filler to the pharmaceutical carrier is (1-6):1, and may be (1-2):1;
preferably:
when the pharmaceutical carrier is a mixture of povidone and hydroxypropyl cellulose or hydroxypropyl cellulose, and the filler is sorbitol, a mixture of sorbitol and lactose, or a mixture of sorbitol and mannitol, then the weight ratio of sorbitol in the filler to hydroxypropyl cellulose in the pharmaceutical carrier is (1-2):1, such as 1:1, 1.95:1 or 2:1.

6. The pharmaceutical composition according to claim 1, wherein the drug-containing layer comprises any one of the following components:
(1) an active pharmaceutical ingredient, a pharmaceutical carrier and a lubricant;
(2) an active pharmaceutical ingredient, a pharmaceutical carrier, a filler and a lubricant;
(3) an active pharmaceutical ingredient, a pharmaceutical carrier, a filler, a surfactant and a lubricant;
(4) an active pharmaceutical ingredient, a pharmaceutical carrier, a filler, a surfactant, an antioxidant and a lubricant;
the active pharmaceutical ingredient is as defined in any one of claims 1-5;
the pharmaceutical carrier, the lubricant, the filler, the surfactant and the antioxidant are all as defined in any one of claims 2-5.

7. The pharmaceutical composition according to claim 1, wherein the active pharmaceutical ingredient in the drug-containing layer is levodopa; the content of the levodopa is 27.7-63wt%;
the dopa decarboxylase inhibitor is carbidopa or a hydrate thereof; the content of the dopa decarboxylase inhibitor is 7.5-14.5wt%;
the drug-containing layer comprises a pharmaceutical excipient, and the pharmaceutical excipient comprises " a pharmaceutical carrier, a filler and a lubricant", "a pharmaceutical carrier, a filler, a surfactant and a lubricant" or "a pharmaceutical carrier, a filler, a surfactant, an antioxidant and a lubricant";
the pharmaceutical carrier is a mixture of povidone and hydroxypropyl cellulose or hydroxypropyl cellulose; the content of the pharmaceutical carrier is 10-40wt%;
the filler is a mixture of sorbitol and lactose, a mixture of sorbitol and mannitol, or sorbitol; the content of the filler is 8.5-20wt%;
the weight ratio of sorbitol in the filler to hydroxypropyl cellulose in the pharmaceutical carrier is (1-2):1; the surfactant is poloxamer; the content of the surfactant is 5-19wt%;
the lubricant is magnesium stearate and/or silica; the content of the lubricant is 1-2.5wt%;
the antioxidant is butylated hydroxytoluene; the content of the antioxidant is 0.1-1.0%;
the cross-sectional shape of the capsule-shaped tablet is a circular shape.

8. The pharmaceutical composition according to claim 1, wherein the push layer comprises one or more of swelling agents, osmotic pressure enhancers, lubricants and colorants, preferably comprises a swelling agent, an osmotic pressure enhancer, a colorant and a lubricant.

9. The pharmaceutical composition according to claim 8, wherein the pharmaceutical composition satisfies one or more of the following conditions:
(1) the swelling agent is one or more of sodium carboxymethyl starch, hypromellose, hydroxypropyl cellulose, sodium carboxymethyl cellulose, hydroxyethyl cellulose, carbomer, carrageenan, polyoxyethylene and sodium alginate, such as sodium carboxymethyl cellulose and hydroxypropyl cellulose;
(2) the content of the swelling agent is 25-89wt%, such as 89wt% or 69wt%;
(3) the osmotic pressure enhancer is one or more of sodium chloride, potassium chloride, magnesium chloride, sodium sulfate, magnesium sulfate, ascorbic acid, tartaric acid, mannitol, sorbitol, xylitol, glucose, lactose and sucrose, such as sorbitol;
(4) the content of the osmotic pressure enhancer is 10-70wt%, and may be 10-30wt%;
(5) the lubricant is one or more of stearic acid, magnesium stearate, calcium stearate, polyethylene glycol and sodium stearyl fumarate, such as magnesium stearate;
(6) the content of the lubricant is 0.1-3wt%, such as 0.5wt%;
(7) the colorant is one or more of iron oxide red, iron oxide yellow, iron oxide violet and iron oxide black, such as iron oxide red;
(8) the content of the colorant is 0.1-2wt%, such as 0.5wt%.

10. The pharmaceutical composition according to claim 1, wherein the tablet core comprises any one of the following components:
(1) in the drug-containing layer: an active pharmaceutical ingredient, a pharmaceutical carrier and a lubricant; in the push layer: a swelling agent, an osmotic pressure enhancer, a colorant and a lubricant;
(2) in the drug-containing layer: an active pharmaceutical ingredient, a pharmaceutical carrier, a filler and a lubricant; in the push layer: a swelling agent, an osmotic pressure enhancer, a colorant and a lubricant;
(3) in the drug-containing layer: an active pharmaceutical ingredient, a pharmaceutical carrier, a filler, a surfactant and a lubricant; in the push layer: a swelling agent, an osmotic pressure enhancer, a colorant and a lubricant;
(4) in the drug-containing layer: an active pharmaceutical ingredient, a pharmaceutical carrier, a filler, a surfactant, an antioxidant and a lubricant; in the push layer: a swelling agent, an osmotic pressure enhancer, a colorant and a lubricant;
in the drug-containing layer: the active pharmaceutical ingredient is as defined in any one of claims 1-5; the pharmaceutical carrier, the lubricant, the filler, the surfactant and the antioxidant are all as defined in any one of claims 2-5;
in the push layer: the swelling agent, the osmotic pressure enhancer, the lubricant and the colorant are all as defined in claim 8 or 9.

11. The pharmaceutical composition according to claim 1, wherein the composition is any one of the following groups:
(1) in the drug-containing layer: an active pharmaceutical ingredient, a pharmaceutical carrier and a lubricant; in the push layer: a swelling agent, an osmotic pressure enhancer, a colorant and a lubricant;
the cross-sectional shape of the capsule-shaped tablet is a non-circular shape or the shape of the capsule-shaped tablet is a capsule shape formed by compressing in a direction perpendicular to the long axis;
(2) in the drug-containing layer: an active pharmaceutical ingredient, a pharmaceutical carrier, a filler and a lubricant; in the push layer: a swelling agent, an osmotic pressure enhancer, a colorant and a lubricant;
the cross-sectional shape of the capsule-shaped tablet is a non-circular shape or the shape of the capsule-shaped tablet is a capsule shape formed by compressing in a direction perpendicular to the long axis;
(3) in the drug-containing layer: an active pharmaceutical ingredient, a pharmaceutical carrier, a filler, a surfactant and a lubricant; in the push layer: a swelling agent, an osmotic pressure enhancer, a colorant and a lubricant;
the cross-sectional shape of the capsule-shaped tablet is a non-circular shape or the shape of the capsule-shaped tablet is a capsule shape formed by compressing in a direction perpendicular to the long axis;
(4) in the drug-containing layer: an active pharmaceutical ingredient, a pharmaceutical carrier, a filler, an antioxidant and a lubricant; in the push layer: a swelling agent, an osmotic pressure enhancer, a colorant and a lubricant;
the cross-sectional shape of the capsule-shaped tablet is a non-circular shape or the shape of the capsule-shaped tablet is a capsule shape formed by compressing in a direction perpendicular to the long axis;
(5) in the drug-containing layer: an active pharmaceutical ingredient, a pharmaceutical carrier, a filler and a lubricant; in the push layer: a swelling agent, an osmotic pressure enhancer, a colorant and a lubricant;
the cross-sectional shape of the capsule-shaped tablet is a circular shape or the shape of the capsule-shaped tablet is a capsule shape formed by compressing in the long-axis direction;
(6) in the drug-containing layer: an active pharmaceutical ingredient, a pharmaceutical carrier, a filler, a surfactant and a lubricant; in the push layer: a swelling agent, an osmotic pressure enhancer, a colorant and a lubricant;
the cross-sectional shape of the capsule-shaped tablet is a circular shape or the shape of the capsule-shaped tablet is a capsule shape formed by compressing in the long-axis direction;
(7) in the drug-containing layer: an active pharmaceutical ingredient, a pharmaceutical carrier, a filler, a surfactant, an antioxidant and a lubricant; in the push layer: a swelling agent, an osmotic pressure enhancer, a colorant and a lubricant;
the cross-sectional shape of the capsule-shaped tablet is a circular shape or the shape of the capsule-shaped tablet is a capsule shape formed by compressing in the long-axis direction;
in the drug-containing layer: the active pharmaceutical ingredient is as defined in any one of claims 1-5; the pharmaceutical carrier, the lubricant, the filler, the surfactant and the antioxidant are all as defined in any one of claims 2-5;
in the push layer, the swelling agent, the osmotic pressure enhancer, the lubricant and the colorant are all as defined in claim 8 or 9.

12. The pharmaceutical composition according to claim 1, wherein the tablet core further comprises a controlled-release membrane coating coated on the tablet core;
the controlled-release membrane coating is a semi-permeable coating membrane, and the membrane-forming material of the semi-permeable coating membrane is one or more of cellulose acetate, ethyl cellulose and acrylic resin, such as cellulose acetate;
the content of the membrane-forming material is 50-90wt%, and may be 60-90wt%, such as 60wt%, 65wt%, 70wt% or 90wt%;
the semi-permeable coating membrane may further comprise a porogen and a plasticizer;
the porogen may be one or more of polyethylene glycol, glycerol, povidone, copovidone and hydroxypropyl cellulose, such as copovidone and hydroxypropyl cellulose;
the plasticizer may be one or more of polyethylene glycol, methyl phthalate, ethyl phthalate, dibutyl sebacate, triethyl citrate, tributyl citrate, acetyl tributyl citrate, glycerine acetate and castor oil, such as polyethylene glycol or triethyl citrate;
the weight ratio of the controlled-release membrane coating to the tablet core may be (2.0%-15.0%):1, may further be (6.6%-8.4%):1, and may further be (7.4%-7.8%):1.

13. The pharmaceutical composition according to claim 12, wherein an isolation coating layer is further comprised between the tablet core and the controlled-release membrane coating;
the membrane-forming material of the isolation coating layer may be hydroxypropyl cellulose EF;
the weight ratio of the isolation coating layer to the tablet core may be (3.0%-5.0%): 1, such as 4.0%: 1.

14. The pharmaceutical composition according to claim 1, wherein the pharmaceutical composition further comprises an immediate-release layer; the immediate-release layer comprises an active pharmaceutical ingredient, a binder and an antioxidant, and may comprise an active pharmaceutical ingredient, a plasticizer, an antioxidant and a binder;
when the immediate-release layer comprises a plasticizer, the content of the plasticizer is preferably 1-2wt%;
when the immediate-release layer comprises a plasticizer, the plasticizer is preferably one or more of polyethylene glycol, methyl phthalate, ethyl phthalate, dibutyl sebacate, triethyl citrate, tributyl citrate, acetyl tributyl citrate, glycerine acetate and castor oil, such as tributyl citrate;
when the immediate-release layer comprises an antioxidant, the content of the antioxidant is preferably 3-4wt%, such as 3.38wt% or 3.81wt%;
when the immediate-release layer comprises an antioxidant, the antioxidant is preferably one or more of butylated hydroxytoluene, butylated hydroxyanisole, dibutylphenol, vitamin C, vitamin E and sodium sulfite, such as butylated hydroxytoluene;
when the immediate-release layer comprises a binder, the content of the binder is preferably 10-20wt%;
when the immediate-release layer comprises a binder, the binder is preferably one or more of povidone, copovidone, hydroxypropyl cellulose and hypromellose, for example, a mixture of hydroxypropyl cellulose and copovidone or hydroxypropyl cellulose;
when the immediate-release layer comprises an active pharmaceutical ingredient, the content of the active pharmaceutical ingredient is preferably 70-90wt%, such as 74.7wt%, 75.7wt%, 86.2wt% or 85.3wt%;
when the immediate-release layer comprises an active pharmaceutical ingredient, the active pharmaceutical ingredient preferably comprises a dopa decarboxylase inhibitor; the dopa decarboxylase inhibitor is preferably a carbidopa hydrate, a pharmaceutically acceptable salt of carbidopa, benserazide or a pharmaceutically acceptable salt of benserazide, more preferably a carbidopa hydrate, such as carbidopa monohydrate.

15. A preparation method for the pharmaceutical composition according to any one of claims 1-14, comprising the following steps:
stacking and composite compressing the raw material particles of the drug-containing layer and the raw material particles of the push layer to form a tablet core;
when the tablet core is further coated with a controlled-release membrane coating, coating is performed on the tablet core, *i.e.,* the controlled-release membrane coating is coated on the tablet core, and the controlled-release membrane coating on one side of the drug-containing layer is punched to form a drug-release hole;
when the pharmaceutical composition is further coated with an immediate-release layer, an immediate-release layer coating is coated on the capsule-shaped tablet coating the controlled-release membrane coating;
preferably, the composite compressing may be performed in the long-axis direction or in a direction perpendicular to the long axis.

16. Use of the pharmaceutical composition according to any one of claims 1-12 in the manufacture of a medicament for preventing or treating morning akinesia;
the morning akinesia is preferably morning akinesia caused by Parkinson's disease;
or, a method for preventing or treating morning akinesia, comprising administering to a subject an effective amount of the pharmaceutical composition according to any one of claims 1-12;
in the method, the morning akinesia is preferably morning akinesia caused by Parkinson's disease.

## Amended claims

### Amended claims under Art. 19.1 PCT

1. A delayed timed release pharmaceutical composition, wherein the dosage form of the pharmaceutical composition is a controlled-release tablet;
the pharmaceutical composition comprises an active pharmaceutical ingredient, and the active pharmaceutical ingredient is levodopa or a derivative thereof, or a mixture of "levodopa or a derivative thereof" and a dopa decarboxylase inhibitor;
the release characteristics of the composition are: before starting to release, the active pharmaceutical ingredient has a time lag of 1-3 hours; after starting to release, the active pharmaceutical ingredient has a cumulative release rate of 85% or more in 6-10 hours;
when the active pharmaceutical ingredient is a mixture of "levodopa or a derivative thereof" and a dopa decarboxylase inhibitor, the content of the dopa decarboxylase inhibitor is ≤ 7.5wt%, but not 0, and the content is the weight percentage of this component in a drug-containing layer.

2. A pharmaceutical composition, wherein the pharmaceutical composition comprises a tablet core, and the tablet core comprises a drug-containing layer and a push layer stacked on the drug-containing layer; the drug-containing layer comprises an active pharmaceutical ingredient;
in the drug-containing layer, the content of the active pharmaceutical ingredient is 5-72.5wt%, and the content is the weight percentage of the active pharmaceutical ingredient in the drug-containing layer;
in the drug-containing layer, the active pharmaceutical ingredient is levodopa or a derivative thereof, or a mixture of "levodopa or a derivative thereof" and a dopa decarboxylase inhibitor;
when the active pharmaceutical ingredient is a mixture of "levodopa or a derivative thereof" and a dopa decarboxylase inhibitor, the content of the dopa decarboxylase inhibitor is ≤ 7.5wt% but not 0, and the content is the weight percentage of this component in the drug-containing layer;
the dosage form of the pharmaceutical composition is a capsule-shaped tablet.

3. The pharmaceutical composition according to claim 2, wherein the pharmaceutical composition satisfies one or more of the following conditions:
(1) in the drug-containing layer, the content of the active pharmaceutical ingredient is 30-72.5wt%, such as 31.5wt%, 50wt%, 53wt%, 58wt%, 59.6wt%, 63wt% or 68.1wt%;
(2) in the drug-containing layer, the content of the levodopa or the derivative thereof is 27.7-63wt%, such as 27.7wt%, 31.5wt%, 41.7wt%, 46.9wt%, 50wt%, 51.1wt%, 53.6wt%, 55.5wt% or 63wt%;
(3) in the drug-containing layer, the derivative of levodopa is a levodopa hydrate, a levodopa alkyl ester, a pharmaceutically acceptable salt of levodopa, a deuterated levodopa alkyl ester or a pharmaceutically acceptable salt of a deuterated levodopa alkyl ester;
(4) in the drug-containing layer, the dopa decarboxylase inhibitor is a carbidopa hydrate, a pharmaceutically acceptable salt of carbidopa, benserazide or a pharmaceutically acceptable salt of benserazide, preferably a carbidopa hydrate, such as carbidopa monohydrate;
(5) in the drug-containing layer, the content of the dopa decarboxylase inhibitor is 3.7-7.5wt%, such as 3.75wt%, 6.91wt% or 7.5wt%%;
(6) the drug-containing layer further comprises a pharmaceutical excipient, and the pharmaceutical excipient comprises one or more of pharmaceutical carriers, fillers, surfactants, lubricants and antioxidants, and may further comprise "pharmaceutical carriers and lubricants", "pharmaceutical carriers, fillers and lubricants", "pharmaceutical carriers, fillers, surfactants and lubricants" or "pharmaceutical carriers, fillers, surfactants, antioxidants and lubricants";
(7) the cross-sectional shape of the capsule-shaped tablet is a circular shape or a non-circular shape;
(8) the shape of the capsule-shaped tablet is a capsule shape formed by compressing in the long-axis direction or a capsule shape formed by compressing in a direction perpendicular to the long axis.

4. The pharmaceutical composition according to claim 3, wherein the pharmaceutical composition satisfies one or more of the following conditions:
(1) the pharmaceutical carrier is one or more of povidone, copovidone, carbomer, hypromellose, hydroxypropyl cellulose, hydroxyethyl cellulose, polyoxyethylene and sodium alginate, and may further be a mixture of povidone and hydroxypropyl cellulose or hydroxypropyl cellulose;
(2) when the drug-containing layer comprises a pharmaceutical excipient and the pharmaceutical excipient comprises a pharmaceutical carrier, then the content of the pharmaceutical carrier is ≤ 40wt%, but not 0, and may be 10-40wt%, such as 15wt%, 16wt%, 29.5wt%, 30.0wt%, 31wt% or 38.5wt%;
(3) the filler is one or more of lactose, starch, pregelatinized starch, dextrin, mannitol, sorbitol and microcrystalline cellulose, such as a mixture of sorbitol and lactose, a mixture of sorbitol and mannitol, sorbitol, or mannitol;
(4) when the drug-containing layer comprises a pharmaceutical excipient and the pharmaceutical excipient comprises a filler, then the content of the filler is ≤ 51wt% but not 0, and may be 5-51wt%, such as 5wt%, 8.5wt%, 10wt%, 15wt%, 18wt%, 19wt%, 19.5wt%, 20wt%, 32wt%, 41.5wt% or 51wt%;
(5) the surfactant is one or more of polysorbate, poloxamer, fatty acid glyceride, sodium dodecylbenzenesulfonate and sodium dodecyl sulfate, such as poloxamer;
(6) when the drug-containing layer comprises a pharmaceutical excipient and the pharmaceutical excipient comprises a surfactant, then the content of the surfactant is ≤ 19wt%, but not 0, and may further be 5-19wt%, such as 5wt%, 10wt% or 19wt%;
(7) the lubricant is one or more of stearic acid, silica, magnesium stearate, calcium stearate, polyethylene glycol and sodium stearyl fumarate, such as magnesium stearate and/or silica;
(8) when the drug-containing layer comprises a pharmaceutical excipient and the pharmaceutical excipient comprises a lubricant, then the content of the lubricant is ≤ 2.5wt%, but not 0, and may be 1-2.5wt%, such as 1wt% or 2.5wt%;
(9) the antioxidant is one or more of butylated hydroxytoluene, butylated hydroxyanisole, *tert-*butylhydroquinone, propyl gallate, vitamin C and vitamin E, such as butylated hydroxytoluene;
(10) when the drug-containing layer comprises a pharmaceutical excipient and the pharmaceutical excipient comprises an antioxidant, then the content of the antioxidant is ≤ 1.0wt%, but not 0, and may be 0.1-0.5%, such as 0.33wt%;
(11) when the cross-sectional shape of the capsule-shaped tablet is a circular shape, the circular shape has a diameter of 5-10 mm and a height of 4-30 mm; the cross-sectional diameter may be 5-7.5 mm, and the height may be 10-20 mm;
(12) when the cross-sectional shape of the capsule-shaped tablet is a non-circular shape, the non-circular shape is a non-circular shape having a symmetry axis, and the length of the long axis of the non-circular shape with the symmetry axis may be 10-25 mm, and the length of the short axis may be 5-25 mm; the length of the long axis of the non-circular shape with the symmetry axis may further be 16-19 mm, and the length of the short axis may further be 7-7.5 mm.

5. The pharmaceutical composition according to claim 4, wherein when the pharmaceutical excipient comprises a pharmaceutical carrier and a filler, the weight ratio of the filler to the pharmaceutical carrier is (1-6):1, and may be (1-2):1;
preferably:
when the pharmaceutical carrier is a mixture of povidone and hydroxypropyl cellulose or hydroxypropyl cellulose, and the filler is sorbitol, a mixture of sorbitol and lactose, or a mixture of sorbitol and mannitol, then the weight ratio of sorbitol in the filler to hydroxypropyl cellulose in the pharmaceutical carrier is (1-2):1, such as 1:1, 1.95:1 or 2:1.

6. The pharmaceutical composition according to claim 1, wherein the drug-containing layer comprises any one of the following components:
(1) an active pharmaceutical ingredient, a pharmaceutical carrier and a lubricant;
(2) an active pharmaceutical ingredient, a pharmaceutical carrier, a filler and a lubricant;
(3) an active pharmaceutical ingredient, a pharmaceutical carrier, a filler, a surfactant and a lubricant;
(4) an active pharmaceutical ingredient, a pharmaceutical carrier, a filler, a surfactant, an antioxidant and a lubricant;
the active pharmaceutical ingredient is as defined in any one of claims 1-5;
the pharmaceutical carrier, the lubricant, the filler, the surfactant and the antioxidant are all as defined in any one of claims 2-5.

7. The pharmaceutical composition according to claim 1, wherein the active pharmaceutical ingredient in the drug-containing layer is levodopa or a mixture of "levodopa or a derivative thereof" and a dopa decarboxylase inhibitor; the content of the levodopa is 27.7-63wt%;
when the active pharmaceutical ingredient is a mixture of "levodopa or a derivative thereof" and a dopa decarboxylase inhibitor, the dopa decarboxylase inhibitor is carbidopa or a hydrate thereof; the content of the dopa decarboxylase inhibitor is ≤ 7.5wt%, but not 0;
the drug-containing layer comprises a pharmaceutical excipient, and the pharmaceutical excipient comprises " a pharmaceutical carrier, a filler and a lubricant", "a pharmaceutical carrier, a filler, a surfactant and a lubricant" or "a pharmaceutical carrier, a filler, a surfactant, an antioxidant and a lubricant";
the pharmaceutical carrier is a mixture of povidone and hydroxypropyl cellulose or hydroxypropyl cellulose; the content of the pharmaceutical carrier is 10-40wt%;
the filler is a mixture of sorbitol and lactose, a mixture of sorbitol and mannitol, or sorbitol; the content of the filler is 8.5-20wt%;
the weight ratio of sorbitol in the filler to hydroxypropyl cellulose in the pharmaceutical carrier is (1-2):1;
the surfactant is poloxamer; the content of the surfactant is 5-19wt%;
the lubricant is magnesium stearate and/or silica; the content of the lubricant is 1-2.5wt%;
the antioxidant is butylated hydroxytoluene; the content of the antioxidant is 0.1-1.0%;
the cross-sectional shape of the capsule-shaped tablet is a circular shape.

8. The pharmaceutical composition according to claim 1, wherein the push layer comprises one or more of swelling agents, osmotic pressure enhancers, lubricants and colorants, preferably comprises a swelling agent, an osmotic pressure enhancer, a colorant and a lubricant.

9. The pharmaceutical composition according to claim 8, wherein the pharmaceutical composition satisfies one or more of the following conditions:
(1) the swelling agent is one or more of sodium carboxymethyl starch, hypromellose, hydroxypropyl cellulose, sodium carboxymethyl cellulose, hydroxyethyl cellulose, carbomer, carrageenan, polyoxyethylene and sodium alginate, such as sodium carboxymethyl cellulose and hydroxypropyl cellulose;
(2) the content of the swelling agent is 25-89wt%, such as 89wt% or 69wt%;
(3) the osmotic pressure enhancer is one or more of sodium chloride, potassium chloride, magnesium chloride, sodium sulfate, magnesium sulfate, ascorbic acid, tartaric acid, mannitol, sorbitol, xylitol, glucose, lactose and sucrose, such as sorbitol;
(4) the content of the osmotic pressure enhancer is 10-70wt%, and may be 10-30wt%;
(5) the lubricant is one or more of stearic acid, magnesium stearate, calcium stearate, polyethylene glycol and sodium stearyl fumarate, such as magnesium stearate;
(6) the content of the lubricant is 0.1-3wt%, such as 0.5wt%;
(7) the colorant is one or more of iron oxide red, iron oxide yellow, iron oxide violet and iron oxide black, such as iron oxide red;
(8) the content of the colorant is 0.1-2wt%, such as 0.5wt%.

10. The pharmaceutical composition according to claim 1, wherein the tablet core comprises any one of the following components:
(1) in the drug-containing layer: an active pharmaceutical ingredient, a pharmaceutical carrier and a lubricant; in the push layer: a swelling agent, an osmotic pressure enhancer, a colorant and a lubricant;
(2) in the drug-containing layer: an active pharmaceutical ingredient, a pharmaceutical carrier, a filler and a lubricant; in the push layer: a swelling agent, an osmotic pressure enhancer, a colorant and a lubricant;
(3) in the drug-containing layer: an active pharmaceutical ingredient, a pharmaceutical carrier, a filler, a surfactant and a lubricant; in the push layer: a swelling agent, an osmotic pressure enhancer, a colorant and a lubricant;
(4) in the drug-containing layer: an active pharmaceutical ingredient, a pharmaceutical carrier, a filler, a surfactant, an antioxidant and a lubricant; in the push layer: a swelling agent, an osmotic pressure enhancer, a colorant and a lubricant;
in the drug-containing layer: the active pharmaceutical ingredient is as defined in any one of claims 1-5; the pharmaceutical carrier, the lubricant, the filler, the surfactant and the antioxidant are all as defined in any one of claims 2-5;
in the push layer: the swelling agent, the osmotic pressure enhancer, the lubricant and the colorant are all as defined in claim 8 or 9.

11. The pharmaceutical composition according to claim 1, wherein the composition is any one of the following groups:
(1) in the drug-containing layer: an active pharmaceutical ingredient, a pharmaceutical carrier and a lubricant; in the push layer: a swelling agent, an osmotic pressure enhancer, a colorant and a lubricant;
the cross-sectional shape of the capsule-shaped tablet is a non-circular shape or the shape of the capsule-shaped tablet is a capsule shape formed by compressing in a direction perpendicular to the long axis;
(2) in the drug-containing layer: an active pharmaceutical ingredient, a pharmaceutical carrier, a filler and a lubricant; in the push layer: a swelling agent, an osmotic pressure enhancer, a colorant and a lubricant;
the cross-sectional shape of the capsule-shaped tablet is a non-circular shape or the shape of the capsule-shaped tablet is a capsule shape formed by compressing in a direction perpendicular to the long axis;
(3) in the drug-containing layer: an active pharmaceutical ingredient, a pharmaceutical carrier, a filler, a surfactant and a lubricant; in the push layer: a swelling agent, an osmotic pressure enhancer, a colorant and a lubricant;
the cross-sectional shape of the capsule-shaped tablet is a non-circular shape or the shape of the capsule-shaped tablet is a capsule shape formed by compressing in a direction perpendicular to the long axis;
(4) in the drug-containing layer: an active pharmaceutical ingredient, a pharmaceutical carrier, a filler, an antioxidant and a lubricant; in the push layer: a swelling agent, an osmotic pressure enhancer, a colorant and a lubricant;
the cross-sectional shape of the capsule-shaped tablet is a non-circular shape or the shape of the capsule-shaped tablet is a capsule shape formed by compressing in a direction perpendicular to the long axis;
(5) in the drug-containing layer: an active pharmaceutical ingredient, a pharmaceutical carrier, a filler and a lubricant; in the push layer: a swelling agent, an osmotic pressure enhancer, a colorant and a lubricant;
the cross-sectional shape of the capsule-shaped tablet is a circular shape or the shape of the capsule-shaped tablet is a capsule shape formed by compressing in the long-axis direction;
(6) in the drug-containing layer: an active pharmaceutical ingredient, a pharmaceutical carrier, a filler, a surfactant and a lubricant; in the push layer: a swelling agent, an osmotic pressure enhancer, a colorant and a lubricant;
the cross-sectional shape of the capsule-shaped tablet is a circular shape or the shape of the capsule-shaped tablet is a capsule shape formed by compressing in the long-axis direction;
(7) in the drug-containing layer: an active pharmaceutical ingredient, a pharmaceutical carrier, a filler, a surfactant, an antioxidant and a lubricant; in the push layer: a swelling agent, an osmotic pressure enhancer, a colorant and a lubricant;
the cross-sectional shape of the capsule-shaped tablet is a circular shape or the shape of the capsule-shaped tablet is a capsule shape formed by compressing in the long-axis direction;
in the drug-containing layer: the active pharmaceutical ingredient is as defined in any one of claims 1-5; the pharmaceutical carrier, the lubricant, the filler, the surfactant and the antioxidant are all as defined in any one of claims 2-5;
in the push layer, the swelling agent, the osmotic pressure enhancer, the lubricant and the colorant are all as defined in claim 8 or 9.

12. The pharmaceutical composition according to claim 1, wherein the tablet core further comprises a controlled-release membrane coating coated on the tablet core;
the controlled-release membrane coating is a semi-permeable coating membrane, and the membrane-forming material of the semi-permeable coating membrane is one or more of cellulose acetate, ethyl cellulose and acrylic resin, such as cellulose acetate;
the content of the membrane-forming material is 50-90wt%, and may be 60-90wt%, such as 60wt%, 65wt%, 70wt% or 90wt%;
the semi-permeable coating membrane may further comprise a porogen and a plasticizer;
the porogen may be one or more of polyethylene glycol, glycerol, povidone, copovidone and hydroxypropyl cellulose, such as copovidone and hydroxypropyl cellulose;
the plasticizer may be one or more of polyethylene glycol, methyl phthalate, ethyl phthalate, dibutyl sebacate, triethyl citrate, tributyl citrate, acetyl tributyl citrate, glycerine acetate and castor oil, such as polyethylene glycol or triethyl citrate;
the weight ratio of the controlled-release membrane coating to the tablet core may be (2.0%-15.0%):1, may further be (6.6%-8.4%):1, and may further be (7.4%-7.8%):1.

13. The pharmaceutical composition according to claim 12, wherein an isolation coating layer is further comprised between the tablet core and the controlled-release membrane coating;
the membrane-forming material of the isolation coating layer may be hydroxypropyl cellulose EF;
the weight ratio of the isolation coating layer to the tablet core may be (3.0%-5.0%): 1, such as 4.0%: 1.

14. The pharmaceutical composition according to claim 1, wherein the pharmaceutical composition further comprises an immediate-release layer; the immediate-release layer comprises an active pharmaceutical ingredient, a binder and an antioxidant, and may comprise an active pharmaceutical ingredient, a plasticizer, an antioxidant and a binder;
when the immediate-release layer comprises a plasticizer, the content of the plasticizer is preferably 1-2wt%;
when the immediate-release layer comprises a plasticizer, the plasticizer is preferably one or more of polyethylene glycol, methyl phthalate, ethyl phthalate, dibutyl sebacate, triethyl citrate, tributyl citrate, acetyl tributyl citrate, glycerine acetate and castor oil, such as tributyl citrate;
when the immediate-release layer comprises an antioxidant, the content of the antioxidant is preferably 3-4wt%, such as 3.38wt% or 3.81wt%;
when the immediate-release layer comprises an antioxidant, the antioxidant is preferably one or more of butylated hydroxytoluene, butylated hydroxyanisole, dibutylphenol, vitamin C, vitamin E and sodium sulfite, such as butylated hydroxytoluene;
when the immediate-release layer comprises a binder, the content of the binder is preferably 10-20wt%;
when the immediate-release layer comprises a binder, the binder is preferably one or more of povidone, copovidone, hydroxypropyl cellulose and hypromellose, for example, a mixture of hydroxypropyl cellulose and copovidone or hydroxypropyl cellulose;
when the immediate-release layer comprises an active pharmaceutical ingredient, the content of the active pharmaceutical ingredient is preferably 70-90wt%, such as 74.7wt%, 75.7wt%, 86.2wt% or 85.3wt%;
when the immediate-release layer comprises an active pharmaceutical ingredient, the active pharmaceutical ingredient preferably comprises a dopa decarboxylase inhibitor; the dopa decarboxylase inhibitor is preferably a carbidopa hydrate, a pharmaceutically acceptable salt of carbidopa, benserazide or a pharmaceutically acceptable salt of benserazide, more preferably a carbidopa hydrate, such as carbidopa monohydrate.

15. A preparation method for the pharmaceutical composition according to any one of claims 1-14, comprising the following steps:
stacking and composite compressing the raw material particles of the drug-containing layer and the raw material particles of the push layer to form a tablet core;
when the tablet core is further coated with a controlled-release membrane coating, coating is performed on the tablet core, *i.e.,* the controlled-release membrane coating is coated on the tablet core, and the controlled-release membrane coating on one side of the drug-containing layer is punched to form a drug-release hole;
when the pharmaceutical composition is further coated with an immediate-release layer, an immediate-release layer coating is coated on the capsule-shaped tablet coating the controlled-release membrane coating;
preferably, the composite compressing may be performed in the long-axis direction or in a direction perpendicular to the long axis.

16. Use of the pharmaceutical composition according to any one of claims 1-12 in the manufacture of a medicament for preventing or treating morning akinesia;
the morning akinesia is preferably morning akinesia caused by Parkinson's disease;
or, a method for preventing or treating morning akinesia, comprising administering to a subject an effective amount of the pharmaceutical composition according to any one of claims 1-12;
in the method, the morning akinesia is preferably morning akinesia caused by Parkinson's disease.
